# EUROPEAN PATENT SPECIFICATION

(11) **EP 3 047 039 B1**
(45) Date of publication and mention of the grant of the patent: **31.07.2019**
(21) Application number: 14846222.9
(22) Date of filing: 18.09.2014
(51) Int. Cl.: C12Q 1/68

(54) **INHIBITING CANCER METASTASIS**
HEMMUNG VON KREBSMETASTASEN
INHIBITION DE MÉTASTASES CANCÉREUSES

(30) Priority: 18.09.2013 US 201361879514 P
(43) Date of publication of application: 27.07.2016
(73) Proprietor: Memorial Sloan-Kettering Cancer Center, New York, NY 10065 (US)
(72) Inventor: MASSAGUE, Joan, New York, NY 10065 (US); VALIENTE, Manuel, Brooklyn, NY 11216 (US)
(74) Representative: Zacco Denmark A/S
(86) International application number: PCT/US2014/056379
(87) International publication number: WO 2015/042303

(56) References cited:
- EP-A1- 2 574 930
- WO-A1-2008/046529
- WO-A2-2004/037198
- WO-A2-2009/127414
- US-A1- 2010 029 748
- US-A1- 2011 293 600
- Yves Allory ET AL: "The L1 Cell Adhesion Molecule Is Induced in Renal Cancer Cells and Correlates with Metastasis in Clear Cell Carcinomas", Clinical Cancer Research, 1 February 2005 (2005-02-01), page 1190, XP055350042, United States Retrieved from the Internet: URL:http://clincancerres.aacrjournals.org/ content/clincanres/11/3/1190.full.pdf
- NAGAHARA A ET AL: "SERPINE2 is a possible candidate promotor for lymph node metastasis in testicular cancer", BIOCHEMICAL AND BIOPHYSICAL RESEARCH COMMUNICATIONS, ELSEVIER, AMSTERDAM, NL, vol. 391, no. 4, 22 January 2010 (2010-01-22), pages 1641-1646, XP026863420, ISSN: 0006-291X, DOI: 10.1016/J.BBRC.2009.12.105 [retrieved on 2010-01-21]
- QUARTA S ET AL: "SERPINB3 induces epithelial mesenchymal transition", DIGESTIVE AND LIVER DISEASE, W.B. SAUNDERS, GB, vol. 41, no. 5, 1 May 2009 (2009-05-01), pages A1-A2, XP026070402, ISSN: 1590-8658, DOI: 10.1016/J.DLD.2009.02.012 [retrieved on 2009-03-31]
- GIANLUIGI MAZZOCCOLI ET AL: "and: potential biomarkers for tumor aggressiveness in colorectal cancer", JOURNAL OF CANCER RESEARCH AND CLINICAL ONCOLOGY, SPRINGER, BERLIN, DE, vol. 138, no. 3, 24 December 2011 (2011-12-24), pages 501-511, XP035014199, ISSN: 1432-1335, DOI: 10.1007/S00432-011-1126-6
- VALIENTE ET AL.: 'Cooption of the reactive stroma by brain metastasis relies on protease inhibitors' PROCEEDINGS OF THE AACR SPECIAL CONFERENCE ON TUMOR INVASION AND METASTASIS, CANCER RESEARCH vol. 73, no. A29, 01 February 2013, pages 1 - 2, XP008183049

## Description

### 1. INTRODUCTION

The present invention relates to methods and compositions for inhibiting metastatic spread of cancer in a subject. In particular embodiments, it provides for methods and materials for determining whether a cancer patient is at increased risk for developing metastatic spread of the cancer and, if that is the case, for treating the patient such that the risk of metastatic spread is reduced.

### 2. BACKGROUND OF THE INVENTION

Metastasis is the main cause of death from cancer, but biologically metastasis is a rather inefficient process. Most cancer cells that leave a solid tumor perish, and much of this attrition happens as circulating cancer cells infiltrate distant organs (Chambers et al., 2002; Fidler, 2003; Nguyen et al., 2009a; Schreiber et al., 2011; Valastyan and Weinberg, 2011). Even cell lines that were experimentally enriched for metastasis-initiating activity suffer severe attrition in the organs they invade. The scarcity of survival signals in the host parenchyma, lack of a supportive stroma for cancer stem cells, and an overexposure to innate immunity are postulated causes of elimination of disseminated cancer cells. Although recent work revealed mechanisms for early steps of tumor cell dispersion and for late stages of macrometastatic outgrowth (Valastyan and Weinberg, 2011; Vanharanta and Massagué, 2013), what factors determine the survival and adaptation of disseminated cancer cells in vital organs remain unknown.

Identifying these factors is particularly critical in the case of brain metastasis. Brain relapse is the most devastating complication of cancer, with acute neurologic distress and high mortality as typical traits (Gavrilovic and Posner, 2005; Lutterbach et al., 2002). The incidence of brain metastasis is ten times higher than that of all primary brain tumors combined, and is on the rise (Maher et al., 2009). Lung cancer and breast cancer are the top sources of brain metastasis, together accounting for nearly two thirds of cases. Melanoma, colorectal cancer, and renal cell carcinoma account for most of the rest (Barnholtz-Sloan et al., 2004; Schouten et al., 2002), However, it is in the brain that infiltrating cancer cells face a particularly high rate of attrition, as shown in experimental models (Heyn et al., 2006; Kienast et al., 2010; Perera et al., 2012; Steeg et al., 2011).

In line with this phenomenon, brain metastasis tends to be a late complication of cancer in the clinic (Feld et al., 1984; Karrison et al., 1999; Schmidt-Kittler et al., 2003) and is rare in mice with genetically engineered tumors that readily metastasize to other organs (Francia et al., 2011; Meuwissen et al., 2003; Moody et al., 2002; Regales et al., 2009; Siegel et al., 2003; Winslow et al., 2011). When brain metastasis eventually emerges, the lesions are highly aggressive and resistant to therapy. This point is dramatically illustrated by the current rise in the incidence of brain metastasis of HER2+ breast cancer, a disease in which antibodies targeting the HER2 oncoprotein are effective in controlling extracranial disease but not so against brain metastasis (Leyland-Jones, 2009; Lin and Winer, 2007; Palmieri et al., 2007; Sledge, 2011; Stemmler et al., 2006).

The severe attrition of metastatic cells in the brain and the late occurrence of brain metastasis in the clinic argue that circulating cancer cells face major hurdles in colonizing this organ. One obstacle is the tight nature of the brain capillary walls, the blood-brain barrier (BBB). Cancer cells require specialized mechanisms to traverse the BBB, and molecular mediators of this process were recently identified (Bos et al., 2009; Li et al., 2013). However, most cancer cells that pass the BBB die (Heyn et al., 2006; Kienast et al., 2010; Perera et al., 2012; Steeg et al., 2011) despite the presence of stromal signals and cell-autonomous activities that would favor cell proliferation (Kim et al., 2011; Nguyen et al., 2009a; Qian et al., 2011; Seike et al., 2011). Interestingly, cancer cells that succeed at infiltrating the brain present the striking feature of adhering to the surface of brain capillaries and growing as a furrow around the vessels. Cancer cells that fail to coopt the vasculature in this manner also fail to thrive (Kienast et al., 2010). What kills a majority of cancer cells that pass through the BBB, and what enables the few cells that survive to coopt the vasculature are questions of biologic and clinical interest.

### 3. SUMMARY OF THE INVENTION

The present invention relates to methods and compositions for determining whether a cancer patient is at increased risk for developing metastatic spread of the cancer and, if the patient is at increased risk, for treating the patient such that the risk of metastasis is reduced. It is based, at least in part, on the discoveries that overexpression of serpin and serpin secretion by cancer cells and L1CAM-mediated vascular cooption promote metastasis of lung and breast cancer to the brain, and that antagonism of serpin or L1CAM reduced metastasis.

In certain non-limiting embodiments, the present invention provides for methods and compositions for determining whether a subject having a cancer is at increased risk of having or developing a metastasis of the cancer comprising determining whether a cell of the cancer overexpresses and/or secretes a serpin, where if the cancer cell overexpresses and/or secretes a serpin, then the subject is at increased risk of having or developing a metastasis of the cancer. In certain non-limiting embodiments, said subject is at increased risk of having or developing a metastasis to brain.

In certain non-limiting embodiments, the present invention provides for methods and compositions for treating a subject having a cancer that overexpresses and/or secretes a serpin. In certain non-limiting embodiments, the activity of a cell adhesion molecule associated with blood vessel cooption is inhibited. For example, the activity of L1CAM is inhibited. In other non-limiting embodiments, the serpin itself is inhibited.

Certain non-limiting embodiments include:
In a subject having a cancer, a method of inhibiting metastatic spread of the cancer, comprising determining whether a cell of the cancer overexpresses a serpin and, if the cell does overexpress the serpin, treating the subject with a therapeutic amount of a L1CAM inhibitor.
- The above method where the serpin is neuroserpin, serpin B2, serpin D1 or serpin E2.
- The above method where the cancer is breast cancer.
- The above method where the cancer is lung cancer.
- The above method where the L1CAM inhibitor is an immunoglobulin.
- The above method where the L1CAM inhibitor is an interfering RNA.
- The above method where the metastasis inhibited is metastasis to the brain.
- The above method where the metastasis inhibited is metastasis to the lung.
- The above method where the metastasis inhibited is metastasis to the liver.
- The above method where the metastasis inhibited is metastasis to a bone.

A method of treating a subject suffering from a cancer, comprising (i) determining whether the subject is at increased risk for metastatic spread of the cancer, comprising determining whether a cell of the cancer overexpresses a serpin, where overexpression of the serpin indicates that the subject is at higher risk of metastatic spread of the cancer; and (ii) where the subject is at increased risk of metastasic spread, performing or recommending a treatment modality that would inhibit the growth or development of a metastasis and thereby reduce the risk of metastatic spread of the cancer.
- The above method where the serpin is neuroserpin, serpin B2, serpin D1 or serpin E2.
- The above method where the cancer is breast cancer.
- The above method where the cancer is lung cancer.
- The above method where the risk is for metastasis to the brain.
- The above method where the risk is for metastasis to the lung.
- The above method where the risk is for metastasis to the liver.
- The above method where the risk is for metastasis to a bone.

A method of determining whether the subject is at increased risk for metastatic spread of the cancer, comprising determining whether a cell of the cancer overexpresses a serpin, where overexpression of the serpin indicates that the subject is at increased risk of metastatic spread of the cancer, and informing the subject or a health care worker of the result of the determination and the associated risk.
- The above method where the serpin is neuroserpin, serpin B2, serpin D1 or serpin E2.
- The above method where the cancer is breast cancer.
- The above method where the cancer is lung cancer.
- The above method where the risk is for metastasis to the brain.
- The above method where the risk is for metastasis to the lung.
- The above method where the risk is for metastasis to the liver.
- The above method where the risk is for metastasis to a bone.

A kit for determining whether a subject having a cancer is at increased risk for metastatic spread of the cancer, comprising means for determining whether a cell of the cancer overexpresses a serpin, and instructional material that indicates that overexpression of the serpin indicates that the subject is at higher risk of metastatic spread of the cancer.
- The above kit where the serpin is neuroserpin, serpin B2, serpin D1 or serpin E2.
- The above kit where the means for determining whether a cell overexpresses a serpin comprises an immunoglobulin or a fragment thereof.
- The above kit where the means for determining whether a cell overexpresses a serpin comprises a pair of PCR primers.

In a subject having a cancer, a method of inhibiting metastatic spread of the cancer, comprising determining whether a cell of the cancer (i) overexpresses a serpin and (ii) expresses L1CAM and, if the cell does overexpress the serpin and expresses L1CAM, treating the subject with a therapeutic amount of a L1CAM inhibitor.
- The above method where the serpin is neuroserpin, serpin B2, serpin D1 or serpin E2.
- The above method where the cancer is breast cancer.
- The above method where the cancer is lung cancer.
- The above method where the L1CAM inhibitor is an immunoglobulin.
- The above method where the L1CAM inhibitor is an interfering RNA.
- The above method where the metastasis inhibited is metastasis to the brain.
- The above method where the metastasis inhibited is metastasis to the lung.
- The above method where the metastasis inhibited is metastasis to the liver.
- The above method where the metastasis inhibited is metastasis to a bone.

A kit for determining whether a subject having a cancer is at increased risk for metastatic spread of the cancer, comprising means for determining whether a cell of the cancer overexpresses a serpin, and instructional material that indicates that overexpression of the serpin indicates that the subject is at higher risk of metastatic spread of the cancer.
- The above kit where the serpin is neuroserpin, serpin B2, serpin D1 or serpin E2.
- The above kit where the means for determining whether a cell overexpresses a serpin comprises an immunoglobulin or a fragment thereof.
- The above kit where the means for determining whether a cell overexpresses a serpin comprises a pair of PCR primers.
- The above kit, where the instructional material includes a recommendation that where the cancer cell of the subject overexpresses a serpin, the subject should be treated with an L1CAM inhibitor.

A kit for determining whether a subject having a cancer is at increased risk for metastatic spread of the cancer, comprising means for determining whether a cell of the cancer overexpresses a serpin and expresses L1CAM, and instructional material that indicates that overexpression of the serpin indicates that the subject is at higher risk of metastatic spread of the cancer and expression of L1CAM indicates that a higher risk subject may benefit from L1CAM inhibitor therapy.
- The above kit where the serpin is neuroserpin, serpin B2, serpin D1 or serpin E2.
- The above kit where the means for determining whether a cell overexpresses a serpin comprises an immunoglobulin or a fragment thereof.
- The above kit where the means for determining whether a cell overexpresses a serpin comprises a pair of PCR primers.
- The above kit where the means for determining whether a cell expresses L1CAM comprises an immunoglobulin or a fragment thereof.
- The above kit where the means for determining whether a cell expresses L1CAM comprises a pair of PCR primers.

### 4. BRIEF DESCRIPTION OF THE FIGURES

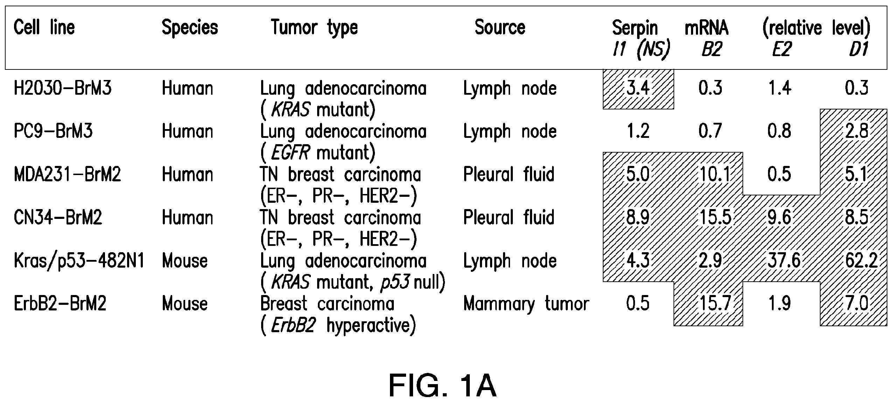

FIGURE 1A-I. Association of PA-inhibitory serpins with the brain metastatic phenotype (A) Serpin mRNA levels in brain metastatic cell lines relative to the levels in counterparts not metastatic to brain. qRT-PCR values are averages of at least three independent reactions. Sources of the metastatic cells are indicated. TN, triple negative breast cancer; ER-, estrogen receptor negative, PR-, progesterone receptor negative. (B) qRT-PCR analysis of the indicated serpins in the parental MDA231 cell line and derivatives with metastatic tropism to bone, lung, or brain. Bars in each graph are, left to right, parental, bone metastasis, lung metastasis, and brain metastasis. Error bars, 95% confidence interval. (C) Representative ex vivo bioluminescence (BLI) images of brains from immunocompetent mice inoculated with different *Kras^{G12D}; p53*^{*-*/*-*} mouse lung cancer cell lines. The percentage of mice developing brain metastasis and the mean BLI photon flux signal are indicated. n=10. (D) Heatmap of serpin mRNA expression in *Kras^{G12D};* p53^{-/-} derivatives based on qRTPCR analysis. (E) Summary of the serpin-PA-plasmin cascade. (F) Inhibition of plasminogen conversion into plasmin by cell culture supernatants of the indicated cell lines. Plasmin activity was determined by a chromogenic assay. Data are averages ± SEM from triplicate experiments. (G) Kaplan-Meier analysis of brain metastasis-free survival in 106 cases of lung adenocarcinoma classified based on *SERPINB2* and *SERPINI1* mRNA levels in the primary tumor. P value calculated from a Cox proportional hazard model, with *SERPINB2* and *SERPINI1* expression treated as a continuous variable. (H) Representative human brain metastasis samples from lung and breast cancer stained with antibodies against neuroserpin or serpin B2. (I) Proportion of metastasis samples scoring positive for neuroserpin immunostaining (red) or serpin B2 immunostaining (orange) in 33 cases of non-small cell lung carcinoma and 123 cases of breast carcinoma. Small diagrams in the breast cancer set represent the primary tumor subtype (TN, triple negative; HER2, HER2+; ER/PR, hormone receptor positive; TP, triple positive) of the serpin-positive samples for which this information was available. Brain metastases scoring positive for both serpins comprise 42% and 34% of the lung cancer and breast cancer cases, respectively. Samples scored as positive had >80% of neoplastic cells showing positive reactivity. Scale bar: 100 µm.

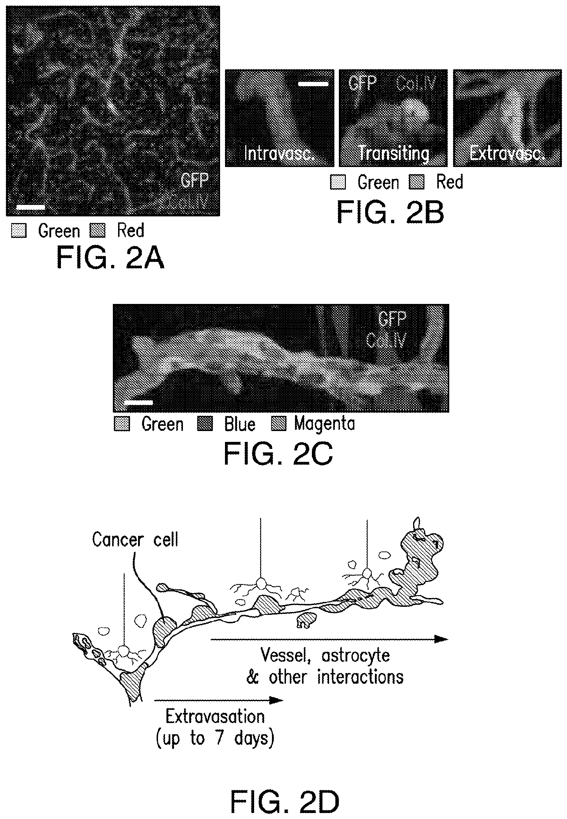

FIGURE 2A-O. Vascular cooption, outgrowth, and escape from stromal plasmin action. (A) Metastatic cell interactions with brain capillaries. MDA231-BrM2 cells (green) remain bound to brain capillaries (red) after completing extravasation. (B) Confocal analysis of the extravasation steps showing an MDA231-BrM2 cell lodged intravascularly in a brain capillary, a cell transiting through the capillary wall, and an extravasated cell that is spreading over the abluminal capillary surface. (C) Cluster of extravasated MDA231-BrM2 cells forming a furrow around a brain capillary. All extravasated cells initially grew in this manner. Red or magenta, collagen IV in vasculature. Green, GFP. Blue, nuclear staining with bis-benzamide. (D) Schema representing the initial steps and interactions during metastatic colonization of the brain. (E) Exposure of metastatic H2030-BrM3 cells to GFAP+ reactive astrocytes (arrowheads) in the brain parenchyma at different time points after inoculation of cancer cells into the circulation. Day 3: red, collagen IV; white, GFAP; green, GFP+ cancer cells. Day 7 onwards: red, GFAP; green, GFP; blue, nuclear staining. (F,G) tPA and uPA immunofluorescence staining (red, arrowheads) associated with GFAP+ astrocytes (blue) in a mouse brain harboring GFP+ H2030-BrM3 cells (green). (H) Plasminogen immunofluorescence staining (white, arrowheads) is associated with NeuN+ neuron bodies (red) near a cluster of GFP+ metastatic cells (green) in a mouse brain. Blue, nuclear staining. (I) Schema of brain slice organotypic cultures. Cancer cells placed on the surface of slices migrate into the tissue and seek microcapillaries. (J) Representative image of a brain slice harboring infiltrated H2030-BrM3 cells that are still round (open arrowheads) or already spread over brain capillaries (closed arrowheads). (K) Representative confocal images of brain slice tissue infiltrated with the indicated cancer cells, α2-antiplasmin was added to the indicated cultures. Note the lower density and isorganized aspect of parental cells compared with the stretched morphology of BrM3 cells or parental cells with α2-antiplasmin. (L) Quantification of GFP+ cancer cells in the experiments of panel K. Number of cells per field of view (FOV) are averages + SEM. n=6-10 brain slices, scoring at least two fields per slice, in at least 2 independent experiments. (M) Cleaved caspase-3 immunofluorescence staining in brain slices harboring the indicated cells and additions. (N) Quantification of cleaved caspase-3 positive cancer cells in the experiments of panel M. Values are normalized to H2030-BrM3, and are averages ±SEM. n=6-10 brain slices, scoring at least two fields per slice, from at least 2 independent experiments. (O) Schematic summary showing neurons and astrocytes as sources of plasminogen and PA, respectively, and lethal effect of the resulting plasmin on infiltrating cancer cells. All P values by Student's t-test. Scale bars: 25µm (A), 5µm (B-C), 5µm (Day 3), 15µm (Day 7), 25µm (Day 14), 70µm (micrometastasis), 100µm (macrometastasis) (D), 10µm (F-H), 100µm (K), 5µm (M).

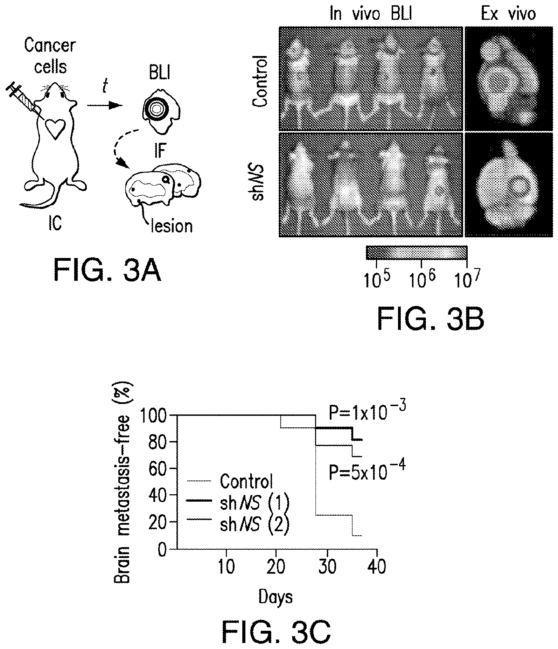

FIGURE 3A-N. Neuroserpin mediates brain metastasis (A) Schema of experimental design. Brain metastases develop in mice after inoculation of cancer cells into the arterial circulation. Brain lesions are analyzed by bioluminescence imaging (BLI) based on the expression of firefly luciferase in cancer cells and by immunofluorescence (IF) based on the expression of GFP. (B) Representative images of whole-body BLI and brain ex vivo BLI 5 weeks after inoculation of H2030-BrM3 cells transduced with control shRNA or neuroserpin shRNA (shNS). (C) Kaplan-Meier plot of brain metastasis-free survival in the experiment of panel B. Control (n=20) and two different shNS [shNS (1), n=11; shNS (2), n=13] were analyzed P values were obtained with log rank Mantel-Cox test. (D) Quantification of ex vivo BLI in brains from panel B. (E) Representative images of coronal brain sections analyzed for GFP IF 21 or 35 days after inoculation of H2030-BrM3 cells into mice. Lesion contours are marked. (F) Quantification of brain lesions according to size at 21 day time point in panel E. Control n=5, shNS n=6 brains. P value refers to size distribution. For the total number of lesions, p<0.05. (G) Quantification of brain tumor burden in the experiment of panel E. Control n=5, shNS n=6. (H) Representative images of control and *neuroserpin-depleted* H2030-BrM3 cells in brain slice assays. Insets show cleaved caspase-3 immunofluorescence. (I) Quantification of GFP+ cells in the experiment of panel H. Data are averages ± SEM. n=6-10 slices, scoring at least two fields per slice, in at least 2 independent experiments. (J) Quantification of cells that were positive for cleaved caspase-3 in the experiment of panel H. Values were normalized to the control group. Data are averages ± SEM. n=6-10 slices, scoring at least two fields per slice, from at least 2 independent experiments. (K,L) Quantification of cells that were positive for cleaved caspase-3 comparing parental and BrM cell lines, and the effect of overexpressing neuroserpin wild type or a mutant form unable to target PA (NS^{Δloop}) in parental cell lines H2030 (K) and MDA231 (L). Values were normalized to the corresponding BrM cell lines. Data are averages ±SEM. n=6-10 slices, scoring at least two fields per slice, from at least 2 independent experiments. (M) Representative ex vivo BLI images of brains and hindlimbs from mice 21 days after inoculation with PC9-BrM3. Cells were transduced with empty vector (n=5) or vectors encoding wild type neuroserpin (n=7) or £Gloop neuroserpin mutant (n=8). (N) Ratio of photon flux in brain versus bone in the experiment of panel M. Ex vivo brain mean BLI values are also shown. All P values were calculated by Student's t-test, except in panel C. Scale bar: 250µm (E), 100µm, 5µm (inset) (H).

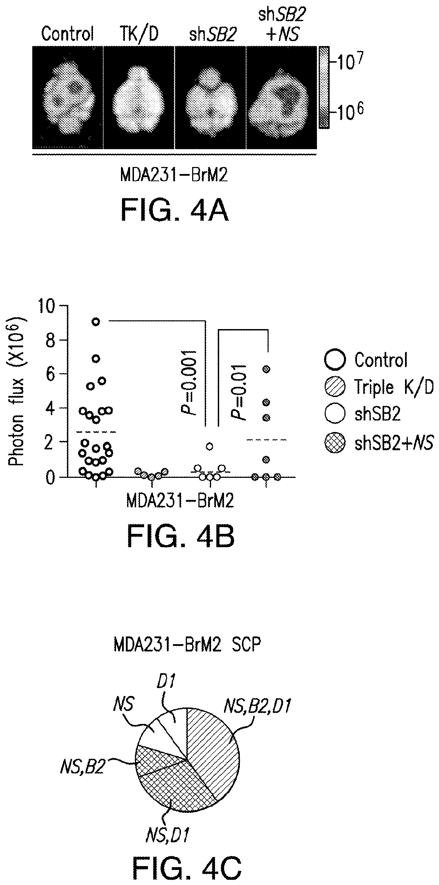

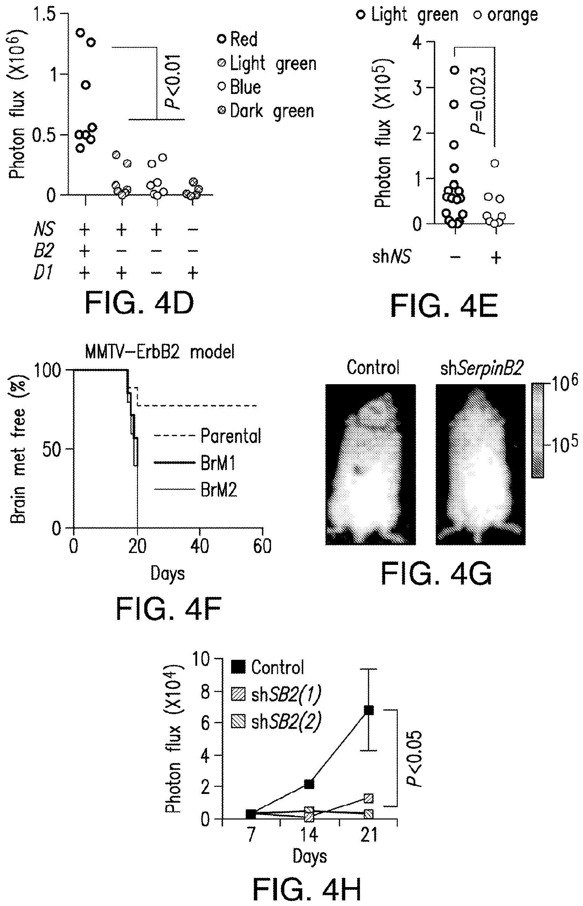

FIGURE 4A-H. Anti-PA serpins mediate brain metastasis by breast cancer cells (A,B) MDA231-BrM2 cells transduced with control vector, shRNA vectors targeting *neuroserpin, SERPINB2* and *SERPIND1* (triple K/D), *SERPINB2* shRNA (*shSB2*), or sh*SB2* plus a neuroserpin expressing vector were inoculated into the arterial circulation of immunodeficient mice. Brain metastasis burden was visualized by ex vivo brain BLI (A) and quantitated (B). Control n=22; triple K/D n=9, sh*SB2* n=14; shSB2 and neuroserpin n=8. (C) Distribution of clones (single cell progenies ¡VSCP-) overexpressing one, two, or three of the indicated serpins among ten clonal cell lines isolated from the MDA213- BrM2 population. (D) Ex vivo brain BLI quantification from different MDA231-BrM2 SCP injected. Red dots SCP (high levels of all serpins), n=8; light green dots SCP (low levels of serpin B2), n=11; blue dots SCP (low levels of serpin B2 and D1), n=8; dark green dots SCP (low levels of serpin B2 and neuroserpin), n=5. P value was determined by Student's t-test. (E) SCP with high levels of neuroserpin and serpin D1, were subjected to neuroserpin knock down and tested for brain metastatic activity. Metastatic load was quantitated by ex vivo brain BLI after 21 days. (F) Kaplan-Meier survival curves for brain metastasis-free survival in immunocompetent mice inoculated with congenic parental ErbB2-P cells (n=9) or brain metastatic derivatives ErbB2-BrM1 (n=7) and ErbB2-BrM2 (n=5). Survival curves were compared using log rank Mantel-Cox test. ErbB2-P versus ErbB2-BrMl, P=0.0045, and versus ErbB2-BrM2 P=0.0053. (G) Representative whole body BLI images of metastatic lesions formed by ErbB2-BrM2 or these cells expressing a serpin B2 shRNA. (H) Quantification of brain BLI photon flux in the experiment of panel G. Control ErbB2- BrM2 cells (n=10), and the same cells expressing two different serpin B2 shRNAs, shSB2 (1), n=10; shSB2 (2), n=6, were analyzed. Data are averages+ SEM. All P values were determined by Student's t-test, except in panel G.

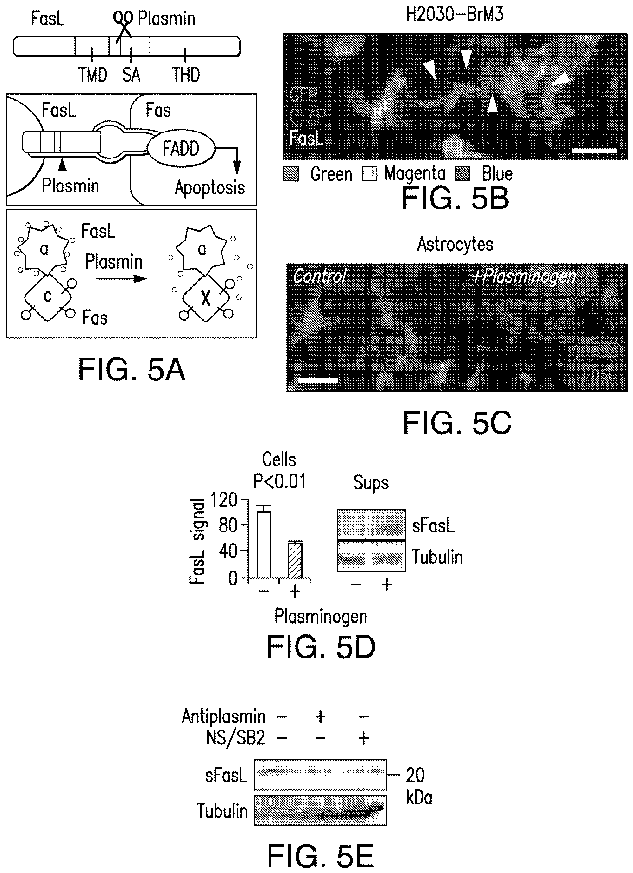

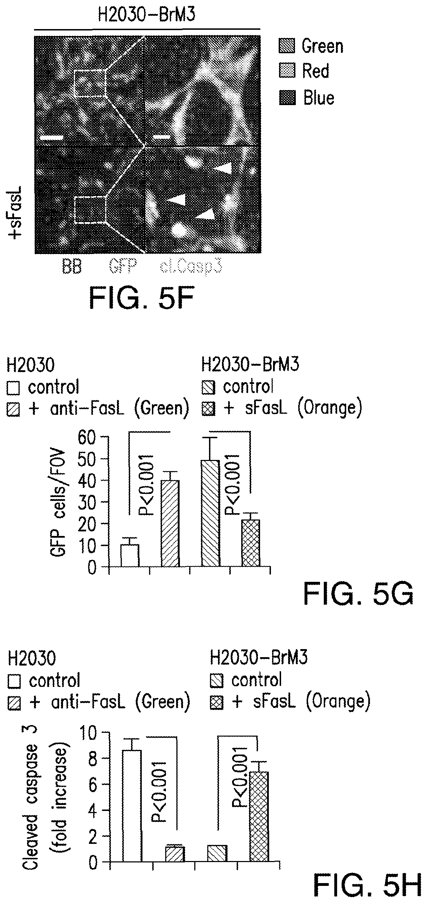

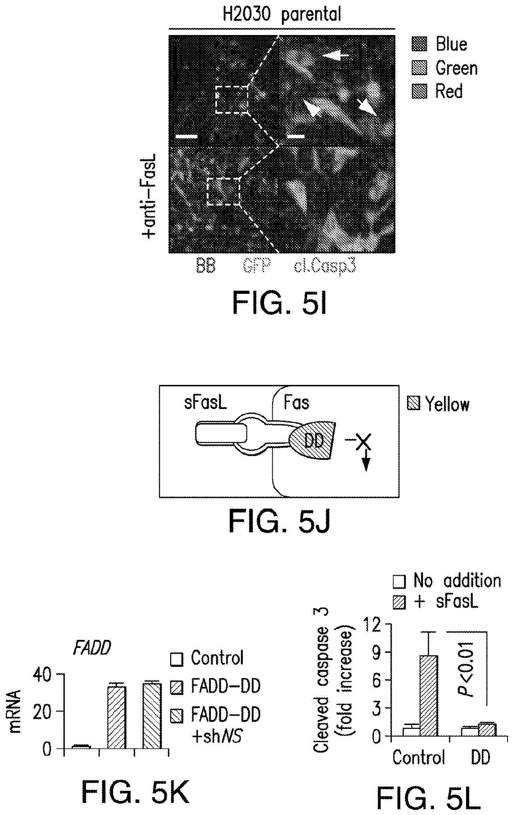

FIGURE 5A-O. Neuroserpin shields cancer cells from FasL death signals (A) Schema of FasL and its conversion by plasmin into sFasL, a diffusible trigger of apoptosis through Fas-FADD signaling. TMD, transmembrane domain; SA, trimeric selfassembly domain; THD, tumor necrosis factor-homology domain. Red crosses, apoptotic cells. a, astrocyte. c, cancer cell. (B) Immunofluorescence with antibodies against GFP (cancer cells, green), GFAP (reactive astrocytes, blue) and FasL (magenta) in a mouse brain harboring metastatic cells 21 days after arterial inoculation of H2030-BrM3. (C) Images of astrocyte cultures incubated with exogenous plasminogen (1µM) or no additions. Immunofluorescence staining was performed with antibodies against the extracellular domain (ECD) or the intracellular domain of FasL (ICD). (D) Western immunoblotting of supernatants from cultures shown in panel C, using anti- FasL ECD antibodies. Tubulin was used as loading control. (E) Mouse brain slices were incubated with α2-antiplasmin, neuroserpin and serpin B2, or no additions. sFasL in tissue lysates was detected by western immunoblotting analysis with anti-FasL ECD antibodies. (F) GFP+ H2030-BrM3 cells (green) were allowed to infiltrate brain slices in media containing added sFasL or no additions. With sFasL the cancer cells scored positive for apoptosis marker cleaved caspase-3 (red, in inset). (G, H) Quantification of total GFP+ cell numbers (G), and apoptotic GFP+ cells (H) in the experiments of panels F (orange bars) and I (green bars). Data are averages + SEM. n=6-10 slices, scoring at least two fields per slice, from at least 2 independent experiments. (I) GFP+ H2030 cells (green) were allowed to infiltrate brain slices in media containing anti-FasL blocking antibody or no additions. Anti-FasL prevented endogenous signals from triggering caspase-3 activation (red, in inset). (J) Depiction of FADD-DD overexpression (yellow shape) to suppress pro-apoptotic Fas signaling in cancer cells. (K) qRT-PCR analysis of FADD expression in H2030-BrM3 transduced with a FADD-DD vector, a *neuroserpin* shRNA vector, or empty vector, as indicated. (L) Quantification of apoptotic cells following sFasL addition to H2030-BrM3 cells transduced with the indicated vectors. (M, N) Quantification of total GFP+ cells (M), and apoptotic GFP+ cells (N) in brain slices harboring the indicated GFP+ H2030-BrM3 transfectants and/or additions. Data are averages + SEM. n=5-8 slices, scoring at least two fields per slice, from at least 2 independent experiments. (O) Brain metastatic activity of H2030-BrM3 cells transduced with the indicated vectors and inoculated into the arterial circulation of mice. BLI photon flux was quantitated in cells transduced with control shRNA (n=11), FADD-DD (n=4), *neuroserpin* shRNA (n=14), or this shRNA and FADD-DD (n=12). All P values were determined by Student's t-test. Scale bars: 25µm (B), 200µm (C), 100µm (F,I), 5µm (insets in F,I).

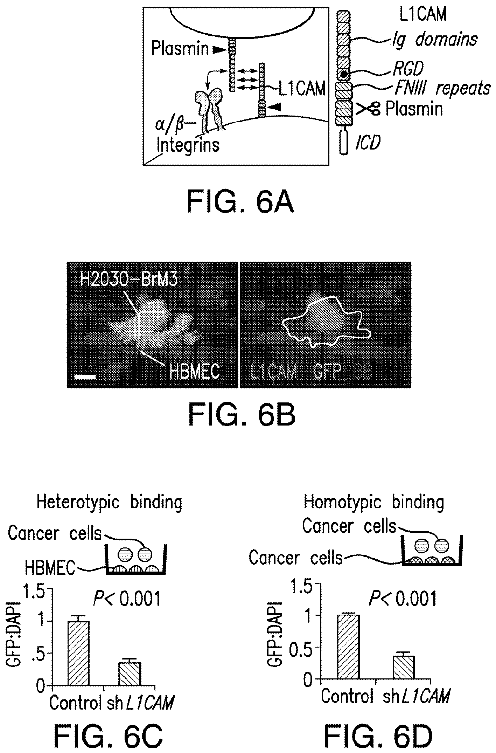

FIGURE 6A-N. The plasmin target L1CAM mediates vascular cooption by brain metastatic cells (A) Schema of L1CAM as a mediator of homophilic and heterophilic (e.g., integrins) cell adhesive interactions, and its conversion by plasmin into an adhesion defective fragment. Immunoglobulin-like (Ig) and fibronectin type III (FNIII) domain repeats, the intracellular domain (ICD), and an integrin-binding RGD sequence are indicated. (B) Suspensions of GFP+ H2030-BrM3 cells were placed on top of a monolayer of human brain microvascular endothelial cells (HBMEC), and HBMEC-bound cancer cells where imaged 20 min later for GFP, L1CAM immunostaining, and nuclear staining with bis-benzamide. L1CAM is highly expressed in the cancer cells and at lower level in the HBMECs. (C, D) Analysis of H2030-BrM3 binding to HBMEC monolayers (C) or to H2030-BrM3 monolayers (D), and effect of L1CAM knockdown. Data are averages ± SEM. n=5, scoring at least 10 fields per coverslip. (E) Flow cytometric analysis of cell-surface L1CAM in the indicated brain cells expressing *L1CAM* shRNA or incubated with plasmin, compared to untreated controls. (F) Anti-L1CAM western immunoblotting analysis of cells and culture supernatants after incubation with or without plasmin. (G,H) Cancer cells were treated with plasmin and subjected to HBMEC adhesion assays. Data are averages + SEM. n=3, scoring at least 5 fields per coverslip. (I) Control or *L1CAM-*depleted H2030-BrM3 cells after infiltrating brain tissue slices. GFP+ cancer cells (green) and vasculature (collagen IV immunostaining, red) were visualized after 2 days. Two representative images are shown per condition. Lower panels, high magnification. (J,K) Quantification of cells that were spread on capillaries (J) and Ki67+ cells (K) in the experiments of panel I. Data are averages ± SEM. n=6 slices, scoring at least three fields per slice, from 2 independent experiments. (L) Effect of neuroserpin overexpression and *L1CAM* depletion on the interaction of PC9-BrM3 cells with capillaries in brain slices. (M,N) Quantification of cells that were spread on capillaries (M) and Ki67+ cells (N) in the experiments of panel L. Data are averages + SEM. n=6 slices, scoring at least three fields per slice, from 2 independent experiments. All P values by Student's t-test. Scale bars: 10µm (B), 50µm (I,L).

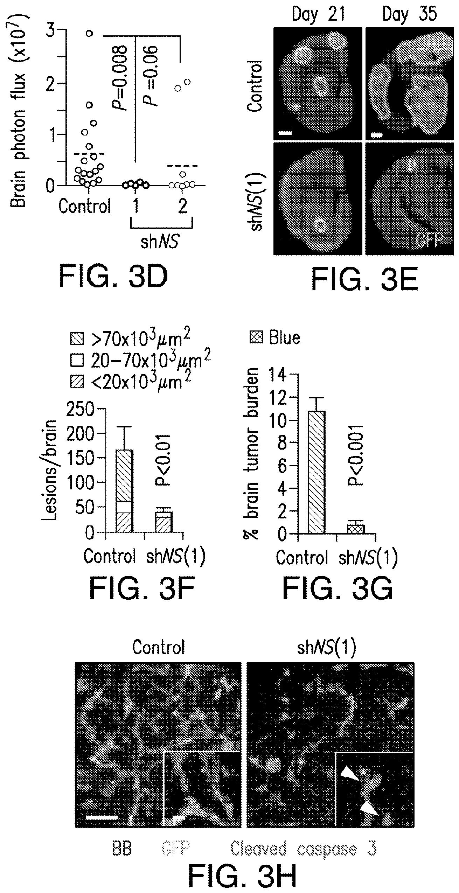

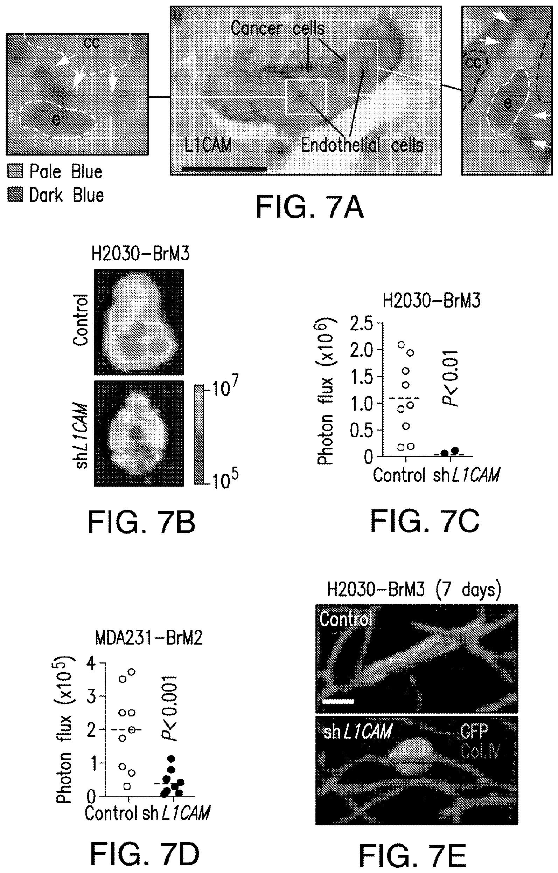

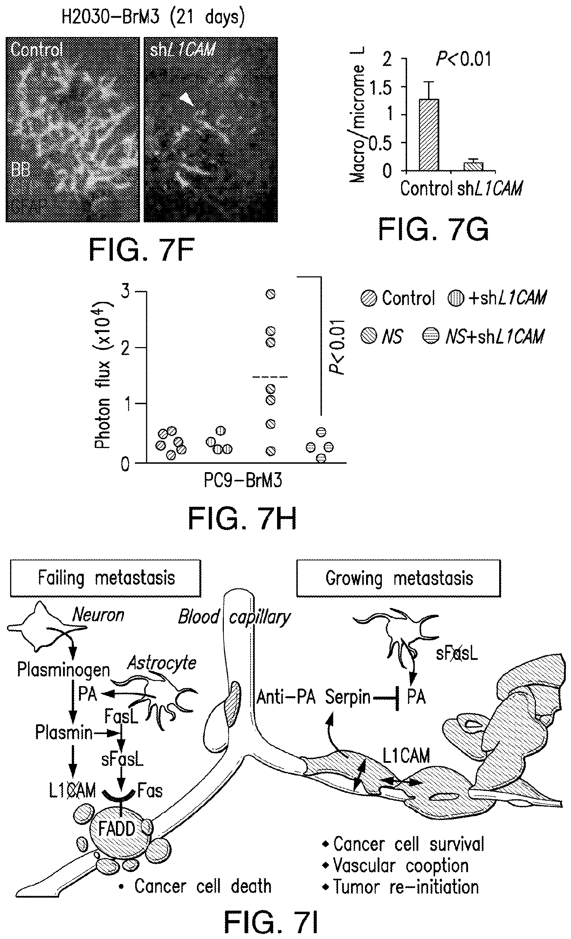

FIGURE 7A-I. L1CAM mediates metastatic outgrowth in the brain. (A) Immunohistochemical staining with anti-L1CAM antibodies and H&E counterstaining of incipient brain colonies formed by H2030-BrM3. Cancer cells (cc, pale blue nuclei) remain close to each other and interact with endothelial cells (e, dark blue nuclei). Insets, higher magnification of cell-cell contact areas. (B) Ex vivo BLI of representative brains from mice that were arterially inoculated with indicated H2030-BrM3 cells. (C) Quantification of ex vivo brain photon flux in the experiments of panel B. Control shRNA, n=9; sh*L1CAM,* n=6. (D) Quantification of ex vivo BLI of brains from mice that were arterially inoculated with indicated MDA231-BrM2 cells. Control shRNA, n=9; sh*L1CAM,* n=10. (E) H2030-BrM3 cells infiltrating the brain 7 days after intracardiac injection, and effect of *L1CAM* depletion. (F) Representative images of GFP+ metastatic lesions from brains in panel C. (G) Relative abundance of macrometastasis over micrometastasis (as defined in Figure 3F) in brains shown in panel C. Number of lesions: control= 283.2 ± 84.8, sh*L1CAM*= 69.8 ±11.5. Data are averages ±SEM. n=3 brains. (H) Quantification of ex vivo BLI photon flux of brains from mice that were arterially inoculated with the indicated PC9-BrM3 cells (n=5-7). All P values were determined by Student's t-test. Scale bar: 25µm (A), 30µm (E), 200µm (F). (I) Model of the action of the stromal PA-plasmin system against cancer cells that infiltrate the brain, and role of anti-PA serpins in protecting brain metastatic cells from stromal PA-plasmin. Reactive astrocytes produce PAs in the presence of extravasated cancer cells. Metastasis fails (left side) when PAs generate plasmin from neuron-derived plasminogen and plasmin mobilizes FasL from astrocytes to kill cancer cells. Additionally, plasmin cleaves and inactivates L1CAM, a cell adhesion molecule that cancer cells express for vascular cooption . Metastasis proceeds (right side) when brain metastatic cells express anti-PA serpins that prevent the generation of plasmin and its deleterious effects on the survival and vascular attachment of the cancer cells.

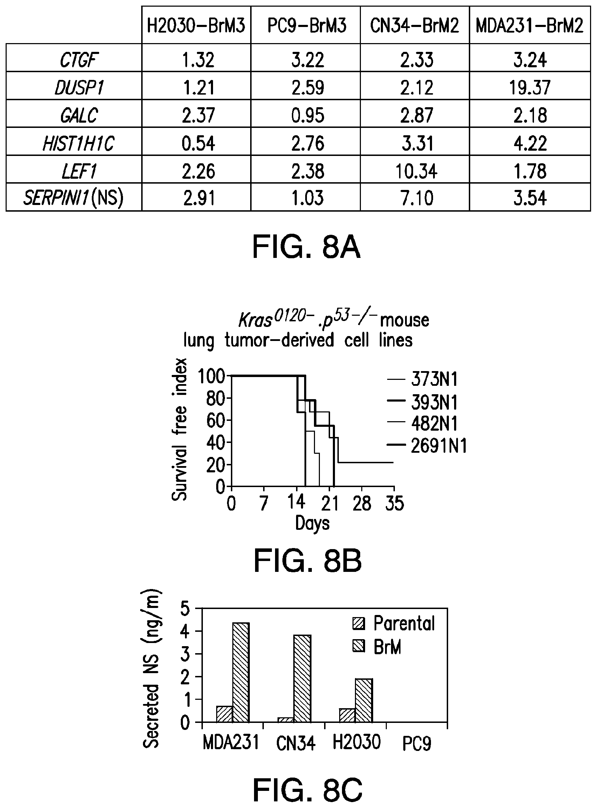

FIGURE 8A-J. Highly expressed genes in brain metastasis models and clinical samples (related to FIGURE 1A-I). (A) Genes that were previously associated with brain metastatic activity in two lung adenocarcinoma brain metastasis models (H2030-BrM3 and PC9-BrM3; Nguyen et al, 2009) or two breast cancer brain metastasis models (MDA231-BrM2 and CN34-BrM2; Bos et al, 2009) and found to be shared among these models. Values indicate fold increase in the expression of these genes in BrM cells compared to non-brain metastatic counterparts in GeneChip transcriptional data sets (Nguyen et al. 2009, Bos et al, 2009), (B) Cell lines derived from genetically engineered Kras^{G12D};p53^{-/-} mouse lung tumors (373N1, 393N1, 482N1, 2691N1) were tested for overall metastatic activity from the arterial circulation in syngeneic mice. Kaplan-Meier plots of metastasis-free survival, n=10 mice per cell line. All cell lines showed multiorganic metastatic activity as previously described (Winslow et al, 2011), but differed in brain metastatic activity. (C) Neuroserpin (NS) protein levels in low-serum cell culture supernatants, as determined by ELISA. (D) Serpin B2 (SB2) protein levels in cell lysates determined by western immunoblotting. (E) Plasminogen conversion into plasmin was inhibited to different extents by cell culture supernatants from the indicated cells lines. Plasmin activity was determined by a chromogenic assay. P values were determined by Student's t-test. * P<0.05, *** P<0.001. (F,G) Kaplan-Meier analysis of bone metastasis-free survival and contralateral lung metastasis-free survival in 106 cases of lung adenocarcinoma classified based on *SERPINB2* and *SERPINI1* mRNA levels in the primary tumor. P value calculated by a Cox proportional hazard model, with *SERPINB2* and *SERPINI1* expression treated as a continuous variable. (H) Kaplan-Meier analysis of brain metastasis-free survival in 615 cases of breast adenocarcinoma (EMC-MSK dataset) classified based on *SERPINB2* and *SERPINI1* mRNA levels in the primary tumor. P value calculated from a Cox proportional hazard model, with *SERPINB2* and *SERPINI1* expression treated as a continuous variable. (I) Immunohistochemistry against NS and SB2 in brains from mice intracardiacally inoculated with indicated cell lines. (J) Representative brain metastasis tissue microarray cores stained with neuroserpin or serpin B2 antibodies. Scale bars: 100µm.

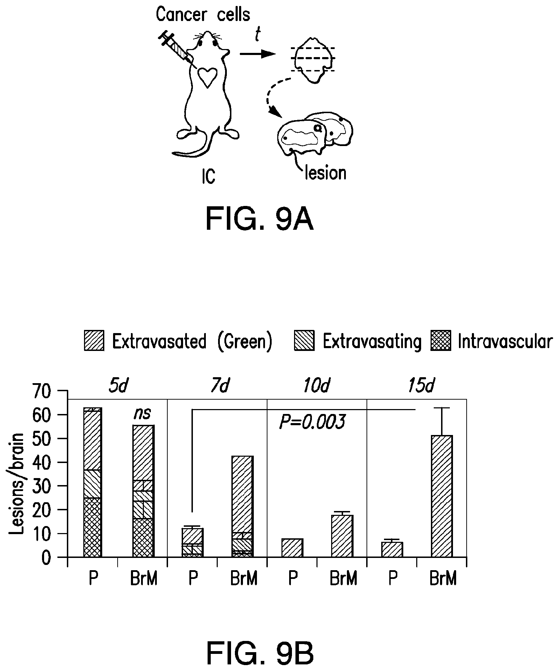

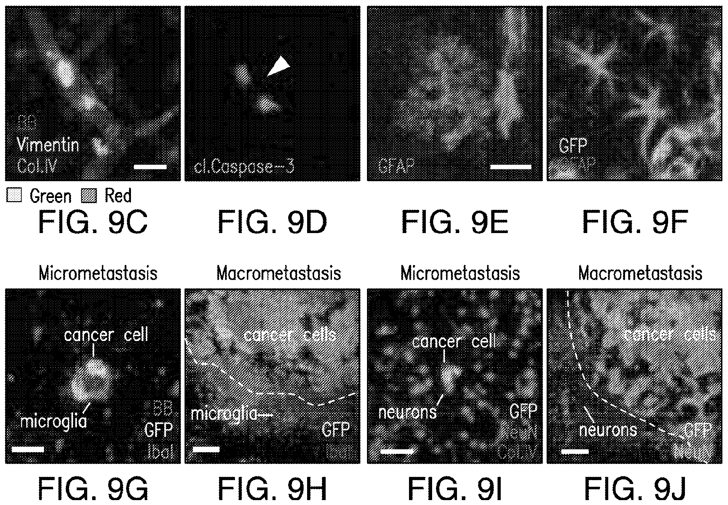

FIGURE 9A-P. Interactions of metastatic cells with the brain parenchyma (related to FIGURE 2A-O). (A) Experimental design for the analysis of brain metastatic colonies formed by circulating cancer cells. (B) Quantification of parental MDA231 (P) and MDA231-BrM2 (BrM) cells in the brain at the indicated times after inoculation into the arterial circulation of mice. Data are averages ± SEM of multiple fields in 2 brains. P values were determined by Student's t test comparing cells extravasated in the parental and brain metastatic populations before and after 7 days. (C,D) Cells (vimentin+, green) that remained in the lumen of brain capillaries (collagen IV positive) 7 days after inoculation scored positive for cleaved caspase-3 immunofluorescence (red, arrowhead in D). (E,F) Non reactive astrocytes (E), located in uninvolved areas and reactive astrocytes in areas that contain metastatic cells (F) can be distinguished based on dramatic morphological changes, including modified interaction with capillaries, and the thickening and reduction in the number of cellular processes. (G-J) Interaction of H2030-BrM3 cells with different components of the brain microenvironment including reactive microglia (Iba1+) and neurons (NeuN+) from extravasation through overt metastasis. (K) D-VLK chromogenic plasmin substrate assay was used to compare the plasmin activity associated with mouse microglia or astrocyte culture supernatants. (L) Quantification of cleaved caspase-3 positive cancer cells in co-cultures with glial cells with added plasminogen. Values are normalized to H2030-BrM3 without plasminogen, and are averages ± SEM. n=6-9 co-cultures per condition, scoring multiple fields per coculture from 3 independent experiments. (M) Quantification of cleaved caspase-3 positive cancer cells in brain slice assays. Values are normalized to MDA231-BrM2, and are averages ± SEM. n=6-10 brain slices, from 2 independent experiments. (N) Schema of experimental design to analyze plasmin activity in brain slices. (O) α2-antiplasmin inhibition of plasmin in brain slices. (P) Plasmin addition to H2030 cells in monolayer culture does not induce cell death. All P values were determined by Student's t-test. Scale bars: 10µm (C), 50µm (E,F), 20µm (G,I), 500µm (H,J).

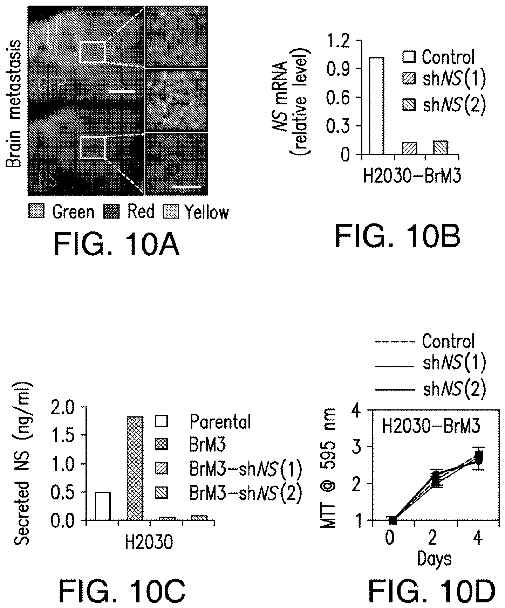

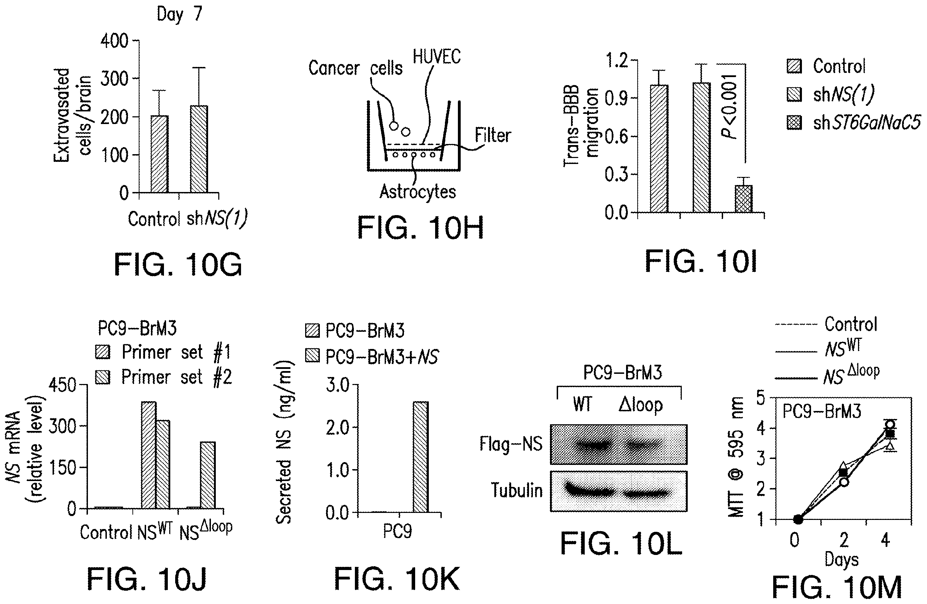

FIGURE 10A-M. Neuroserpin mediates brain metastasis by lung cancer cells. Related to FIGURE 3A-N. (A) Neuroserpin IF (red) in brains harboring GFP+ H2030-BrM3 metastasis (green). Insets show colocalization (yellow) of neuroserpin with GFP+ cancer cells. (B) *Neuroserpin* mRNA levels as determined by qRT-PCR in H2030-BrM3 and derivatives transduced with *neuroserpin* shRNAs. (C) Neuroserpin ELISA was performed on culture supernatants of the indicated H2030 derivatives. (D) MTT cell proliferation assays of H2030-BrM3 cells transduced with control or *NS* shRNA. (E) Tracings and size distribution of metastatic lesions in the brain of animals from Figure 3D. Relative abundance of each size group is shown for every experimental condition. (F) The few macrometastases that were formed by *neuroserpin*-depleted H2030-BrM3 cells scored positive for neuroserpin IF (left panels), whereas micrometastases scored negative for neuroserpin (right panels). (G) *Neuroserpin* knockdown in H2030-BrM3 did not alter the number of extravasated cells on day 7 after inoculation. Data are averages ± SEM from 3 brains. (H) Schema of the assay for cancer cell transmigration through an experimental bloodbrain barrier (BBB) (Bos et al., 2009). HUVEC, primary human umbilical vein endothelial cells. (I) Quantification of cells that migrated through this experimental BBB normalized to H2030-BrM3 control cells. An shRNA targeting the brain extravasation mediator *ST6GalNaC5* (Bos et al., 2009) served as positive control (shNS vs *shST6GalNaC5,* P<0.001). At least 5 independent migration assays were performed for each condition. (J) qRT-PCR analysis of *neuroserpin* mRNA levels in PC9-BrM3 cells that were transfected with the indicated flag epitope-tagged neuroserpin constructs. PCR primer set #1 recognizes only the wild type *neuroserpin* mRNA, whereas set #2 recognizes both the wild type and the Δloop mutant forms. (K) Neuroserpin ELISA was performed on culture supernatants of PC9-BrM3 or this cell line expressing a neuroserpin cDNA. (L) Anti-flag western immunoblotting of culture supernatants from PC9-BrM3 cells expressing the indicated neuroserpin constructs. Tubulin immunoblotting was used as loading control. (M) Proliferation assays of control and neuroserpin overexpressing PC9-BrM3 cells. All P values by Student's t-test. Scale bars: 200µm (A,F), 100µm (A, inset), 250µm (E).

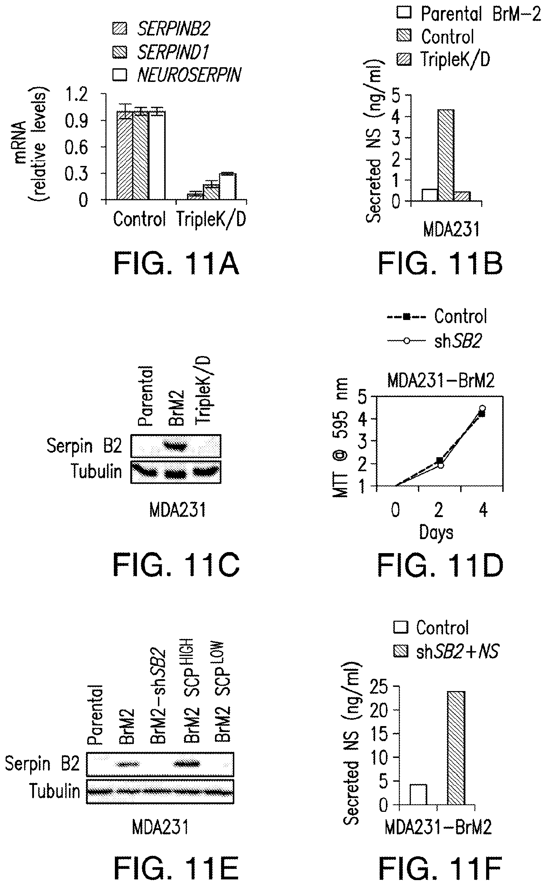

FIGURE 11A-J. Analysis of multiple coexpressed serpins in MDA231-BrM2 cells. Related to FIGURE 4A-H. (A) qRT-PCR of MDA231-BrM2 expressing shRNAs that target the three indicated serpins. (B) Neuroserpin ELISA in low serum culture supernatant from the indicated cell lines. (C) Anti-serpin B2 (SB2) western immunoblotting of lysates from the indicated cell lines. (D) Proliferation assays of sh*SB2* MDA231-BrM2 transduced cell lines. (E) Anti-serpin B2 western immunoblotting of lysates from the indicated cells lines. BrM2-SCP^{HIGH} and BrM2-SCP^{LOW} correspond to clonal lines from MDA231-BrM2 as shown in Figure 4D. (F) Neuroserpin ELISA of culture supernatant from the indicated cell lines. (G) Kaplan-Meier plot of brain metastasis-free survival comparing MDA231-BrM2 control (n=5) and sh*NS* (1) (n=9). P values by log rank Mantel-Cox test. (H) Kaplan-Meier plot of brain metastasis-free survival comparing CN34-BrM2 control (n=12) and sh*SERPINE2* (1) (n=5) were analyzed. P values by log rank Mantel-Cox test. (I) qRT-PCR analysis of serpin expression in single-cell progenies (SCP) isolated from the MDA231-BrM2 cell line. Black, clonal lines; blue, parental population; orange, BrM population. (J) Ex vivo BLI images of representative brains from metastasis assays of MDA231- BrM2 SCP expressing the indicated serpins quantified in Figure 4D.

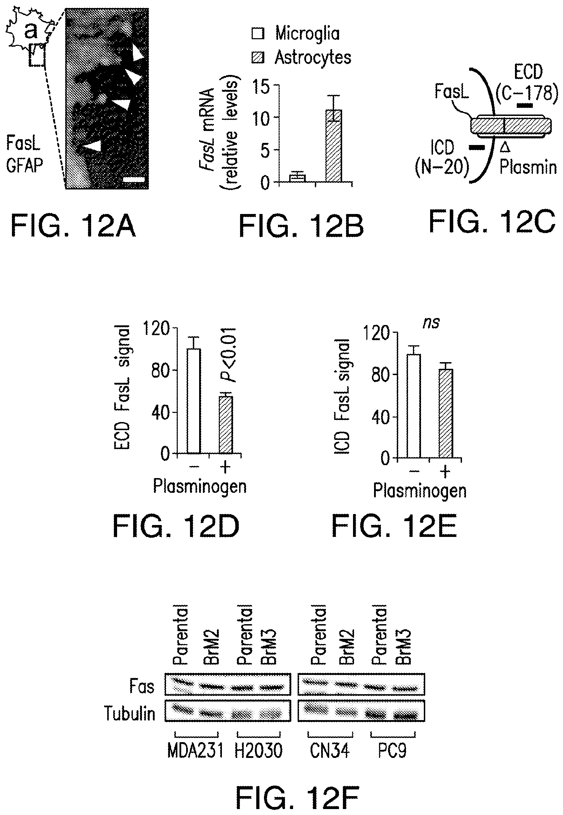

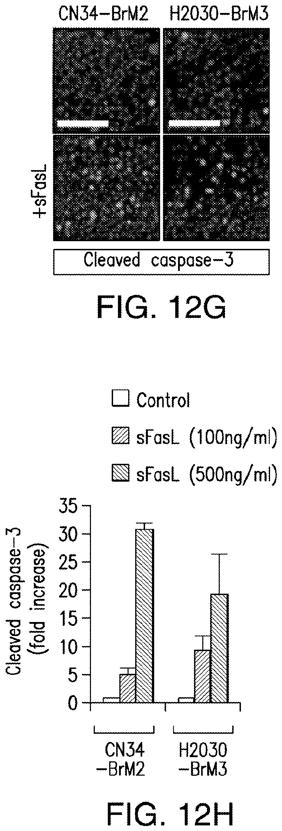

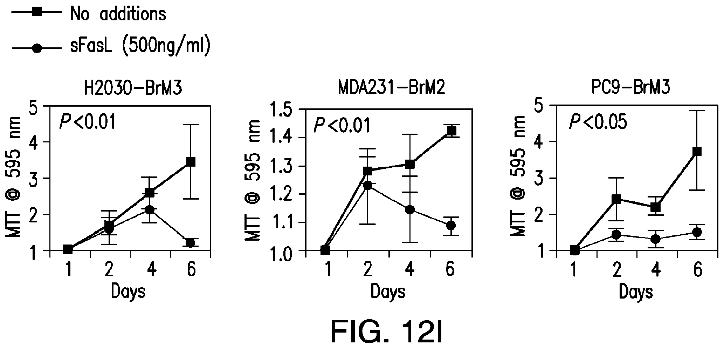

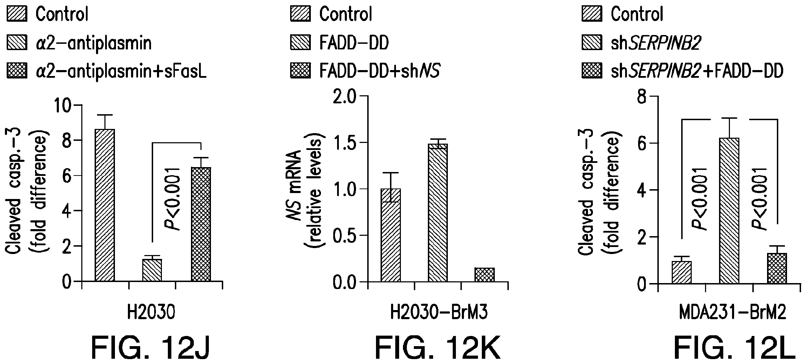

FIGURE 12A-L. sFasL triggers apoptosis in brain metastatic cells. Related to Figure 5A-O. (A) High magnification of an astrocyte stained with anti-GFAP and anti-FasL antibodies in a brain lesion formed by H2030-BrM3 cells. (B) qRT-PCR analysis of *FasL* mRNA levels in primary cultures of mouse astrocytes and microglia. Data are averages of triplicates ± SEM. (C) Schema showing the various anti-FasL antibodies used. (D,E) Quantification of FasL ECD and ICD immunofluorescence signals in Figure 5C. (F) Anti-Fas western immunoblotting of indicated cell lysates. Tubulin immunoblotting was used as loading control. (G) Cleaved caspase-3 IF (red) in breast (CN34-BrM2) and lung (H2030-BrM3) brain metastatic cell monolayers that were incubated with or without addition of sFasL (500ng/ml). Blue, nuclear staining. (H) Quantification of the experiment shown in panel G, at the indicated concentrations of sFasL. Data are averages ± SEM of three independent experiments. All differences with control were P<0.01, as determined by Student's t-test. (I) Cell proliferation assays of the indicated cell lines with or without addition of sFasL (500ng/ml). Data are averages of triplicates ± SEM. P values were determined for the difference on day 6, by Student's t-test. (J) Quantification of cleaved caspase-3 positive H2030 cancer cells in brain slices treated with added α2-antiplasmin, α2-antiplasmin and sFasL, or no additions. Values are normalized to H2030+ α2-antiplasmin, and are averages ± SEM. n=5-8 brain slices, scoring at least two fields per slice, from at least 2 independent experiments. (K) qRT-PCR analysis of *neuroserpin* mRNA levels in H2030-BrM3 cells that were transduced with FADD-DD and *neuroserpin* shRNA vectors as indicated. (L) Quantification of cleaved caspase-3 positive MDA231-BrM2 cancer cells in brain slices. MDA231-BrM2 cells were transduced with a control vector, serpin B2 (*SB2*) shRNA, or this shRNA plus a FADD-DD expression vector. Values are normalized to MDA231-BrM2 control, and are averages ± SEM. n=6-10 brain slices, scoring at least two fields per slice, from at least 2 independent experiments. All P values by Student's t-test. Scale bars: 1µm (A), 100µm (G).

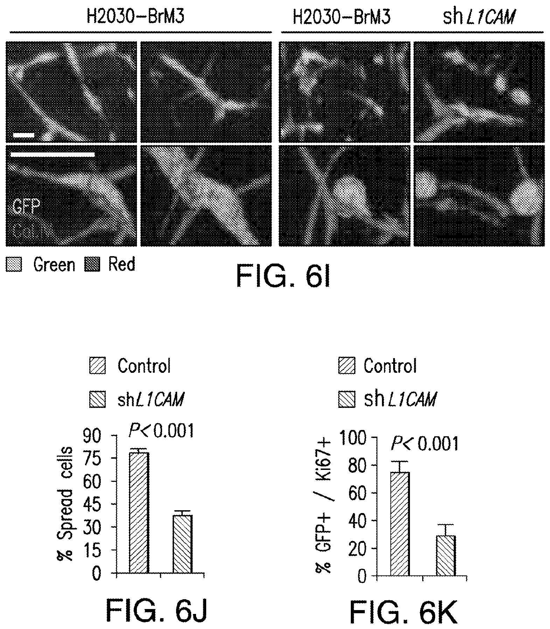

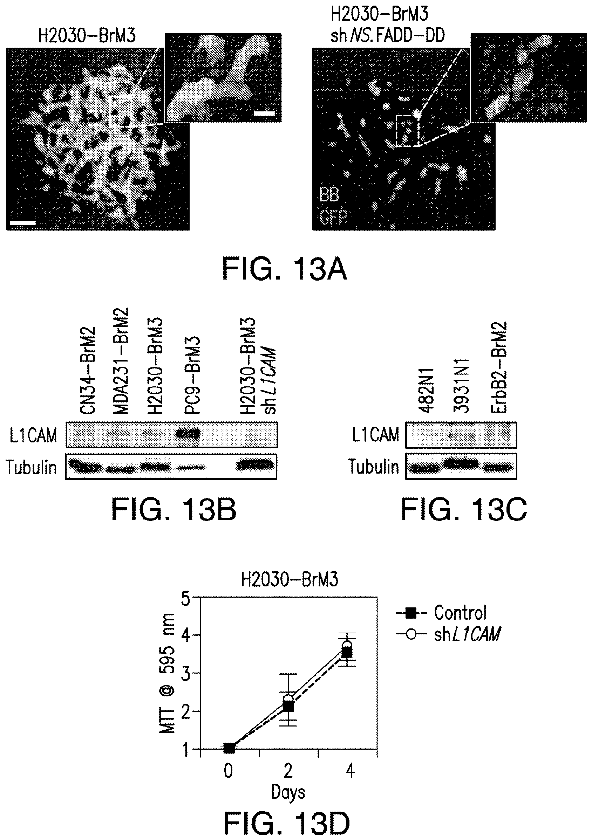

FIGURE 13A-I. L1CAM as a plasmin target and a mediator of brain metastasis. Related to FIGURE 6A-N. (A) Representative GFP IF images from brains harboring lesions formed by the indicated cell lines. Scale bar: 100µm, 25 µm (insets). (B,C) Anti-L1CAM western immunoblotting of cell lysates for the indicated human (B) and murine (C) lung or breast cancer cell lines. (D) MTT proliferation assay of control and L1CAM-depleted H2030-BrM3 cells. (E) Quantification of GFP+ cancer cells that were in contact with, but not necessarily spread on brain capillaries in the experiments of Figure 6I. Ten fields (>180 individual cells) were scored per condition. Data are averages ± SEM. (F) Quantification of apoptotic wild type or L1CAM-depleted H2030-BrM3 cells in brain slice assays. Data are averages ± SEM. n=5-8 slices from two independent experiments, and at least two fields were scored per slice. (G) Quantification of MDA231-BrM2 control or shL1CAM transduced cells that were spread on capillaries. Data are averages ± SEM. n=6 slices, scoring at least three fields per slice, from 2 independent experiments.(H,I) qRT-PCR analysis of L1CAM (H) and *neuroserpin* (I) mRNA levels in PC9-BrM3 cells that were transduced with neuroserpin expression vector and/or *L1CAM* shRNA vector as indicated.

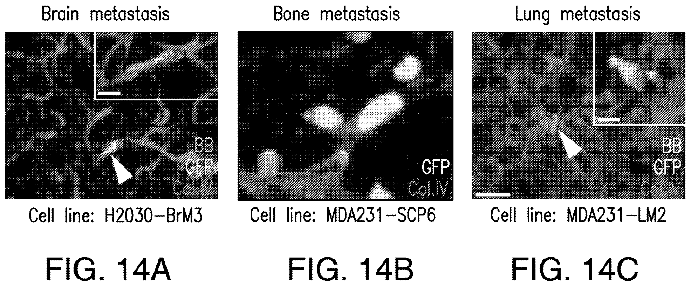

FIGURE 14A-C. Vascular co-option in metastasis initiation by GFP-bearing tumor cells in (A) brain (where the cancer cells are lung adenocarcinoma), (B) bone and (C) lung (in both cases, the cancer cells are claudin-low triple-negative breast adenocarcinoma.

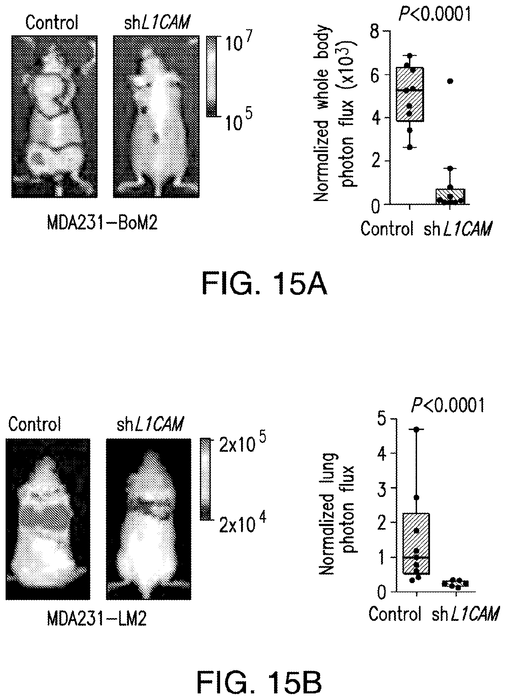

FIGURE 15A-F. RNAi-mediated L1CAM depletion inhibits formation of (A) bone and (B) lung metastases from MDA231 breast cancer cells following intracardiac injection in athymic mice; (C) tumor growth at the site of orthotopic lung injection of H2030 lung cancer cells or (D) metastasis to the contralateral lung; (E) tumor growth at the site of mammary fat pad injection of MDA231 cells or (F) metastasis to lungs or liver.

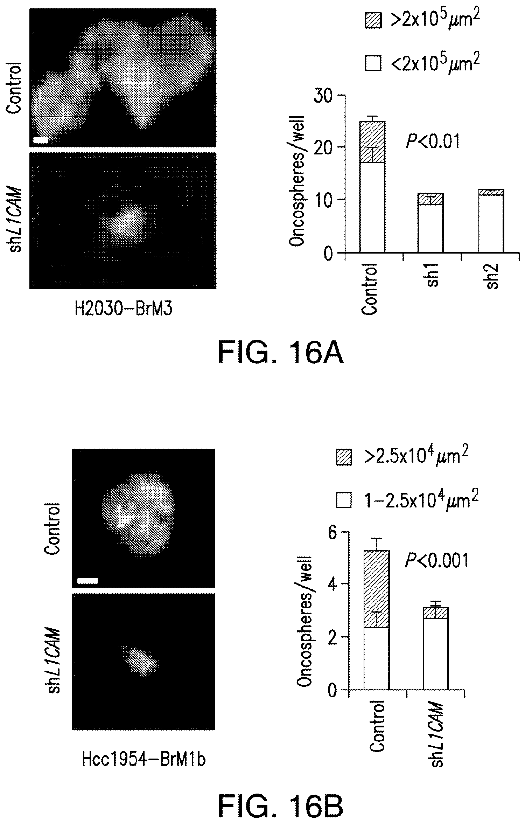

FIGURE 16A-B. L1CAM-depletion inhibits the growth of cell-cell interaction enriched oncosphere aggregates derived from (left) lung or (right) breast cancer cells grown in defined adhesion-free conditions.

### 5. DETAILED DESCRIPTION OF THE INVENTION

For clarity of description, and not by way of limitation, the detailed description of the invention is divided into the following subsections:
(i) metastasis-associated serpins;
(ii) methods and kits for assessing risk; and
(iii) methods of treatment, including
   (a) inhibition of L1CAM; and
   (b) inhibition of serpin.

### 5.1 METASTASIS-ASSOCIATED SERPINS

Serpins which may be used according to the invention include serpins originating in a human or a non-human subject, for example, but not limited to, a non-human primate, a mouse, a rat, a hamster, a guinea pig, a rabbit, a dog, a cat, a pig, a horse, a sheep, a goat, a cow, or a cetacean.

In certain non-limiting embodiments, a human serpin associated with an increased risk of metastasis may be neuroserpin, serpin B2, serpin E1, serpin E2, or serpin D1. In specific non-limiting examples, the neuroserpin may be a human neuroserpin having an amino acid sequence as set forth in GenBank Accession No. AAG01089 and/or encoded by a nucleic acid having a sequence as set forth in GenBank Accession Nos. AF248244, AF248245 and/or AF248246. In specific non-limiting examples, the serpin B2 may be a human serpin B2 having an amino acid sequence as set forth in NCBI Accession No. NP_001137290 and/or encoded by a nucleic acid having a sequence as set forth in NCBI Accession No. NM_001143818. In specific non-limiting examples, the serpin E1 may be a human serpin E1 having an amino acid sequence as set forth in UniProtKB Accession No. P05121 and/or encoded by a nucleic acid having a sequence as set forth in GenBank Accession No. M14083. In specific non-limiting examples, the serpin E2 may be a human serpin E2 having an amino acid sequence as set forth in UniProtKB Accession No. P07093 and/or encoded by a nucleic acid having a sequence as set forth in GenBank Accession No. NM_006216; NM_001136528.1, or NM_001136530.1. In specific non-limiting examples, the serpin D1 may be a human serpin D1 having an amino acid sequence as set forth in UniProtKB Accession No. P05546 or NCBI Accession No. NM_000185 and/or encoded by a nucleic acid having a sequence as set forth in GenBank Accession Nos. M12849 and M19241 and/or NCBI Accession No. NM_000185. Versions of these serpins from non-human species are known and their amino acid and nucleic acid sequences are publicly available.

In certain non-limiting embodiments, a serpin which may be used according to the invention is a serpin which selectively inhibits plasminogen activator. Serpins additional to those listed above which may be used according to the invention may be identified based on their ability to inhibit plasminogen activator.

### 5.2 METHODS AND KITS FOR ASSESSING RISK

In certain non-limiting embodiments, the present invention provides for methods and compositions for determining whether a subject having a cancer is at increased risk of having or developing a metastasis of the cancer comprising determining whether a cell of the cancer overexpresses and/or secretes a serpin, as set forth in the section above, where if the cancer cell overexpresses and/or secretes a serpin, then the subject is at increased risk of having or developing a metastasis of the cancer, for example, a metastasis to the brain. Non-limiting examples of other organs that may be sites for metastasis in the context of increased risk include lung, liver, and bone.

A metastasis is a population of cancer cells at a location that is not physically contiguous with the original location of the cancer.

A subject may be a human or a non-human subject, for example, but not limited to, a non-human primate, a mouse, a rat, a hamster, a guinea pig, a rabbit, a dog, a cat, a pig, a horse, a sheep, a goat, a cow, or a cetacean.

An increased risk is a risk which is greater than that of a subject having a cancer which does not overexpress and/or secrete a plasminogen activator inhibiting serpin.

Overexpression means expression significantly greater than occurs in a non-malignant cell of the tissue of origin, for example, greater expression in ductal adenocarcinoma of the breast relative to non-malignant breast duct cells. In non-limiting examples, overexpression may be at least 20 percent greater, or at least 50 percent greater, than expression in a comparable normal cell.

Expression may be measured by any method known in the art. In non-limiting examples, expression of serpin protein may be measured using immunoglobulin-mediated techniques, for example enzyme-linked immunosorbent assay (ELISA), immunohistochemistry, immunofluorescence, and/or immunoblotting (e.g., see the working example below), by measuring plasminogen activator inhibitory activity, or other techniques known in the art. In other non-limiting examples, expression of serpin may be measured via mRNA expression, for example, using techniques such as qPCR, Northern Blot, dot blot, or other techniques known in the art.

The method may further include informing the subject or a health care worker of the result of the determination and the associated risk.

The method may further include, where an increased risk is indicated, recommending or performing an additional diagnostic procedure, for example an imaging study, to determine whether the subject has detectable metastatic disease. Non-limiting examples of imaging modalities include magnetic resonance imaging, computerized tomography and positron emission tomography.

In certain embodiments, the invention provides for a kit for determining whether a subject having a cancer is at increased risk of having or developing a metastasis of the cancer.
Non-limiting examples of types of kits include, but are not limited to, arrays/microarrays, serpin-specific antibodies and beads, which may contain one or more primer, probe, antibody, or other detection reagent(s) for detecting one or more serpin.

In non-limiting embodiments, the present invention provides for a kit for determining whether a subject having a cancer is at increased risk of having or developing a metastasis of the cancer, for example, but not limited to, a brain metastasis, comprising a means for detecting the protein level of a serpin, for example neuroserpin, serpin B2, serpin E1, serpin E2 and/or serpin D1.

In non-limiting embodiments, a kit may comprise at least one antibody for immunodetection of a serpin(s) to be identified. Antibodies, both polyclonal and monoclonal, including molecules comprising an antibody variable region or subregion thereof, specific for a serpin, may be prepared using conventional immunization techniques, as will be generally known to those of skill in the art. The immunodetection reagents of the kit may include detectable labels that are associated with, or linked to, the given antibody or antigen itself. Such detectable labels include, for example, chemiluminescent or fluorescent molecules (rhodamine, fluorescein, green fluorescent protein, luciferase, Cy3, Cy5, or ROX), radiolabels (3H, 35S, 32P, 14C, 131I) or enzymes (alkaline phosphatase, horseradish peroxidase). Alternatively, a detectable moiety may be comprised in a secondary antibody or antibody fragment which selectively binds to the first antibody or antibody fragment (where said first antibody or antibody fragment specifically recognizes a serpin).

In a further non-limiting embodiment, a serpin-specific antibody may be provided bound to a solid support, such as a column matrix, an array, or well of a microtiter plate. Alternatively, the support may be provided as a separate element of the kit.

In certain embodiments, types of kits include, but are not limited to, packaged probe and primer sets (e.g. TaqMan probe/primer sets), which may further contain one or more probes, primers, or other detection reagents for detecting one or more serpin, for example neuroserpin, serpin B2, serpin E1, serpin E2 or serpin D1.

In a specific, non-limiting embodiment, a kit may comprise a pair of oligonucleotide primers, suitable for polymerase chain reaction (PCR) or nucleic acid sequencing, for detecting the serpin(s) to be identified. A pair of primers may comprise nucleotide sequences complementary to a serpin set forth above, and be of sufficient length to selectively hybridize with said serpin. Multiple serpin-specific primers may be included in the kit to simultaneously assay a plurality of serpins. The kit may also comprise one or more polymerases, reverse transcriptase, and nucleotide bases, wherein the nucleotide bases can be further detectably labeled.

In non-limiting embodiments, a primer may be at least about 10 nucleotides or at least about 15 nucleotides or at least about 20 nucleotides in length and/or up to about 200 nucleotides or up to about 150 nucleotides or up to about 100 nucleotides or up to about 75 nucleotides or up to about 50 nucleotides in length.

In a further non-limiting embodiment, an oligonucleotide primer may be immobilized on a solid surface or support, for example, on a nucleic acid microarray, and optionally the position of each oligonucleotide primer bound to the solid surface or support is known and identifiable.

In a specific, non-limiting embodiment, a kit may comprise at least one nucleic acid probe, suitable for in situ hybridization or fluorescent in situ hybridization, for detecting the serpin to be identified.

In one specific non-limiting embodiment, a kit may comprise one or more of: a probe, primers, microarray, antibody or antibody fragment suitable for detecting one or more serpin, for example neuroserpin, serpin B2, serpin E1, serpin E2 and/or serpin D1.

In certain non-limiting embodiments, a kit may comprise one or more detection reagents and other components (e.g. a buffer, enzymes such as alkaline phosphatase, antibodies, and the like) necessary to carry out an assay or reaction to determine the expression levels of a biomarker.

In certain embodiments, a kit may comprise a means for detecting and or measuring inhibition of plasminogen activator. Such a kit may comprise plasminogen activator, plasminogen, and optionally a means for detecting cleavage of plasminogen to plasmin.

In certain embodiments, a kit, in addition to a means for detecting and/or measuring serpin expression as described above, may further comprise a means for detecting and/or measuring expression of L1CAM, and as such may comprise a probe, primers, microarray, antibody or antibody fragment suitable for detecting L1CAM expression. Such determination may be useful where therapy utilizing antagonism of L1CAM is contemplated.

A kit may further include instructions for using the kit. Said instructions may include disclosure that if a cancer cell overexpresses and/or secretes a serpin (for example, neuroserpin, serpin B2, serpin E1, serpin E2 or serpin D1, then the subject is at increased risk of having or developing a metastasis of the cancer, for example, but not limited to, metastasis to the brain. Where an L1CAM detecting element is included, said instructions may further include disclosure that a cancer cell expressing a serpin as well as L1CAM may benefit from L1CAM antagonism. In certain embodiments the instructions may recommend that where overexpression of serpin in a cancer cell of a subject is detected, the subject may benefit from inhibition of L1CAM, particularly if it is found that the cancer cell also expresses L1CAM or overexpresses L1CAM relative to its normal counterpart.

### 5.3 METHODS OF TREATMENT

In certain embodiments, the invention provides for a method of treating a subject suffering from a cancer, comprising (i) determining whether the subject is at increased risk for metastatic spread of the cancer, comprising determining whether a cell of the cancer overexpresses a serpin, where overexpression of the serpin indicates that the subject is at higher risk of metastatic spread of the cancer; and (ii) where the subject is at increased risk of metastasic spread, performing or recommending a treatment modality that would inhibit the growth or development of a metastasis.

A treatment modality may include a standard chemotherapy or radiation therapy and/or a therapy according to the invention as described in the sections below.

### 5.3.1 INHIBITION OF L1CAM

In certain non-limiting embodiments, a treatment modality to inhibit the growth or development of a metastasis comprises a means for inhibiting L1CAM-mediated cooption of blood vessels, for example by administering, to a subject identified as being at risk as set forth above, a L1CAM inhibitor. An L1CAM inhibitor is an agent that reduces the ability of L1CAM to co-opt blood vessels and /or reduces the ability of L1CAM to promote tumor growth. An L1CAM inhibitor may act, for example and not by way of limitation, by reducing expression of L1CAM in the cancer cell or removing L1CAM from the cancer cell surface or binding to L1CAM such that its ability to bind to an endothelial cell is reduced, for example by reducing the amount of L1CAM available for endothelial cell binding or by physical inhibition.

In certain non-limiting embodiments, such treatment is administered to a subject having a cancer where a cell of the cancer expresses both a serpin and L1CAM. In certain non-limiting embodiments the invention provides for, in a subject having a cancer, a method of inhibiting metastatic spread of the cancer, comprising determining whether a cell of the cancer (i) overexpresses a serpin and (ii) expresses L1CAM and, if the cell does overexpress the serpin and expresses L1CAM, treating the subject with a therapeutic amount of a L1CAM inhibitor.

In non-limiting embodiments, where the subject is a human, L1CAM to be inhibited is human L1CAM having an amino acid sequence as set forth in UniProtKB Accession No. P32004 and/or NCBI Accession Nos. NM_000425 version NM_000425.4 and/or NM_001278116 version NM_001278116.1.

In non-limiting embodiments, an L1CAM inhibitor may be an antibody or antibody fragment or single chain antibody that specifically binds to L1CAM. Non-limiting examples of such antibodies are disclosed in United States Patent No. 8,138,313, International Patent Application Publication No. WO 2007114550, and International Patent Application Publication No. WO 2008151819, as well as antibodies that compete with the antibodies described in these citations for L1CAM binding. In certain non-limiting embodiments an anti-L1CAM antibody or antibody fragment may be used to prepare a human, humanized, or otherwise chimeric antibody that is specific for L1CAM for use according to the invention. In certain non-limiting embodiments an L1CAM antibody, antibody fragment, or single chain antibody may inhibit binding of L1CAM to an endothelial cell or a blood capillary under physiologic conditions, for example in vitro or in vivo.

In non-limiting embodiments, an L1CAM inhibitor may be a nucleic acid, for example, a short hairpin, interfering, antisense, or ribozyme nucleic acid comprising a region of homology to an L1CAM mRNA. For example, such nucleic acids may be between about 15 and 50 or between about 15 and 30 or between about 20 and 30 nucleotides long, and be able to hybridize to L1CAM mRNA under physiologic conditions. A non-limiting example of a short hairpin (sh) RNA that inhibits L1CAM is set forth in the example below. In non-limiting embodiments, an L1CAM inhibitor which is a nucleic acid may be provided in a L1CAM-expressing cancer cell via a vector, for example a lentivirus, which may be selectively targeted to said cancer cell and/or wherein expression of the L1CAM inhibitor nucleic acid may be directed by a promoter which is selectively active in tumor cells or, in a specific non-limiting embodiment, a serpin promoter. Non-limiting examples of nucleic acid sequence of an L1CAM mRNA include the sequence set forth in NCBI Accession Nos. NM_000425 version NM_000425.4 and/or NM_001278116 version NM_001278116.1. In one specific non-limiting embodiment, the L1CAM inhibitor is RNAi TRCN0000063916 (The RNAi Consortium, Public TRC Portal), having a hairpin sequence 
5'-CCGGACGGCAACAACAGCAACTTTCTCGAGAAAGTTGCTGTTGTTGCC
CGTTTTTG (SEQ ID NO:1)
 and a target sequence ACGGGCAACAACAGCAACTTT (SEQ ID NO:2); or the hairpin sequence 
5'-CCGGCCACTTGTTTAAGGAGAGGATCTCGAGATCCTCTCCTTAAACAAG
TGGTTTTTG (SEQ ID NO:3)
 and a target sequence CCACTTGTTTAAGGAGAGGAT (SEQ ID NO:4); or the hairpin sequence 
5'-CCGGGCCAATGCCTACATCTACGTTCTCGAGAACGTAGATGTAGGCATT
GGCTTTTTG (SEQ ID NO:5)
 and a target sequence GCCAATGCCTACATCTACGTT (SEQ ID NO:6)

### 5.3.2 INHIBITION OF SERPIN

In certain non-limiting embodiments, a treatment modality to inhibit the growth or development of a metastasis comprises a means for inhibiting a serpin, for example, but not by way of limitation, neuroserpin, serpin B2, serpin E1, serpin E2 or serpin D1 or more particularly human neuroserpin, human serpin B2, human serpin E1, human serpin E2 or human serpin D1 . A serpin inhibitor is an agent that reduces the ability of serpin to inhibit plasminogen activator. A serpin inhibitor may act, for example and not by way of limitation, by reducing expression of the serpin in the cancer cell or binding to the serpin such that its ability to inhibit plasminogen activator is reduced.

In certain non-limiting embodiments, the invention provides for a method of inhibiting metastasis of a cancer in a subject, comprising administering, to the subject, an effective amount of a serpin inhibitor, for example an inhibitor of a serpin that inhibits plasminogen activator, for example, but not limited to, neuroserpin, serpin B2, serpin E1, serpin E2, and/or serpin D1.

In non-limiting embodiments, a serpin inhibitor may be an antibody or antibody fragment or single chain antibody that specifically binds to the serpin. Non-limiting examples of such antibodies are disclosed in Irving, J.A. et al., Methods Enzymol. 2011; 501:421-466; Boncela, J. et al., J. Bio. Chem. 2011; 286:43164-43171; and Van De Craen, B. et al., Throm. Res.2012; 129(4):e126-133. In certain non-limiting embodiments an anti-serpin antibody or antibody fragment may be used to prepare a human, humanized, or otherwise chimeric antibody that is specific for serpin for use according to the invention.

In non-limiting embodiments, a serpin inhibitor may be a nucleic acid, for example, a short hairpin, interfering, antisense, or ribozyme nucleic acid comprising a region of homology to a serpin mRNA. For example, such nucleic acids may be between about 15 and 50 or between about 15 and 30 or between about 20 and 30 nucleotides long, and be able to hybridize to serpin mRNA under physiologic conditions. A non-limiting example of a short hairpin (sh) RNA that inhibits serpin is set forth in the example below. In non-limiting embodiments, a serpin inhibitor which is a nucleic acid may be provided in a serpin-expressing cancer cell via a vector, for example a lentivirus, which may be selectively targeted to said cancer cell and/or wherein expression of the serpin inhibitor nucleic acid may be directed by a promoter which is selectively active in tumor cells.

### 6. EXAMPLE:

### 6.1 MATERIALS AND METHODS

**Brain metastatic cell isolation and culture.** Human brain metastatic cell lines were previously described (Bos et al., 2009; Nguyen et al., 2009b). A ErbB2-P cell line was established from MMTV driven-NeuNT transgenic mammary tumors in mice (Muller et al., 1988). ErbB2-P cells were injected intracardiacally to obtain brain metastatic derivatives. Briefly, a cell suspension containing 10⁵ ErbB2-P cells expressing a TKGFP- Luciferase (TGL) construct, in a volume of 100µl was injected in the left cardiac ventricle of anesthetized 4-6 week-old FVB/NCr mice. Tumor development was monitored by weekly bioluminescence imaging using the IVIS-200 imaging system from Xenogen as previously described. Brain lesions were localized by ex vivo bioluminescence imaging, and resected under sterile conditions. Tissue was minced and placed in culture medium containing a 1:1 mixture of DMEM/Ham' s F12 supplemented with 0.125% collagenase III and 0.1% hyaluronidase. Samples were incubated at room temperature for 4-5 h, with gentle rocking. After collagenase treatment, cells were briefly centrifuged, resuspended in 0.25% trypsin, and incubated for a further 15 min in a 37°C water bath. Cells were resuspended in culture media and allowed to grow to confluence on a 10cm dish. GFP+ cells were sorted for further propagation in culture or inoculation in mice.

MDA231-BrM2, ErbB2-BrM2, 373N1, 393N1, 482N1, 2691N1 where cultured in DME media supplemented with 10% fetal bovine serum (FBS), 2mM L-Glutamine, 100IU/ml penicillin/streptomycin and 1£gg/ml amphotericin B. CN34-BrM2 were cultured in M199 media supplemented with 2.5% fetal bovine serum (FBS), 10µg/ml insulin, 0.5µg/ml hydrocortisone, 20ng/ml EGF, 100ng/ml cholera toxin, 1µg/ml amphothericin B, and 100 U/ml penicillin/streptomycin. H2030-BrM3 and PC9-BrM3 were cultured in RPMI1640 media supplemented with 10% fetal bovine serum (FBS), 2mM L-Glutamine, 100IU/ml penicillin/streptomycin, and 1µg/ml amphotericin B. For retrovirus and lentivirus production, GPG29 and 293T cells, respectively, were cultured in DME media supplemented with 10% fetal bovine serum (FBS), 2mM L-Glutamine, 100IU/ml penicillin/streptomycin, and 1µg/ml amphotericin B. In addition the GPG29 media contained 0.3mg/ml G418, 20ng/ml doxycycline and 2µg/ml puromycin. MDA231-BrM2 SCP were prepared by serial dilution as previously shown (Kang et al., 2003) and cultured in DME media supplemented with 10% fetal bovine serum (FBS), 2mM LGlutamine, 100IU/ml penicillin/streptomycin, and 1µg/ml amphotericin B. Mouse microglia cells were acquired at ATCC (CRL-2467). Mouse astrocytes were obtained from two-day old pups (Schildge et al., 2013). In brief, brains were mechanically dissociated, filtered through 100µm filters and cell suspension cultured in a petri dish under normal conditions during the next 10 days. On day 10, the dish was incubated overnight at 37°C with gentle shaking. Next day media was changed and astrocyte enrichment confirmed with >90% of cells staining positive for GFAP.

**Animal studies.** All experiments using animals were done in accordance to a protocol approved by MSKCC Institutional Animal Care and Use Committee (IACUC). Athymic NCR nu/nu (NCI-Frederick), Cr:NIH bg-nu-xid (NCI-Frederick), FVB/NCr (NCIFrederick), and B6129SF1/J (Jackson Laboratory) female mice aged between 4-6 weeks were used for animal experiments. Brain colonization assays were performed as described previously (Bos et al., 2009; Nguyen et al., 2009b). Briefly, 50,000 (for long term experiments) or 500,000 (for short term experiments) of MDA231-BrM2a, CN34BrM-2c, H2030-BrM3, PC9-BrM3 and 100,000 for syngeneic cell lines 373N1, 393N1, 482N1, 2691N1, ErbB2-BrM2 cells resuspended in 100µl of PBS and injected in the left ventricle. Brain colonization was analyzed in-vivo and ex-vivo by bioluminescence imaging (BLI). Anesthetized mice (ketamine 100mg/kg/xylazine 10mg/kg) were injected retro-orbitally with D-Luciferin (150mg/kg) and imaged with an IVIS Spectrum Xenogen machine (Caliper Life Sciences). Bioluminescence analysis was performed using Living Image software, version 2.50.

**Gene expression analysis.** Whole RNA was isolated from cells using RNAeasy Mini Kit (Qiagen). 1000ng RNA was used to generate cDNA using Transcriptor First Strand cDNA synthesis kit (Roche). Gene expression was analyzed using Taqman gene expression assays (Applied Biosystems). Assays used for human genes: *FADD* (Hs04187499_ml), *FASL* (Hs00181225_ml), *L1CAM* (Hs01109748_m1), *SERPINB2* (Hs00234032_m1), *SERPIND1* (Hs00164821_m1), *SERPINE1* (Hs01126604_m1), *SERPINE2* (Hs00385730_m1), *SERPINI1* probe#1 (Hs01115397_m1), *SERPINI1* probe#2 (Hs01115400_m1). Assays used for the mouse genes: f*a*sL (Mm00438864_m1), *serpinb2* (Mm00440905_m1), *serpind1* (Mm00433939_m1), *serpine2* (Mm00436753_m1), *serpinI1* (Mm00436740_m1). Relative gene expression was normalized to the "housekeeping" genes namely β2*M* (Hs99999907_m1) and β2*m* (Mm00437762_m1). Quantitative PCR reaction was performed on ABI 7900HT Fast Real-Time PCR system and analyzed using the software SDS2.2.2 (Applied Biosystems).

**Clinical samples and immunohistochemistry.** Thirty-three and 123 cases from lung and breast cancer brain metastasis respectively were obtained from the Brain Tumor Center and the Department of Pathology at MSKCC. Paraffin embedded tissue microarrays from brain metastases obtained from breast and lung cancer were obtained from the MSKCC Department of Pathology in compliance with protocols approved by the MSKCC Institutional Review Board (IRB). Immunohistochemistry for Neuroserpin (Abcam, ab16171-100, Lot number 158358, 1:250) and SerpinB2 (Santa Cruz, sc-25745, Lot number L1406, 5 µg/ml) were performed by the MSKCC Molecular Cytology Core Facility using standardized automated protocols. Immunoreactivity stainings were evaluated and scored by clinical pathologists in a blinded fashion. 42 brain metastasis samples from breast cancer were annotated for the primary tumor type, corresponding to 27 cases positive for neuroserpin and 12 for serpin B2. Analysis of expression of *SERPINB2* and *SERPINI1* was performed by using the MSKCC dataset #1 (Nguyen et al., 2009b), including 107 samples of which 106 had clinical information available. The hazard ratio of the average value of *SERPINI1* and *SERPINB2* was computed based on Cox Proportional Hazards Models, as implemented by the "coxph"command in R.

**Brain slice assays.** Organotypic slice cultures from adult mouse brain were prepared adapting previously described methods (Polleux and Ghosh, 2002). Brains (4-6 week old athymic NCR nu/nu mice) were dissected in Hank¡¦s Balanced Salt Solution (HBSS) supplemented with HEPES (pH 7.4) (2.5mM), D-glucose (30mM), CaC12 (1mM), MgSO₄ (1mM), NaHCO₃ (4mM), and were embedded in low-melting agarose (Lonza) pre-heated at 42° C. The embedded brains cut into 250µm slices using a vibratome (Leica). Brain slices (bregma -1mm to +3mm) were placed with flat spatulas on top of 0.8µm pore membranes (Millipore) in slice culture media (DMEM, supplemented HBSS, FBS 5%, LGlutamine (1mM), 100IU/mL penicillin, 100µg/mL streptomycin). Brain slices were incubated at 37°C and 5% CO2 for 1 h, and then 3x10⁴ cancer cells suspended in 2 µL of culture media were placed on the surface of the slice and incubated for 48-72 hours. Brain slices could be maintained under these conditions for up to five days without apparent alterations in tissue architecture. α2-antiplasmin (Molecular Innovations, 2.5µg/ml), neuroserpin and serpin B2 (Peprotech, 0.5µg/ml each) were added to the medium. sFasL (Peprotech, 500ng/ml) or FasL blocking antibodies (BD, 12.5µg/ml) were added to the medium, and slices pre-incubated for 24 hours before addition of cancer cells. Brain slices were fixed in PFA 4%, overnight and then free-floating immunofluorescence performed for GFP (Aves lab, ref. GFP-1020, 1:1000), cleaved caspase-3 (Cell Signaling, ref. 9661, 1:500), collagen IV (Millipore, ref. AB756P, 1:500). Nuclei were stained with Bis-Benzamide (SIGMA, 1£gg/ml). Slices were mounted with ProLong Gold anti fade reagent (Invitrogen).

**Plasmids, recombinant proteins and in vitro experiments.** Human Neuroserpin cDNA (Open Biosystems) was subcloned into the pBABE-puro retroviral expression vector. Site directed mutagenesis (Stratagene) was performed to generate the Δloop mutant previously characterized (Takehara et al., 2009). TRC number for shRNAs used in this study are Neuroserpin (TRCN0000052356 and TRCN0000052355), SERPINB2 (TRCN0000052278), SERPINE2 (TRCN 0000052317), L1CAM (TRCN0000063916). All shRNAs were specific against the human gene and expressed in pLKO.1-shRNA vectors (Open Biosystems) with Puromycin, Hygromycin or Neomycin (G418) resistance genes. The *ST6GalNaC5* shRNA was previously described (Bos et al., 2009). The FADD-DD construct (Andrew M. Thornburn) was subcloned in a pLVX-hygro lentiviral expression vector. Neuroserpin ELISA was performed following manufacturer's instructions (Peprotech). DVL-K chromogenic assays were performed by plating 5x10⁴ cells in 24 well plates and starvation in DMEM FBS 0.25% overnight. Plasminogen (Molecular innovations, 0.125µM) was added to cancer cells that were incubated for 24 hours prior to DVL-K chromogenic assays. D-VLK chromogenic substrate (Molecular Innovations) was prepared following manufacturer¡¦s instructions. DVLK was added to cells and a change in absorbance was monitored at 405 nm. For (3- (4,5-dimethylthiazolyl-2-yl)-2,5-diphenyltetrazolium bromide (MTT) cell proliferation assays, 5x10² cells were plated in 96 well plates, and for cleaved Caspase-3, 25x10³ cells were plated in 24 well plates, starved with FBS 0.25% overnight in presence or absence of sFasL (Peprotech, 100-500ng/ml) and incubated for the indicated period of time. Plasmin (Molecular Innovations) treatment of cells was done at 1.6U/ml for 4 hours.

**Immunofluorescence.** Tissue for immunofluorescence was obtained after overnight fixation with PFA 4% at 4°C. Slicing of the brain was done by using a vibratome (Leica) or sliding microtome (Fisher). Both types of brain slices (250µm and 80µm respectively) were blocked in NGS 10%, BSA 2%, Triton 0.25% in PBS for 2 hours at room temperature (RT). Primary antibodies were incubated overnight at 4°C in the blocking solution and the following day for 30 minutes at RT. After extensive washing in PBSTriton 0.25%, the secondary antibody was added in the blocking solution and incubated for 2 hours. After extensive washing in PBS-Triton 0.25%, nuclei were stained with Bis- Benzamide for 7 minutes at RT. Primary antibodies: GFP (Aves Labs, ref. GFP-1020, 1:1000), Plasminogen (Santa Cruz, ref. sc-25546, 1:100), tPA (Molecular Innovations, ref. ASMTPA-GF, 1:50), uPA (Molecular Innovations, ref. ASMUPA-GF, 1:50), GFAP (Dako, ref. Z0334, and Millipore, ref. MAB360, both 1:1000), IbaI (Wako, ref. 019-19741, 1:500), Col.IV (Millipore, ref. AB756P, 1:500), NeuN (Millipore, ref. MAB377, 1:500), Neuroserpin (Abcam, ref. ab16171, 1:250), FasL (Santa Cruz, ref. sc-834 and sc-6237, 1:100), L1CAM (Millipore, ref. CBL275, 1:200 and Covance, ref. SIG-3911, 1£gg/ml). Secondary antibodies: Alexa-Fluor anti-chicken488, anti-rabbit555, anti-mouse555, antimouse 633 (Invitrogen).

**Immunoblotting.** Cell pellets were lysed with RIPA buffer and protein concentrations were determined by BSA Protein Assay Kit (Pierce). Proteins were separated by SDS-PAGE and transferred to nitrocellulose membranes or PVDF membranes. Membranes were immunoblotted with antibodies against FAS (Santa Cruz, ref. sc-715, 1:100), FasL (Santa Cruz, ref. sc-834 and sc-6237 1:100), L1CAM (Millipore, ref. CBL275, eBioscience, ref. 14-1719, and Abcam, ref. ab24345, 1: 200-1000), FLAG (Sigma, 1:2000), Serpin B2 (Abcam, ref. 47742, 1:500), Tubulin (Cell signaling, 1:2000).

**Confocal microscopy and image analysis.** Images were acquired with a Leica SP5 up-right confocal microscope 10X, 20X, 40X and 63X objectives and images were analyzed with ImageJ, Imaris and Metamorph softwares. In brain slice assays, GFP+ cell bodies that were located >40µm from the surface of the slice were considered for analysis in order to avoid cells clusters remaining on the surface. ImageJ was used to determine the spread cell index by using confocal images and applying the round filter with a 0.45 treshold.

**In vitro blood-brain barrier assay.** This assay was performed as previously described (Bos et al., 2009). Briefly, primary human umbilical vein endothelial cells (HUVEC, ScienCell) were co-cultured with human primary astrocytes (ScienCell), on opposite sides of a polylysine-treated, gelatin-coated tissue culture transwell insert for 3 days. In brief, 3µm pore PET tissue culture inserts (Fisher) were treated with polylysine (1µg/ml, Millipore) overnight, washed four times, and coated with 0.2% gelatin (Sigma) for a minimum of 30 min. Inserts were placed upside-down in a 15 cm plate, and 10⁵ primary human astrocytes were plated on the membrane surface. Astrocytes were fed every 15 min for 5 h, and the inserts were then flipped and placed in 24-well plates. 5x10⁵ endothelial cells were plated on the upper chamber of the inserts, and cultures were placed in the incubator, without further perturbation. For BBB transmigration assays, 5x10⁵ cells were seeded on the upper chamber and incubated for 14-18 h. Inserts were washed with PBS and fixed with 4% PFA for 20 min. The membranes were removed from the plastic insert, immunofluorescence against GFP was performed and mounted on a microscope slide. Pictures of multiple fields from 5-8 inserts per experiment were taken, and the number of transmigrated cells was counted.

**Flow cytometry.** Monolayers of adherent cells were detached using 1mM EDTA, resupended in single cell suspensions and incubated with fluorochrome-conjugated monoclonal antibodies of human L1CAM (eBioscience, ref. 12-1719-42). The cell surface expression of L1CAM was analyzed by a FACSCalibur flow cytometer (BD Biosciences).

**Cell adhesion assays.** HBMEC or tumor cells were plated in 2-well culture slides (BD Falcon) and allowed to grow over 90% confluent. Tumor cells were labeled with CellTracker. Green CMFDA (5-Chloromethylfluorescein Diacetate) (Molecular Probes). 7.2 x 10⁴ pre-labeled tumor cells were allowed to adhere to the monolayer of cancer cells for 20 min. After washing off the non-adherent cells, the slides were fixed with 1% paraformaldehyde and mounted with mounting medium with DAPI (Vector Labs). Adherent cells (green) and the nucleus of total cells (blue) were scored by fluorescence microscopy. The number of GFP+ cancer cells adhered to HBMEC or cancer cells covering the bottom of every well was calculated.

### 6.2 RESULTS

**Association of PA-inhibitory serpins with the brain metastatic phenotype.** In order to identify shared mediators of brain metastasis we analyzed transcriptomic signatures of brain metastatic subpopulations (BrM) that were isolated from lymph nodederived human lung adenocarcinoma cell lines H2030 and PC9 (Nguyen et al., 2009b) and from pleural effusion-derived breast cancer cell lines MDA-MB-231 (MDA231 for short) and CN34 (Bos et al., 2009) (Figure 1A). Seven genes were upregulated in brain metastatic cells compared to the source parental lines in at least three of the four models (Figure 8A). Among these genes, LEF1 was previously defined as a mediator of WNT signaling in brain metastasis by lung adenocarcinoma cells (Nguyen et al., 2009b). *SERPINI1* (see below), but none of the other genes, was associated with brain relapse in human primary tumors. *SERPINI1,* encoding neuroserpin (NS), was also intriguing because its expression is normally restricted to neurons, where it protects from PArelated cytotoxicity (Fabbro and Seeds, 2009; Yepes et al., 2000).

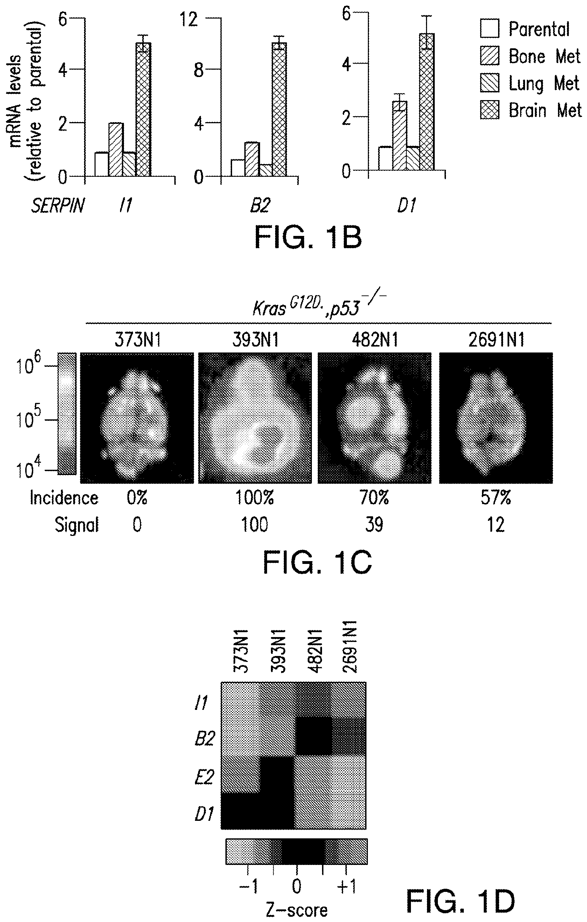

The serpin family in human comprises 36 members that collectively target 18 proteases (Irving et al., 2000). Four of these serpins -neuroserpin and serpins B2, E1, and E2- selectively inhibit PA (Law et al., 2006). Gene expression analysis using qRT-PCR showed that three of the anti-PA serpins were upregulated >3-fold at the mRNA level in brain metastatic cells (Figure 1A). Only one other serpin, *SERPIND1,* was also upregulated (Figure 1A). Serpin D1 inhibits thrombin, which cooperates with plasminogen in cerebral injury (Fujimoto et al., 2008). Bone metastatic derivatives (MDA231-BoM) (Kang et al., 2003) and lung metastatic derivatives (MDA231-LM2) (Minn et al., 2005) were available for comparisons with MDA231-BrM2, and showed little (BoM) or no upregulation (LM2) of the serpins (Figure 1B).

To investigate immune competent models, and a different subtype of breast cancer, we established the cell line ErbB2-P from a mouse mammary tumor driven by a mutant ErbB2 transgene (Muller et al., 1988) and then isolated a brain metastatic derivative (ErbB2-BrM2) by in vivo selection of ErbB2-P in congenic mice. ErbB2-BrM2 cells showed a strong upregulation of serpins B2 and D1 compared to the parental line (Figure 1A). We also screened four cell lines derived from lymph node metastases of genetically engineered Kras*^{G12D};p53*^{*-*/*-*} mouse lung adenocarcinomas (Winslow et al., 7 2011). All four lines were highly metastatic to visceral organs but ranged widely in brain metastatic activity (Figures 1C, S1B); brain metastasis was associated with high expression of serpins I1, B2, E2 and/or D1 (Figure 1C,D).

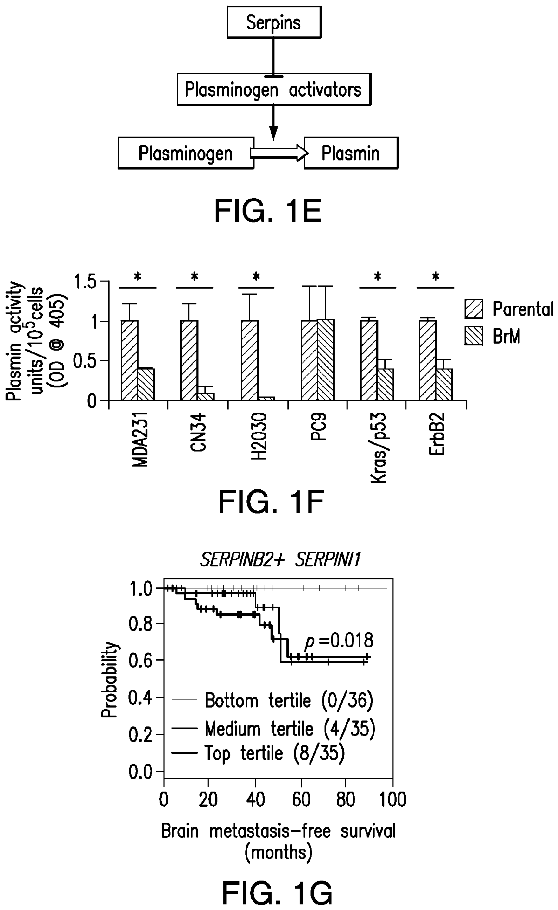

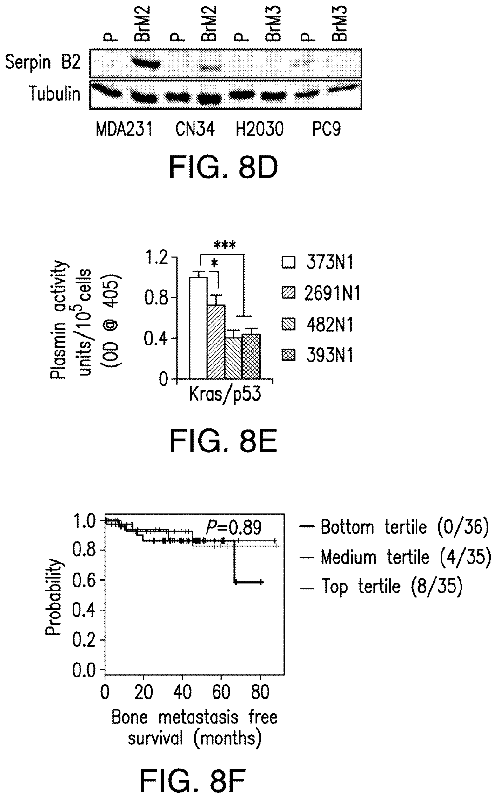

The upregulation of neuroserpin and serpin B2 in brain metastatic cells was confirmed at the protein level (Figure 8C,D). Moreover, conditioned media from brain metastatic cell lines inhibited the conversion of plasminogen into plasmin, as determined using a chromogenic plasmin activity assay (Bai et al., 2011) (Figures 1E,F and 8E). The only exception was PC9-BrM3, a cell line that is less aggressive in brain metastasis compared to H2030-BrM3 (Nguyen et al., 2009b) and lacks upregulated anti-PA serpins (Figure 1A, 8C,D).

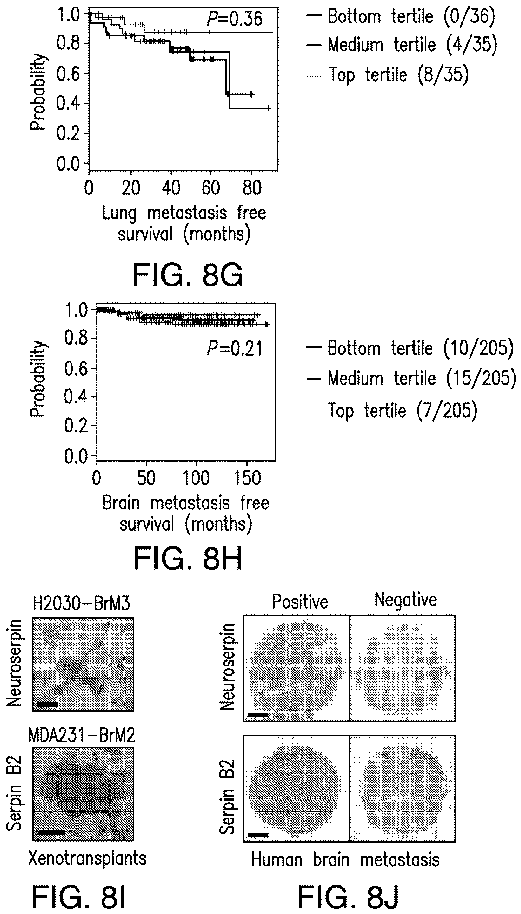

**Neuroserpin and serpin B2 in human brain metastasis tissues.** Focusing on the two most frequently upregulated anti-PA serpins in these models, neuroserpin and serpin B2, we queried gene-expression data from 10⁶ primary lung adenocarcinomas with relapse annotation (Nguyen et al., 2009b). The expression level of *SERPINI1* and *SERPINB2* in the tumors was associated with brain relapse, both as individual genes (data not shown) and combined (p = 0.018, hazard ratio = 2.33 +/-0.3; Figure 1G). Expression of the two genes was not significantly associated with metastasis to bone or lungs (p = 0.89, hazard ratio = 0.91 +/- 0.33; p = 0.36, hazard ratio = 0.76 +/- 0.27; Figure 8F,G). *SERPINI1* and *SERPINB2* expression in primary breast tumors was not a predictor of brain metastasis (p = 0.21, hazard ratio = 0.96 +/-0.16; Figure 8H), though in most of these cases brain relapse was a late event.

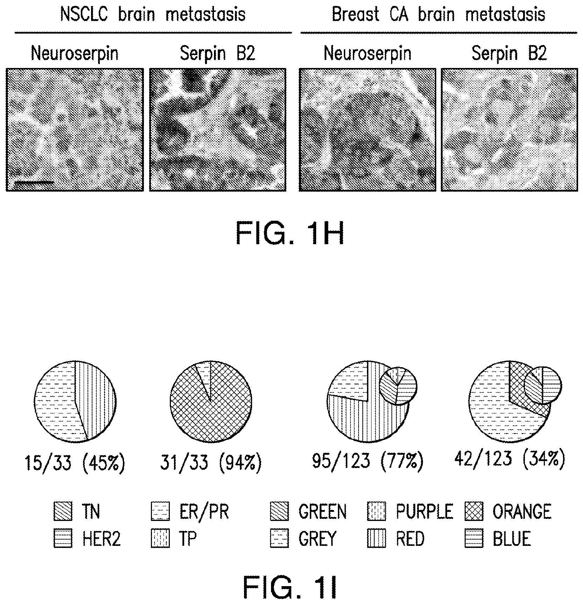

We performed immunohistochemical analysis of neuroserpin and serpin B2 in human brain metastasis tissue, using as a reference brain lesions formed by serpin-expressing human cancer cells in mice (Figure 8I). Among 33 brain metastases of non-small cell lung carcinomas, 45% scored positive for neuroserpin and 94% for serpin B2. Among 123 from breast cancer of various subtypes, 77% scored positive for neuroserpin and 34% for serpin B2 (Figures 1H,I and 8I,J). The immunoreactivity was diffusely distributed in the cytoplasm of carcinoma cells and only minimally in the scant extracellular stroma. Positivity for neuroserpin and serpin B2 in the peritumoral inflammatory infiltrate was limited.

**Plasmin is lethal to cancer cells that invade the brain parenchyma.** The MDA231-BrM2 or H2030-BrM3 models are metastatic to the brain both from orthotopic tumors and from the arterial circulation (Bos et al., 2009; Nguyen et al., 2009b). We inoculated these cells into the arterial circulation of immunodeficient mice via the left cardiac ventricle and fixed the tissue to count cancer cells lodged in the brain capillary network at different time points (Figures 2A-C, 9A). One day after inoculation, we observed isolated cancer cells trapped within brain capillaries (Figure 2B, and H2030-BrM3). Cells passing through the BBB were observed between days 2 and 7 after inoculation (Figures 2B, 9B). All cells remaining within capillaries on day 7 stained positive for the apoptosis marker, cleaved caspase-3 (Figure 9C,D) and disappeared thereafter. In parental MDA231 the number of extravasated cells dropped sharply after day 5 and rarely recovered (Figure 9B). In line with previous reports (Carbonell et al., 2009; Chambers, 2000; Kienast et al., 2010; Lorger and Felding-Habermann, 2010), >90% of cancer cells entering the brain disappeared within days. In MDA231-BrM2 the number of extravasated cells increased until day 7, dropped sharply by day 10, but recovered by day 16. The survivors were bound to and stretched over the abluminal surface of brain capillaries (Figure 2A,B). Outgrowth mainly occurred on the coopted vessels (Figure 2C, summarized in Figure 2D).

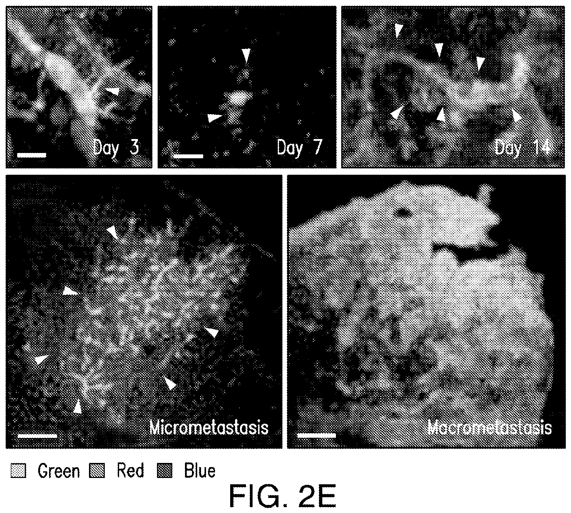

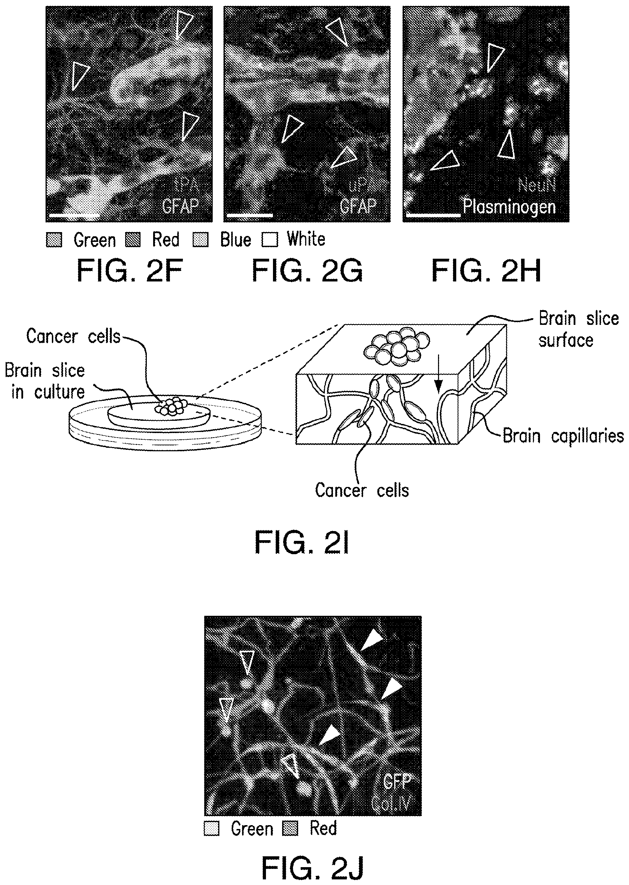

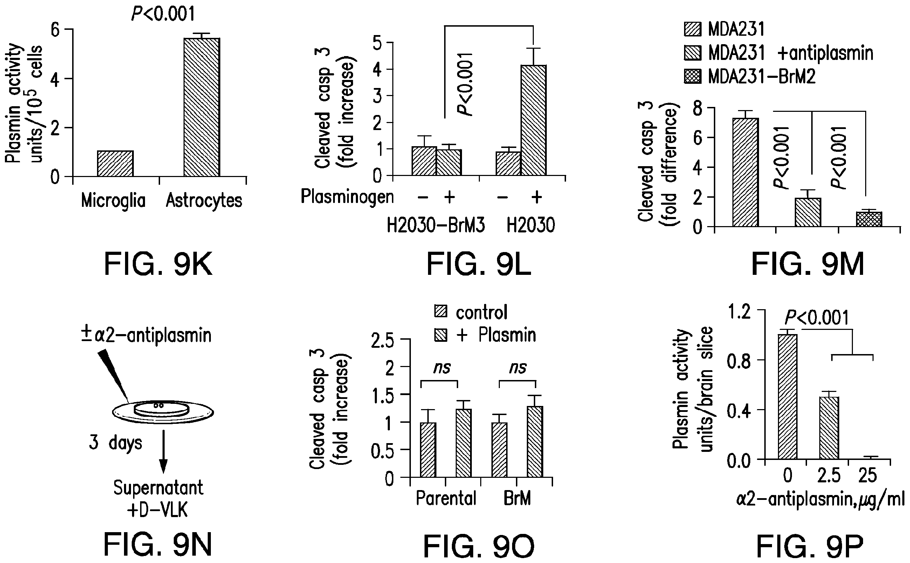

In the brain, metastatic cells were in close proximity to astrocytes (Figures 2E, 9E,F), microglia and neurons (Figure 9G-J). Reactive astrocytes, identified by GFAP overexpression and a stellate morphology, were associated with cancer cells right after extravasation (day 3) and thereafter (Figures 2E, 9E,F). As reactive astrocytes are a major source of PA in brain injury (Adhami et al., 2008; Fabbro and Seeds, 2009; Ganesh and Chintala, 2011), we asked whether these cells were a source of PA in brain metastasis. Mouse brain sections harboring metastatic cells showed tPA and uPA immunoreactivity associated with astrocytes (Figures 2F,G). Mouse astrocytes in culture were superior to microglia at converting plasminogen into plasmin (Figure 9K). Neurons are known to produce plasminogen for neurite and synapse formation (Gutierrez-Fernandez et al., 2009; Hoover-Plow et al., 2001). We confirmed an association of plasminogen immunoreactivity with NeuN+ neurons surrounding metastatic cells in mouse brain (Figure 2H). Thus, the brain metastasis microenvironment contains the necessary components for plasmin production.

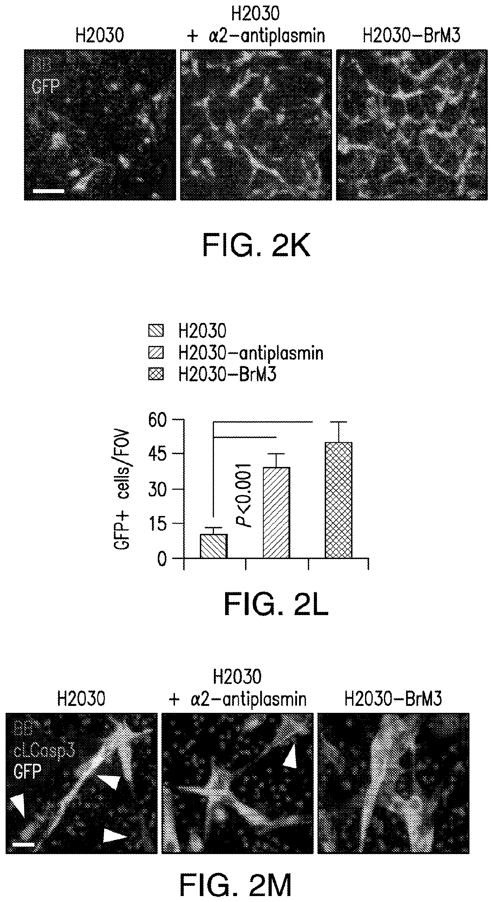

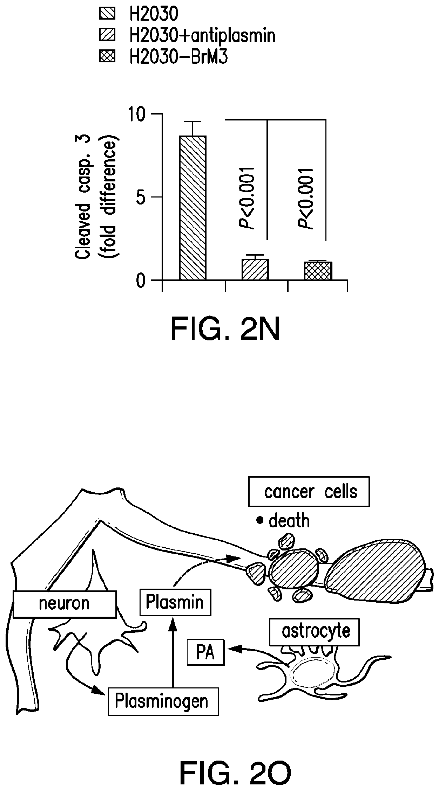

To determine whether plasmin in the brain parenchyma is harmful to metastatic cells we used mouse brain slices in culture (Figure 2I). When placed on top of brain slices H2030-BrM3 cells migrated into the tissue, targeted blood capillaries, and spread on the surface of the vessels (Figure 2J). H2030-BrM3 cells survived and proliferated under these conditions (Figure 2K,L), whereas parental H2030 did not proliferate (Figure 2K,L) and underwent apoptosis (Figure 2M,N). Similar results were obtained with MDA231 cells (Figure 9M). In co-cultures of cancer cells with astrocytes and microglia, plasminogen addition triggered apoptosis in parental H2030 but not in H2030-BrM3 (Figure 9L). The brain tissue slices contained endogenous plasmin activity, and addition of the plasmin inhibitor α2-antiplasmin (Bajou et al., 2008) inhibited this activity (Figure 9N,O). Addition of α2-antiplasmin increased the survival of parental H2030 cells in brain slices (Figure 2K-N). Of note, addition of plasmin to cancer cell monolayer cultures did not trigger apoptosis (Figure 9P). These results suggested that plasmin, acting through unknown substrates in the brain microenvironment, kills infiltrating cancer cells, whereas highly metastatic cells are shielded from this threat (Figure 2O).

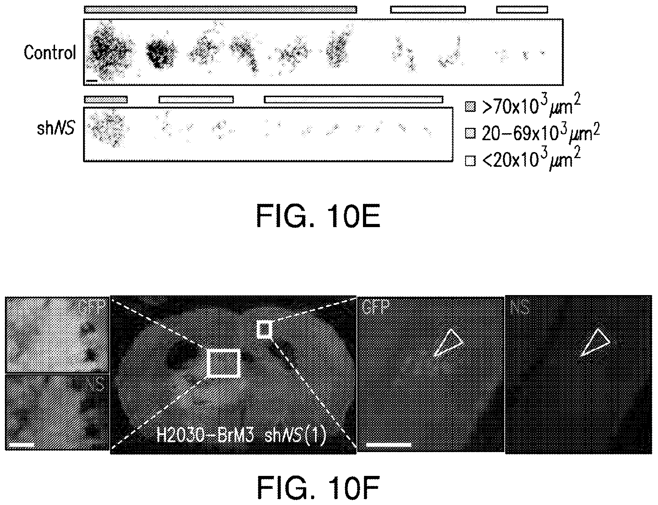

**Neuroserpin protects metastatic cells from plasmin-mediated attrition.** To investigate the role of neuroserpin in brain metastasis we first used the H2030-BrM3 model, in which only this serpin is upregulated (refer to Figure 1A). Brain lesions formed by H2030-BrM3 cells in mice showed strong neuroserpin immunoreactivity (Figure 10A). Two shRNAs that decreased neuroserpin expression and secretion by >85% (Figure 10B,C) did not affect the growth of H2030-BrM3 cells in culture (Figure 10D) but inhibited the metastatic activity of these cells, as shown by bioluminescence imaging (BLI) of marker luciferase in-vivo (Figure 3A-D), BLI ex-vivo (Figures 3B), and marker green fluorescent protein (GFP) expression in brain sections (Figure 3E). Neuroserpin depletion in H2030-BrM3 caused a significant drop in the number and size of brain lesions (Figures 3F, 10E), with a >90% overall reduction in brain tumor burden (Figure 3G). The few macroscopic lesions that developed were rich in neuroserpin (Figure 10F), indicating that these lesions grew from cells that escaped the knockdown.

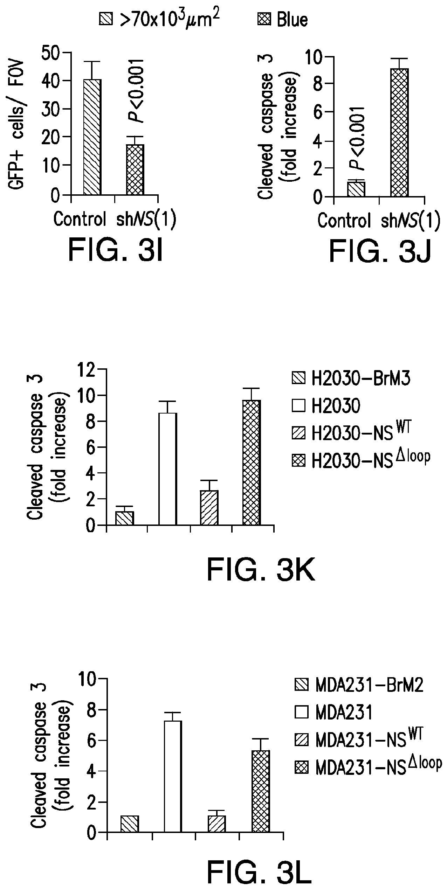

Neuroserpin knockdown did not inhibit the extravasation of H2030-BrM3 cells into the brain parenchyma (Figure 10G). It also did not affect the ability of these cells to cross an endothelial/astrocyte BBB-like barrier in vitro, whereas the knockdown of *ST6GalNaC5,* a mediator of BBB extravasation (Bos et al., 2009), did (Figure 10H,I). In brain slice assays, neuroserpin knockdown in H2030-BrM3 cells decreased the number of infiltrated cells (Figure 3H,I) and increased apoptosis (Figure 3H,J), whereas overexpression of neuroserpin in parental H2030 and MDA231 cells had the opposite effects (Figure 3K,L). In sum, neuroserpin expression in cancer cells supported their survival and outgrowth in the brain parenchyma.

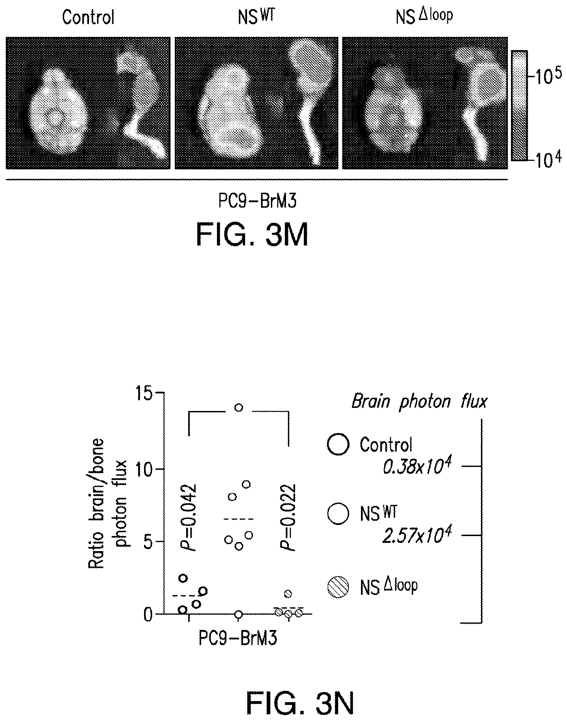

**Brain metastasis mediated by the PA inhibitory function of neuroserpin.** To determine whether neuroserpin can increase the brain metastatic activity of lung cancer cells in vivo we used the PC9-BrM3 model. PC9-BrM3 cells can infiltrate the brain but are less aggressive than H2030-BrM3 (Nguyen et al., 2009b) and do not show upregulation of anti-PA serpins (refer to Figure 1A, 8C,D). PC9-BrM3 cells were stably transduced with vectors encoding the wild type neuroserpin or a mutant (neuroserpin Δloop) that is devoid of PA inhibitory function (Takehara et al., 2009) (Figure 10J-L). The wild type neuroserpin significantly increased the brain metastatic activity of PC9-BrM3 cells whereas the mutant neuroserpin did not (Figure 3M,N) without increasing the proliferation of these cells in culture (Figure 10M). PC9-BrM3 cells are also metastatic to bone (Nguyen et al., 2009b); neuroserpin overexpression did not markedly affect this activity (Figure 3M,N). NeuroserpinΔloop was also ineffective at protecting the parental H2030 and MDA231 cells from apoptosis in brain tissue (Figure 3K,L). These results suggest that neuroserpin mediates brain metastatic activity in cancer cells by inhibiting PA.

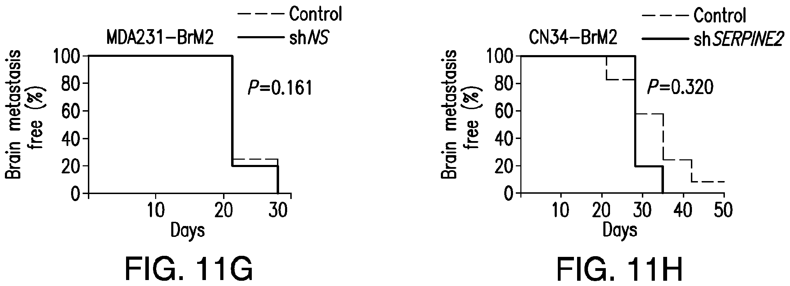

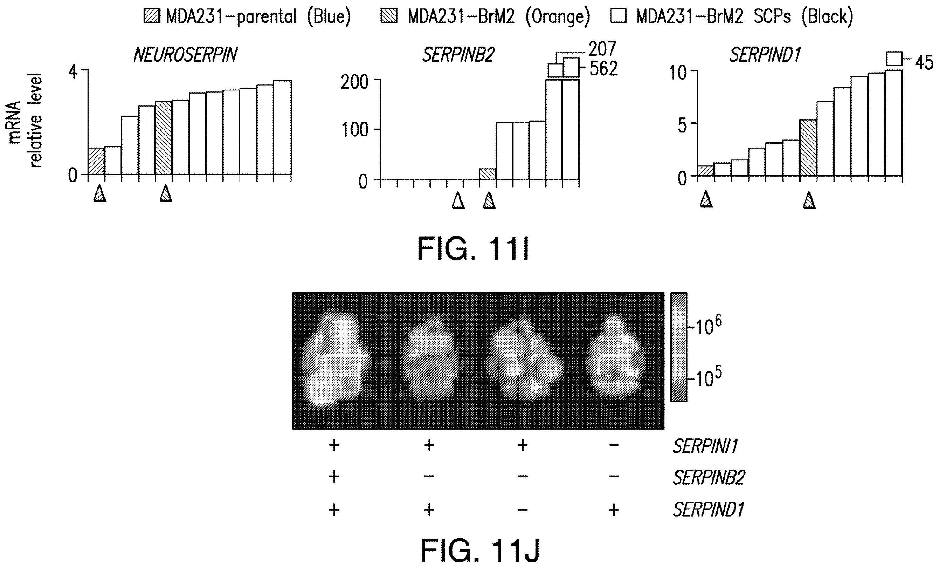

**Role of anti-PA serpins in brain metastatic breast cancer cells.** Unlike the H2030-BrM3 cells, most other brain metastatic models and a large proportion of human brain metastatic tissues overexpressed not one but multiple anti-PA serpins (refer to Figure 1A,I). In MDA231-BrM2, a triple knockdown of the three overexpressed serpins -serpins B2, D1 and neuroserpin- (Figure 11A-C) inhibited the brain metastatic activity of the cells more than did the knockdown of any individual serpin (Figures 4A,B, 11G,H). The knockdown of serpin B2 (Figure 11D,E) partially inhibited the brain metastatic activity of MDA231-BrM2, and the lost activity could be rescued by enforced overexpression of neuroserpin (Figures 4A,B, 11F). We isolated ten clonal cell lines from the MDA231-BrM2 population and determined the expression levels of neuroserpin, serpin B2 and serpin D1 in each cell line. Clonal heterogeneity in serpin expression was evident, with individual clones overexpressing one, two, or all three serpins. Compared to the parental MDA231 population, neuroserpin was upregulated in 9/10 of the clones, serpin B2 in 5/10 and serpin D1 in 8/10 (Figures 4C, 11I). As a trend, clones overexpressing three serpins were more metastatic to the brain than were clones overexpressing fewer (Figures 4D, 11J). Clones that overexpressed neuroserpin and serpin D1 lost brain metastatic activity when transduced with neuroserpin shRNA (Figure 4E). Serpin B2 was the only upregulated anti-PA serpin in the ErbB2-BrM2 model (refer to Figure 1A). Serpin B2 knockdown in these cells strongly decreased their brain metastatic activity in immunocompetent mice (Figure 4F-H). In sum, the evidence indicated that expression of one or more anti-PA serpins provides lung cancer and breast cancer cells with a critical advantage in the formation of brain metastases.

**Metastatic cells face Fas**L **in the brain.** We searched plasmin substrate databases (MEROPS, CutDB) for proteins whose cleavage by plasmin might be relevant to brain metastasis. Besides cleaving fibrin in the fibrinolytic cascade, plasmin can cleave certain cytokines, membrane proteins, and extracellular matrix components (Bajou et al., 2008; Chen and Strickland, 1997; Nayeem et al., 1999; Pang et al., 2004). We focused first on FasL as a protein whose cleavage by plasmin might be deleterious to metastatic cells in the brain. FasL is a membrane-anchored homotrimeric protein that binds to the receptor Fas, which activates proapoptotic caspases through the adaptor protein FADD (Ashkenazi and Dixit, 1998). FasL is highly expressed in reactive astrocytes in ischemia, brain trauma, Alzheimer's disease, encephalomyelitis and multiple sclerosis (Choi and Benveniste, 2004; Dietrich et al., 2003). Astrocytes are the main source of FasL against invading T cells in experimental encephalomyelitis (Wang et al., 2013). Plasmin cleaves membrane-anchoredFasL at Arg144, releasing a soluble pro-apoptotic fragment (sFasL) (Bajou et al., 2008; Fang et al., 2012). Therefore, we tested the hypothesis that anti-PA serpins shield cancer cells from the lethal action of plasmin-mobilized sFasL (Figure 5A).

Immunofluorescence staining of brain sections harboring H2030-BrM3 lesions confirmed that FasL was mainly expressed on reactive astrocytes in the lesions (Figures 5B, 12A). Human and mouse astrocytes also expressed FasL in culture (Figures 5C, 12B). Addition of plasminogen to these cultures decreased the level of cell-associated FasL increasing the cleaved product in the supernatant, as determined by immunostaining and western blotting with antibodies against the extracellular domain of FasL (Figures 5C,D, 12C-E). Mouse brain slices, which contain active plasmin (refer to Figures 2K, 9O), also contained sFasL. Addition of anti-PA serpins or antiplasmin decreased the level of sFasL in these tissues (Figure 5E). These results suggested a capacity of the PA-plasmin system to mobilize stromal FasL in the brain.

Next we asked whether cancer cells that infiltrate the brain are susceptible to FasL-mediated killing. H2030, PC9, MDA231 and CN34 expressed Fas, as did their BrM derivatives (Figure 12F). Addition of sFasL to BrM cell monolayers caused apoptosis (Figure 12G-I). Addition of sFasL to brain slices harboring H2030-BrM3 (Figure 5F-H), even when α2-antiplasmin (refer to Figure 2N) was present in the culture (Figure S5J). Conversely, addition of anti-FasL blocking antibody protected parental H2030 cells from apoptosis (Figure 5G-I). Thus, brain metastatic cells are highly susceptible to apoptosis if exposed to FasL in the brain parenchyma.

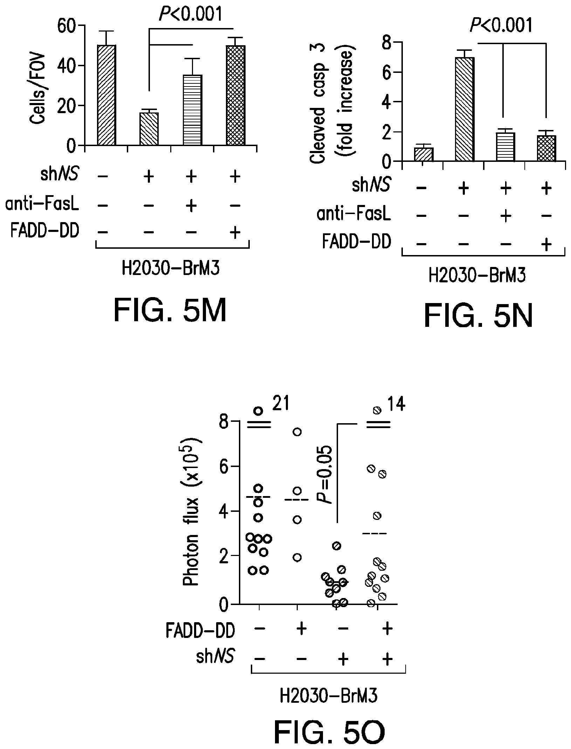

**Neuroserpin shields brain metastatic cells from Fas-mediated killing.** To determine whether Fas signaling caused the death of cancer cells that infiltrated the brain, we used a FADD truncation mutant that lacks the death effector domain (FADD-DD construct) and acts as a dominant-negative inhibitor of Fas signaling (Chinnaiyan et al., 1996) (Figure 5J). Transduction of FADD-DD in H2030-BrM3 cell line (Figure 5K) prevented the activation of caspase 3 by sFasL (Figure 5L). The apoptosis that anti-PA serpin-depleted H2030-BrM3 or MDA231-BrM2 cells suffer in brain tissue (refer to Figures 3H-J, 12L) could be prevented by adding anti-FasL blocking antibodies to the tissue culture as well as by enforcing the expression of FADD-DD in the cells (Figures 5M,N, 12L). Moreover, FADD-DD partially rescued the ability of neuroserpin-depleted H2030-BrM3 cells to metastasize in the brain (Figure 5O). Collectively, these results showed that cancer cells that infiltrate the brain succumb to Fas signaling, and anti-PA serpin activity can shield the metastatic cells from FasL attack.

**The plasmin target L1CAM mediates cancer cell spreading on brain endothelial cells.** Although inhibition of Fas signaling with FADD-DD clearly protected neuroserpin- depleted cancer cells from death in the brain, the metastatic activity of these cells was not fully restored compared to that of wild-type H2030-BrM3 cells (Figure 5O). The neuroserpin-depleted, FADD-DD expressing H2030-BrM3 cells formed smaller lesions that were less well organized alongside capillaries in the brain (Figure 13A). Therefore we postulated that anti-PA serpins promote brain metastasis by doing more than just preventing FasL action.

Several clues led us to consider L1 cell adhesion molecule (L1CAM) as an additional mediator of brain metastasis downstream of the serpin-PA-plasmin system. L1CAM is mainly expressed in neural tissues and in tumors (Schafer and Altevogt, 2010). It consists of six immunoglobulin-like (Ig) domains, five fibronectin-like (FN) domains, a transmembrane region, and an intracellular domain (Figure 6A). The L1CAM Ig-like repeats mediate homo- and heterophilic interactions for axon guidance during brain development (Maness and Schachner, 2007). L1CAM binds to itself and also to β integrins (Felding-Habermann et al., 1997) and other proteins (Castellani et al., 2002; Donier et al., 2012; Kulahin et al., 2008), and triggers signaling and cytoskeleton remodeling (Herron et al., 2009). Inherited *L1CAM* mutations cause the L1 neurological syndrome (Demyanenko et al., 1999; Maness and Schachner, 2007; Vos and Hofstra, 2010), whereas L1CAM expression in tumors is associated with poor prognosis (Boo et al., 2007; Fogel et al., 2003; Hai et al., 2012; Thies et al., 2002; Tsutsumi et al., 2011; Zhu et al., 2010). Although L1CAM has been implicated in cell invasion (Voura et al., 2001), little is known about its role in cancer. Plasmin cleaves L1CAM at dibasic motifs (Lys860/Lys863), disrupting the capacity for cell-cell adhesion (Nayeem et al., 1999; Silletti et al., 2000) (Figure 6A).

L1CAM was expressed in most parental lines and all the brain metastatic derivatives that we examined, regardless of species or tumor type of origin (Figure 13B,C). We investigated the role of L1CAM as a mediator of heterotypic interactions between H2030-BrM3 cells and monolayers of human brain microvascular endothelial cells (HBMEC) and homotypic interactions with monolayers of H2030-BrM3 cells. H2030- BrM3 cells readily adhered on HBMEC monolayers (Figure 6B). Notably, RNAi-mediated knockdown of L1CAM (Figure 13B) inhibited the ability of H2030-BrM3 cells to bind to HBMEC (Figure 6C) or H2030-BrM3 monolayers (Figure 6D).

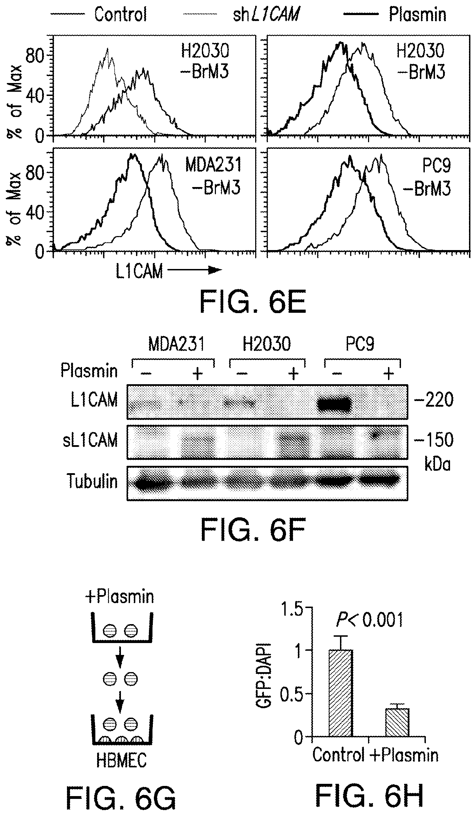

Addition of plasmin to monolayers of H2030-BrM3, MDA231-BrM2 and PC9-BrM3 caused a decrease in cell-associated 220kDa L1CAM levels, as shown by anti-L1CAM flow cytometry (Figure 6E) and by the accumulation of a 150kDa L1CAM fragment in the supernatants (Figure 6F) (Mechtersheimer et al., 2001). Moreover, plasmin-treated H2030-BrM3 cells lost capacity to bind to HBMEC monolayers (Figure 6G,H).

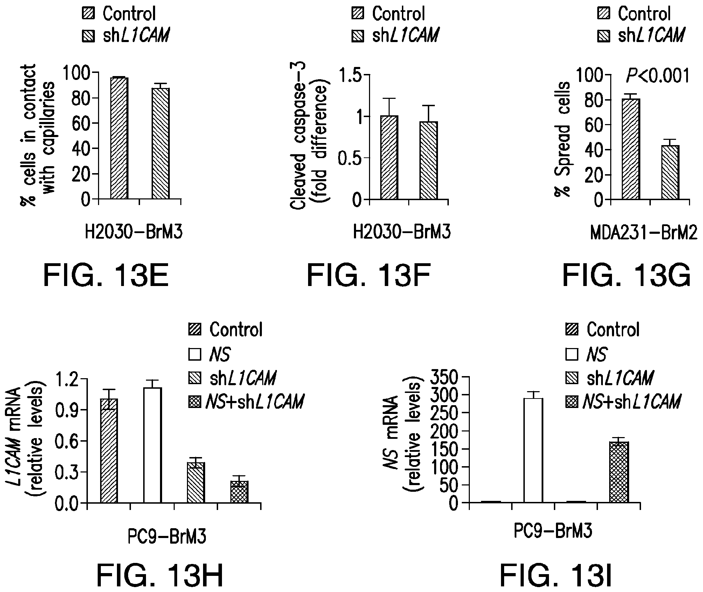

**L1CAM mediates vascular co-option and metastatic outgrowth.** The molecular basis for vascular cooption by cancer cells remains unknown. Given the ability of L1CAM to mediate adhesion of brain metastatic cells to HBMECs, we investigated whether cancer cell L1CAM participates in vascular cooption in the brain. The wild type and the L1CAM-depleted H2030-BrM3 showed a similar proliferation rate in culture (Figure 13D) and a similar ability to infiltrate brain tissue and seek brain capillaries (Figures 6I, 13E). However, L1CAM depletion significantly reduced the abilityof H2030-BrM3 and MDA231-BrM2 cells to spread on the abluminal surface of brain capillaries (Figures 6I,J, 13G). Notably, this was accompanied with a marked decrease in the proliferation marker Ki67 in the vessel-associated cancer cells (Figure 6K) but not with changes in apoptosis markers (Figure 13F).

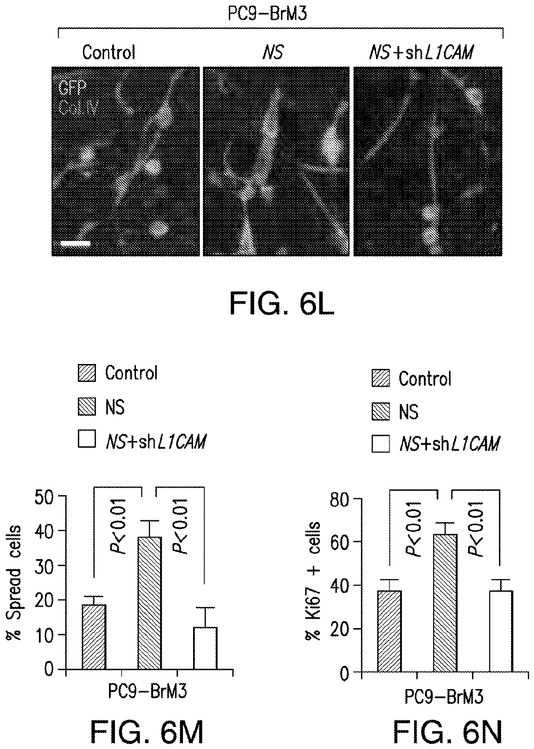

PC9-BrM3 cells do not overexpress endogenous anti-PA serpins. Interestingly, only a small proportion of PC9-BrM3 cells spread on the capillaries in brain slice assays (Figure 6L,M). Enforced expression of neuroserpin in PC9-BrM3 cells, which augments the metastatic activity of these cells (see Figure 3N), significantly increased their spreading on brain capillaries (Figure 6L,M) and their proliferation on the coopted vessels (Figure 6N). Importantly, L1CAM depletion in neuroserpin-overexpressing PC9-BrM3 cells (Figure 13H,I) abrogated the neuroserpin-dependent gains in vascular cooption and cell proliferation (Figure 6L-N). These results showed that L1CAM mediates vascular cooption and outgrowth of metastatic cells in the brain.

**L1CAM supports metastasis initiation downstream of neuroserpin.** We investigated the role of L1CAM in brain metastasis in vivo. Immunohistochemical analysis of L1CAM of H2030-BrM3 micrometastases showed a localization of this molecule at the interfaces with endothelial cells (identified by nuclei of small, flat morphology and intense H-E staining) and with adjacent cancer cells (Figure 7A). L1CAM knockdown in H2030-BrM3 and MDA231-BrM2 markedly decreased the metastatic activity of these cells in mice (Figure 7B-D). Histologic analysis at day 7 showed that L1CAM-depleted cells did not spread over the capillary network after extravasating in the brain (Figure 7E). Twenty-one days later, these colonies were stalled at the micrometastatic stage (as defined in Figure 3F) (Figure 7F,G). Whereas the wild type cell readily expanded over the capillary network and formed large colonies, the LICAM-depleted cells remained mostly as single cells or small clusters that were poorly bound to capillary vessels (Figure 7F,G). Moreover, the gain in metastatic activity imparted by enforced overexpression of neuroserpin in PC9-BrM3 was abrogated by the L1CAM knockdown in these cells (Figure 7H). These results argued that L1CAM expression in metastatic cells acts downstream of neuroserpin to mediate cooption of brain capillaries and metastatic outgrowth.

### 6.3 DISCUSSION

The growing incidence of brain metastasis warrants a better understanding of the molecular mechanisms that underlie this condition. Our findings illuminate two critical requisites for metastatic colonization of the brain, namely, the escape of infiltrating cancer cells from decimation by lethal signals from the reactive stroma, and the striking ability of the surviving cancer cells to coopt brain capillaries during metastatic expansion. We show that a stromal PA-plasmin pathway and its inhibition by carcinoma-derived anti-PA serpins control both of these processes in brain metastasis from lung cancer and breast cancer, suggesting a unified mechanism for metastatic colonization of the brain.

**Anti-PA serpins as common mediators of brain metastasis.** Brain metastasis involves close and sustained interactions of cancer cells with brain capillaries and reactive astrocytes. Previous work (Kienast et al., 2010; Lorger and Felding-Habermann, 2010) and our own data show that circulating cancer cells in brain capillaries interact with the BBB endothelium not only during extravasation but subsequently as well, by attaching to the abluminal surface for metastatic outgrowth as a furrow along the coopted vessels. The cancer cells are also immediately exposed to astrocytes, which are present in the perivascular space and contact the endothelium to form the BBB (Abbott et al., 2006). We show that astrocytes act as a source of deleterious signals to repel invading cells. Astrocytes may eventually support the growth of brain metastasis by providing growth factors (Seike et al., 2011) and GAP junctions (Lin, 2010). However, in order to benefit from these trophic inputs, cancer cells must first avert the deleterious effects of the reactive stroma.

Expression of anti-PA serpins in the cancer cells provides such a shield. We show that brain metastatic lung and breast cancer cells from human or murine origins express high levels of anti-PA serpins compared to counterparts that are lowly metastatic to the brain. Three out of four known anti-PA serpins, and serpin D1 are expressed in the six experimental models that we investigated. The most prominent anti-PA serpins in these models, neuroserpin and serpin B2, are also expressed in a majority of the human brain metastasis samples from lung cancer and breast cancer patients that we examined. In functional assays these serpins and their PA inhibitory activity are limiting for metastatic colonization of the brain.

The PA-plasmin system is well characterized in connection with its role in blood clot resolution. In cancer however the PA-plasmin system is paradoxically implicated both in tumor suppression and tumor progression. Plasmin is thought to promote cancer cell proliferation and invasion by cleaving growth factor precursors and extracellular matrix components (McMahon and Kwaan, 2008). However, the anti-PA serpin E1 in tumors and in blood is associated with poor clinical outcome in lung, breast, and gastrointestinal cancers (Allgayer et al., 1997; Berger, 2002; Foekens et al., 1995; Harbeck et al., 1999). The same holds for serpin B2 in lung cancer (Morita et al., 1998). The role of PA and plasmin in tumor progression therefore has remained obscure. Here we show that anti-PA serpins shield metastatic cells from PA-plasmin in the brain, with a clear prometastatic advantage.

**Shielding cancer cells from Fas death signals in the brain.** Our results suggest that the PA-plasmin system acting through FasL creates a highly hostile environment for infiltrating cancer cells in the brain. Although FasL plays important roles in immune homeostasis (Krammer, 2000) and is present in tumors (Baldini et al., 2009) its expression is particularly acute in reactive astrocytes (Beer et al., 2000). Astrocytes are the main source of FasL in response to infiltrating leukocytes, and of PAs in response to brain injury (Adhami et al., 2008; Bechmann et al., 2002; Ganesh and Chintala, 2011; Teesalu et al., 2001). Astrocyte-derived FasL plays a central role in repelling invading autoimmune T cells in the brain (Wang et al., 2013). We observed that metastasis-associated astrocytes express both PA and FasL, that plasmin releases membrane-bound FasL from astrocytes, and that sFasL levels in brain tissue depend on plasmin. Addition of anti-PA serpins, anti-plasmin serpins, or FasL-blocking antibodies to brain tissue protects infiltrating cancer cells. Moreover, brain metastatic cells from lung or breast cancers are highly sensitive to sFasL-induced apoptosis, and suffer Fasdependent death in the brain unless they express anti-PA serpins. We conclude that the attrition of infiltrating cancer cells in the brain is mediated by Fas signaling and impeded by anti-PA serpins. Cancer cells that express anti-PA serpins therefore have a strong advantage in the PA-rich microenvironment of the brain.

**L1CAM-mediated vascular cooption for metastatic outgrowth.** Avoiding FasL-mediated death is not the only pro-metastatic benefit provided by anti-PA serpins. We show that neuroserpin additionally promotes vascular cooption -the spreading of the cancer cells on the vasculature. This effect depends on expression of the plasmin-labile molecule L1CAM in cancer cells. L1CAM expression is normally restricted to neurons where it mediates axonal guidance through interactions of the growth cone with surrounding components (Castellani et al., 2002; Wiencken-Barger et al., 2004). We show that L1CAM expression in cancer cells mediates their adhesion and spreading on brain endothelial cells in culture and on capillaries in the brain. L1CAM additionally mediates interactions between cancer cells. Plasmin cleaves L1CAM inactivating these binding activities. When depleted of L1CAM, brain metastatic cells fail to coopt brain capillaries, and metastatic outgrowth stalls. The evidence suggests that neuroserpin prevents plasmin-mediated destruction of L1CAM in brain metastatic cells, fostering vascular cooption by these cells and further enhancing metastasis.

The finding that L1CAM is a mediator of cancer cell spreading on capillaries provides unexpected insights into the molecular basis for vascular cooption in cancer. A striking feature of brain metastasis is the ability of metastatic cells to remain closely attached to the capillary network after extravasation (Kienast et al., 2010; Lorger and Felding- Habermann, 2010). Vascular cooption is thought to be important for brain metastasis (Carbonell et al., 2009) and for cancer cell escape from therapy-induced hypoxia (Leenders et al., 2004). Despite the likely importance of vascular cooption in cancer, the molecular basis of this process is unknown. The present identification of L1CAM as a mediator of metastatic vascular cooption provides an opening for the mechanistic and functional dissection of this process.

**Implications beyond brain metastasis.** The molecular mechanisms identified here protect metastatic cells from selective pressures that are particularly acute in the brain but may also be relevant in other contexts. The high mortality of cancer cells that infiltrate distant organs is characteristic of metastasis in general (Gupta and Massagué, 2006; Valastyan and Weinberg, 2011), and vascular cooption has been observed in metastasis to other organs and by other types of cancer (Blouw et al., 2003; Leenders et al., 2002; Leenders et al., 2004). The brain microenvironment can certainly select for brain-specific metastatic traits in cancer cells (Bos et al., 2009; Nguyen et al., 2009b). However, evading death signals and interacting with the vasculature are basic needs of metastatic cells in all organs, not only in the brain. We note that the serpins overexpressed in our brain metastatic models are also expressed, albeit at lower levels, in counterparts that are metastatic to other organs (refer to Figure 1B). Moreover, L1CAM expression in primary tumors is associated with poor prognosis in various types of cancer (Boo et al., 2007; Doberstein et al., 2011; Fogel et al., 2003; Schroder et al., 2009; Thies et al., 2002; Tischler et al., 2011; Tsutsumi et al., 2011). PA, plasmin, and FasL have also been implicated in disease progression in other cancers (McMahon and Kwaan, 2008; Timmer et al., 2002). The reactive brain stroma and its high capacity to generate PA-plasmin and FasL may be more challenging to infiltrating cancer cells than is the stroma in other organs. As a result, the brain may select for more accentuated versions of otherwise general metastatic traits. Although anti-PA serpins, plasmin, FasL and L1CAM had not been previously connected in a unified mechanism or linked to metastatic cell survival and vascular cooption, their repeated clinical association with poor prognosis may reflect a wider role in metastasis.

### 7. EXAMPLE: VASCULAR CO-OPTION IN METASTASIS INITIATION

Vascular co-option was observed in the initiation of metastases in brain, bone and lung. Figure 14A-C shows vascular co-option of GFP-expressing cancer cells, where blood vessels are indicated by red ColIV staining. In the left-most panel, the cancer cells are lung adenocarcinoma, KRAS mutant (cell line H2030-BrM3). In the central panel, the cancer cells are breast adenocarcinoma, subtype claudin-low triple-negative, cell line MDA231-SCP6. In the right-most panel, the cancer cells are breast adenocarcinoma, subtype claudin-low triple-negative, cell line MDA231-LM2.

### 8. EXAMPLE: L1CAM-ENGAGEMENT IS A GENERAL MECHANISM REQUIRED FOR CANCER CELL GROWTH AND DISSEMINATION

Aberrant L1CAM expression has been demonstrated at the leading edge of primary tumors, and is associated with invasion, metastasis and poor prognosis in many human cancers including lung, breast and colon carcinomas (Voura et al., 2001; Ben et al., 2010; Tsutsumi et al., 2011; Schroder et al, 2009; Tischler et al., 2011; Boo et al., 2007; Chen et al., 2013; Fogel et al., 2003a; Doberstein et al., 2011; Fogel et al., 2003b; Kim et al., 2009; Maness et al., 2007). This suggested that the selective advantage gained from L1CAM-expression is not restricted to the brain. Accordingly, experiments were performed to evaluate the role of L1CAM in metastasis outside of the central nervous system.

L1CAM expression in cancer cells was inhibited using RNAi ((TRCN0000063916 ;The RNAi Consortium, Public TRC Portal), including RNAi having a hairpin sequence 5' CGGACGGGCAACAACAGCAACTTTCTCGAGAAAGTTGCTGTTG TTGCCCGTTTTTTG (SEQ ID NO:1) and a target sequence ACGGGCAACAACAGCAACTTT (SEQ ID NO:2), introduced into the cancer cells via a pLKO.1-shRNA vector. Human MDA231 breast cancer cells and H2030 human lung cancer cells were rendered L1CAM-depleted in this manner (denoted *"shL1CAM"* in the figures).

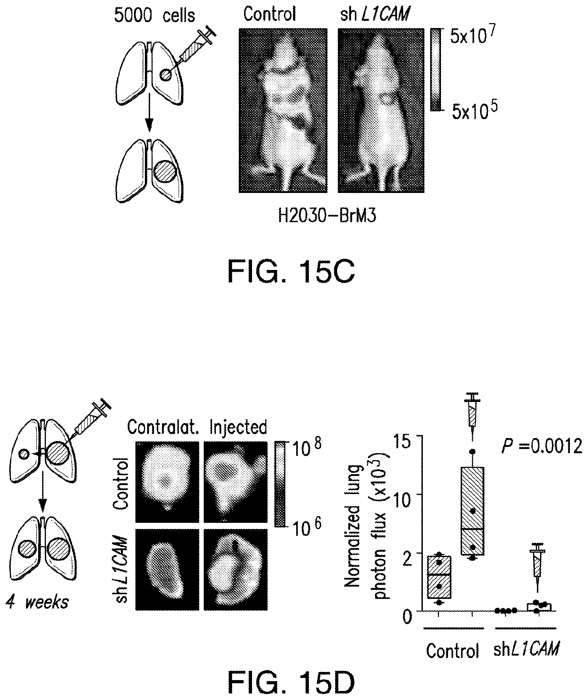

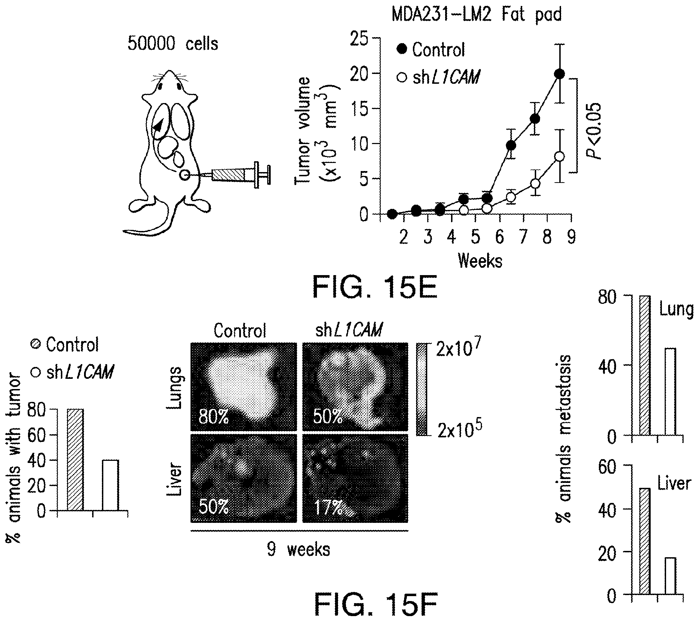

100,000 *shL1CAM* MDA231-BoM2 (for bone metastasis) or MDA231-LM2 (for lung metastasis) cells, or control MDA231-BoM2 or MDA231-LM2 (for lung metastasis) cells, were introduced into athymic mice by intracardiac injection (for bone metastasis) or tail vein injection (for lung metastasis), and the amount of bone and lung metastasis determined after 21 days using bioluminescence imaging. As shown in Figures 15A and B, the extent of metastatic disease in bone and lung, respectively, was dramatically reduced in mice that had received L1CAM-depleted cancer cells. Similar results were observed when 5000 control (undepleted) or *shL1CAM* H2030 human lung cancer cells were injected into mouse lung ; Figure 15C shows tumor growth at the site of orthotopic lung injection after 4 weeks. Figure 15D shows metastasis to the contralateral lung after four weeks. Figure 15E shows the tumor volume at the mammary fat pad injection site of 50,000 control or *shL1CAM* MDA231-LM2 cancer cells and Figure 15F shows the extent of metastasis of those cells, after 9 weeks, to lungs or liver. In all these experiments, L1CAM depletion (L1CAM inhibition) significantly reduced the progression of metastatic disease.

Further, as shown in Figure 16A-B, L1CAM-depletion was observed to inhibit the growth of cell-cell interaction enriched oncosphere aggregates derived from (A) lung (H2030-BrM3) or (B) breast (Hcc1954-BrM1b) cancer cells grown in defined adhesion-free conditions.

These experimental results indicate that L1CAM engagement, whether derived from endothelial cells or from intratumor cell-cell contacts, confers growth and survival benefits to cancer cells.

### 9. REFERENCES

Abbott, N.J., Ronnback, L., and Hansson, E. (2006). Astrocyte-endothelial interactions at the blood-brain barrier. Nat Rev Neurosci 7, 41-53.
Adhami, F., Yu, D., Yin, W., Schloemer, A., Burns, K.A., Liao, G., Degen, J.L., Chen, J., and Kuan, C.Y. (2008). Deleterious effects of plasminogen activators in neonatal cerebral hypoxia-ischemia. Am J Pathol 172, 1704-1716.
Allgayer, H., Heiss, M.M., and Schildberg, F.W. (1997). Prognostic factors in gastric cancer. Br J Surg 84, 1651-1664.
Ashkenazi, A., and Dixit, V.M. (1998). Death receptors: signaling and modulation. Science 281, 1305-1308.
Bai, H., Baik, N., Kiosses, W.B., Krajewski, S., Miles, L.A., and Parmer, R.J. (2011). The novel plasminogen receptor, plasminogen receptor(KT) (Plg-R(KT)), regulates catecholamine release. J Biol Chem 286, 33125-33133.
Bajou, K., Peng, H., Laug, W.E., Maillard, C., Noel, A., Foidart, J.M., Martial, J.A., and DeClerck, Y.A. (2008). Plasminogen activator inhibitor-1 protects endothelial cells from FasL-mediated apoptosis. Cancer Cell 14, 324-334.
Baldini, E., Ulisse, S., Marchioni, E., Di Benedetto, A., Giovannetti, G., Petrangeli, E., Sentinelli, S., Donnorso, R.P., Reale, M.G., Mottolese, M., et al. (2009). Expression of Fas and Fas ligand in human testicular germ cell tumours. Int J Androl 32, 123-130.
Barnholtz-Sloan, J.S., Sloan, A.E., Davis, F.G., Vigneau, F.D., Lai, P., and Sawaya, R.E. (2004). Incidence proportions of brain metastases in patients diagnosed (1973 to 2001) in the Metropolitan Detroit Cancer Surveillance System. J Clin Oncol 22, 2865-2872.
Bechmann, I., Steiner, B., Gimsa, U., Mor, G., Wolf, S., Beyer, M., Nitsch, R., and Zipp, F. (2002). Astrocyte-induced T cell elimination is CD95 ligand dependent. J Neuroimmunol 132, 60-65.
Beer, R., Franz, G., Schopf, M., Reindl, M., Zelger, B., Schmutzhard, E., Poewe, W., and Kampfl, A. (2000). Expression of Fas and Fas ligand after experimental traumatic brain injury in the rat. J Cereb Blood Flow Metab 20, 669-677.
Ben QW, Wang JC, Liu J, Zhu Y, Yuan F, Yao WY, Yuan YZ. Positive expression of L1-CAM is associated with perineural invasion and poor outcome in pancreatic ductal adenocarcinoma. Ann Surg Oncol. 2010 Aug;17(8):2213-21.
Benarroch, E.E. (2007). Tissue plasminogen activator: beyond thrombolysis. Neurology 69, 799-802.
Berger, D.H. (2002). Plasmin/plasminogen system in colorectal cancer. World J Surg 26, 767-771.
Ben QW, Wang JC, Liu J, Zhu Y, Yuan F, Yao WY, Yuan YZ. Positive expression of L1-CAM is associated with perineural invasion and poor outcome in pancreatic ductal adenocarcinoma. Ann Surg Oncol. 2010 Aug;17(8):2213-21.
Blouw, B., Song, H., Tihan, T., Bosze, J., Ferrara, N., Gerber, H.P., Johnson, R.S., and Bergers, G. (2003). The hypoxic response of tumors is dependent on their microenvironment. Cancer Cell 4, 133-146.
Boo, Y.J., Park, J.M., Kim, J., Chae, Y.S., Min, B.W., Um, J.W., and Moon, H.Y. (2007). L1 expression as a marker for poor prognosis, tumor progression, and short survival in patients with colorectal cancer. Ann Surg Oncol 14, 1703-1711.
Bos, P.D., Zhang, X.H., Nadal, C., Shu, W., Gomis, R.R., Nguyen, D.X., Minn, A.J., van de Vijver, M.J., Gerald, W.L., Foekens, J.A., et al. (2009). Genes that mediate breast cancer metastasis to the brain. Nature 459, 1005-1009.
Carbonell, W.S., Ansorge, O., Sibson, N., and Muschel, R. (2009). The vascular basement membrane as "soil" in brain metastasis. PloS one 4, e5857.
Castellani, V., De Angelis, E., Kenwrick, S., and Rougon, G. (2002). Cis and trans interactions of L1 with neuropilin-1 control axonal responses to semaphorin 3A. EMBO J 21, 6348-6357.
Chambers, A.F., Groom, A.C., and MacDonald, I.C. (2002). Dissemination and growth of cancer cells in metastatic sites. Nat Rev Cancer 2, 563-572.
Chambers, A.M., I; Schmidt, E; Morris, V; Groom, A (2000). Clinical targets for antimetastasis therapy. Adv Cancer Res 79, 91-121.
Chen, Z.L., and Strickland, S. (1997). Neuronal death in the hippocampus is promoted by plasmin-catalyzed degradation of laminin. Cell 91, 917-925.
Chen DL, Zeng ZL, Yang J, Ren C, Wang DS, Wu WJ, Xu RH. L1cam promotes tumor progression and metastasis and is an independent unfavorable prognostic factor in gastric cancer. J Hematol Oncol. 2013 Jun 27;6:43.
Chinnaiyan, A.M., Tepper, C.G., Seldin, M.F., O'Rourke, K., Kischkel, F.C., Hellbardt, S., Krammer, P.H., Peter, M.E., and Dixit, V.M. (1996). FADD/MORT1 is a common mediator of CD95 (Fas/APO-1) and tumor necrosis factor receptor-induced apoptosis. J Biol Chem 271, 4961-4965.
Choi, C., and Benveniste, E.N. (2004). Fas ligand/Fas system in the brain: regulator of immune and apoptotic responses. Brain Res Rev 44, 65-81.
Demyanenko, G.P., Tsai, A.Y., and Maness, P.F. (1999). Abnormalities in neuronal process extension, hippocampal development, and the ventricular system of L1 knockout mice. J Neurosci 19, 4907-4920.
Dietrich, P.Y., Walker, P.R., and Saas, P. (2003). Death receptors on reactive astrocytes: a key role in the fine tuning of brain inflammation? Neurology 60, 548-554.
Dippel V, Milde-Langosch K, Wicklein D, Schumacher U, Altevogt P, Oliveira-Ferrer L, Jänicke F, Schroder C. (2013). Influence of L1-CAM expression of breast cancer cells on adhesion to endothelial cells. J Cancer Res Clin Oncol. 2013 Jan;139(1):107-21. doi: 10.1007/s00432-012-1306-z. Epub 2012 Sep 16.
Doberstein, K., Wieland, A., Lee, S.B., Blaheta, R.A., Wedel, S., Moch, H., Schraml, P., Pfeilschifter, J., Kristiansen, G., and Gutwein, P. (2011). L1-CAM expression in ccRCC correlates with shorter patients survival times and confers chemoresistance in renal cell carcinoma cells. Carcinogenesis 32, 262-270.
Donier, E., Gomez-Sanchez, J.A., Grijota-Martinez, C., Lakoma, J., Baars, S., Garcia- Alonso, L., and Cabedo, H. (2012). L1CAM binds ErbB receptors through Ig-like domains coupling cell adhesion and neuregulin signalling. PloS one 7, e40674.
Fabbro, S., and Seeds, N.W. (2009). Plasminogen activator activity is inhibited while neuroserpin is up-regulated in the Alzheimer disease brain. J Neurochem 109, 303-315.
Fang, H., Placencio, V.R., and DeClerck, Y.A. (2012). Protumorigenic activity of plasminogen activator inhibitor-1 through an antiapoptotic function. J Natl Cancer Inst 104, 1470-1484.
Feld, R., Rubinstein, L.V., and Weisenberger, T.H. (1984). Sites of recurrence in resected stage I non-small-cell lung cancer: a guide for future studies. J Clin Oncol 2, 1352-1358.
Felding-Habermann, B., Silletti, S., Mei, F., Siu, C.H., Yip, P.M., Brooks, P.C., Cheresh, D.A., O'Toole, T.E., Ginsberg, M.H., and Montgomery, A.M. (1997). A single immunoglobulin-like domain of the human neural cell adhesion molecule L1 supports adhesion by multiple vascular and platelet integrins. J Cell Biol 139, 1567-1581.
Fidler, I.J. (2003). The pathogenesis of cancer metastasis: the 'seed and soil' hypothesis revisited. Nat Rev Cancer 3, 453-458.
Foekens, J.A., Look, M.P., Peters, H.A., van Putten, W.L., Portengen, H., and Klijn, J.G. (1995). Urokinase-type plasminogen activator and its inhibitor PAI-1: predictors of poor response to tamoxifen therapy in recurrent breast cancer. J Natl Cancer Inst 87, 751- 756.
Fogel, M., Gutwein, P., Mechtersheimer, S., Riedle, S., Stoeck, A., Smirnov, A., Edler, L., Ben-Arie, A., Huszar, M., and Altevogt, P. (2003a). L1 expression as a predictor of progression and survival in patients with uterine and ovarian carcinomas. Lancet 362, 869-875.
Fogel M, Mechtersheimer S, Huszar M, Smirnov A, Abu-Dahi A, Tilgen W, Reichrath J, Georg T, Altevogt P, Gutwein P. L1 adhesion molecule (CD 171) in development and progression of human malignant melanoma. Cancer Lett. (2003b) Jan 28;189(2):237-47.
Francia, G., Cruz-Munoz, W., Man, S., Xu, P., and Kerbel, R.S. (2011). Mouse models of advanced spontaneous metastasis for experimental therapeutics. Nat Rev Cancer 11, 135-141.
Fujimoto, S., Katsuki, H., Ohnishi, M., Takagi, M., Kume, T., and Akaike, A. (2008). Plasminogen potentiates thrombin cytotoxicity and contributes to pathology of intracerebral hemorrhage in rats. J Cereb Blood Flow Metab 28, 506-515.
Ganesh, B.S., and Chintala, S.K. (2011). Inhibition of reactive gliosis attenuates excitotoxicity-mediated death of retinal ganglion cells. PloS one 6, e18305.
Gavrilovic, I.T., and Posner, J.B. (2005). Brain metastases: epidemiology and pathophysiology. J Neurooncol 75, 5-14.
Gupta, G.P., and Massagué, J. (2006). Cancer metastasis: building a framework. Cell 127, 679-695.
Gutierrez-Fernandez, A., Gingles, N.A., Bai, H., Castellino, F.J., Parmer, R.J., and Miles, L.A. (2009). Plasminogen enhances neuritogenesis on laminin-1. J Neurosci 29, 12393- 12400.
Hai, J., Zhu, C.Q., Bandarchi, B., Wang, Y.H., Navab, R., Shepherd, F.A., Jurisica, I., and Tsao, M.S. (2012). L1 cell adhesion molecule promotes tumorigenicity and metastatic potential in non-small cell lung cancer. Clin Cancer Res 18, 1914-1924.
Harbeck, N., Thomssen, C., Berger, U., Ulm, K., Kates, R.E., Hofler, H., Janicke, F., Graeff, H., and Schmitt, M. (1999). Invasion marker PAI-1 remains a strong prognostic factor after long-term follow-up both for primary breast cancer and following first relapse. Breast Cancer Res Treat 54, 147-157.
Herron, L.R., Hill, M., Davey, F., and Gunn-Moore, F.J. (2009). The intracellular interactions of the L1 family of cell adhesion molecules. Biochem J 419, 519-531.
Heyn, C., Ronald, J.A., Ramadan, S.S., Snir, J.A., Barry, A.M., MacKenzie, L.T., Mikulis, D.J., Palmieri, D., Bronder, J.L., Steeg, P.S., et al. (2006). In vivo MRI of cancer cell fate at the single-cell level in a mouse model of breast cancer metastasis to the brain. Magn Reson Med 56, 1001-1010.
Hoffman, E/. Minthz, C. D., Wang, S., McNickie, D,m Salton, S., Benson, D. (2008) Effects of alcohol on axon outgrowth and branching in developing rat cortical neurons. Neurosci. 157(3), 556-565.
Hoover-Plow, J., Skomorovska-Prokvolit, O., and Welsh, S. (2001). Selective behaviors altered in plasminogen-deficient mice are reconstituted with intracerebroventricular injection of plasminogen. Brain Res 898, 256-264.
Irving, J.A., Pike, R.N., Lesk, A.M., and Whisstock, J.C. (2000). Phylogeny of the serpin superfamily: implications of patterns of amino acid conservation for structure and function. Genome Res 10, 1845-1864.
Kang, Y., Siegel, P.M., Shu, W., Drobnjak, M., Kakonen, S.M., Cordón-Cardo, C., Guise, T.A., and Massagué, J. (2003). A multigenic program mediating breast cancer metastasis to bone. Cancer Cell 3, 537-549.
Karrison, T.G., Ferguson, D.J., and Meier, P. (1999). Dormancy of mammary carcinoma after mastectomy. J Natl Cancer Inst 91, 80-85.
Kienast, Y., von Baumgarten, L., Fuhrmann, M., Klinkert, W.E., Goldbrunner, R., Herms, J., and Winkler, F. (2010). Real-time imaging reveals the single steps of brain metastasis formation. Nat Med 16, 116-122.
Kim HS, Yi SY, Jun HJ, Ahn JS, Ahn MJ, Lee J, Kim Y, Cui ZY, Hong HJ, Kim JM, Li S, Hwang IG, Park K. L1 cell adhesion molecule as a predictor for recurrence in pulmonary carcinoids and large-cell neuroendocrine tumors. APMIS. 2009 Feb;117(2):140-6.
Kim, S.J., Kim, J.S., Park, E.S., Lee, J.S., Lin, Q., Langley, R.R., Maya, M., He, J., Kim, S.W., Weihua, Z., et al. (2011). Astrocytes upregulate survival genes in tumor cells and induce protection from chemotherapy. Neoplasia 13, 286-298.
Krammer, P.H. (2000). CD95's deadly mission in the immune system. Nature 407, 789- 795.
Kulahin, N., Li, S., Hinsby, A., Kiselyov, V., Berezin, V., and Bock, E. (2008). Fibronectin type III (FN3) modules of the neuronal cell adhesion molecule L1 interact directly with the fibroblast growth factor (FGF) receptor. Mol Cell Neurosci 37, 528-536.
Law, R.H., Zhang, Q., McGowan, S., Buckle, A.M., Silverman, G.A., Wong, W., Rosado, C.J., Langendorf, C.G., Pike, R.N., Bird, P.I., et al. (2006). An overview of the serpin superfamily. Genome Biol 7, 216.
Leenders, W.P., Kusters, B., and de Waal, R.M. (2002). Vessel co-option: how tumors obtain blood supply in the absence of sprouting angiogenesis. Endothelium 9, 83-87.
Leenders, W.P., Kusters, B., Verrijp, K., Maass, C., Wesseling, P., Heerschap, A., Ruiter, D., Ryan, A., and de Waal, R. (2004). Antiangiogenic therapy of cerebral melanoma metastases results in sustained tumor progression via vessel co-option. Clin Cancer Res 10, 6222-6230.
Leyland-Jones, B. (2009). Human epidermal growth factor receptor 2-positive breast cancer and central nervous system metastases. J Clin Oncol 27, 5278-5286.
Li, B., Wang, C., Zhang, Y., Zhao, X.Y., Huang, B., Wu, P.F., Li, Q., Li, H., Liu, Y.S., Cao, L.Y., et al. (2013). Elevated PLGF contributes to small-cell lung cancer brain metastasis. Oncogene 32, 2952-2962.
Lin, N.U., and Winer, E.P. (2007). Brain metastases: the HER2 paradigm. Clin Cancer Res 13, 1648-1655.
Lin, Q.B., K.; Fan, D.; Kim, S-J.; Guo, L.; Wang, H.; Bar-Eli, M.; Aldape, K. D.; Fidler, I. J. (2010). Reactive astrocytes protect melanoma cells from chemotherapy by sequestering intracellular calcium through gap junction communication channels. Neoplasia 12, 748-754.
Lorger, M., and Felding-Habermann, B. (2010). Capturing changes in the brain microenvironment during initial steps of breast cancer brain metastasis. Am J Pathol 176, 2958-2971.
Luo JL, Tan W, Ricono JM, Korchynskyi O, Zhang M, Gonias SL, Cheresh DA, Karin M. (2007). "Nuclear cytokine-activated IKKalpha controls prostate cancer metastasis by repressing Maspin". Nature. 446 (7136): 690-4. doi:10.1038/nature05656. PMID 17377533.
Lutterbach, J., Bartelt, S., and Ostertag, C. (2002). Long-term survival in patients with brain metastases. J Cancer Res Clin Oncol 128, 417-425.
Maher, E.A., Mietz, J., Arteaga, C.L., DePinho, R.A., and Mohla, S. (2009). Brain metastasis: opportunities in basic and translational research. Cancer Res 69, 6015-6020.
Maness, P.F., and Schachner, M. (2007). Neural recognition molecules of the immunoglobulin superfamily: signaling transducers of axon guidance and neuronal migration. Nat Neurosci 10, 19-26.
McMahon, B., and Kwaan, H.C. (2008). The plasminogen activator system and cancer. Pathophysiol Haemost Thromb 36, 184-194.
Mechtersheimer, S., Gutwein, P., Agmon-Levin, N., Stoeck, A., Oleszewski, M., Riedle, S., Postina, R., Fahrenholz, F., Fogel, M., Lemmon, V., et al. (2001). Ectodomain shedding of L1 adhesion molecule promotes cell migration by autocrine binding to integrins. J Cell Biol 155, 661-673.
Meuwissen, R., Linn, S.C., Linnoila, R.I., Zevenhoven, J., Mooi, W.J., and Berns, A. (2003). Induction of small cell lung cancer by somatic inactivation of both Trp53 and Rb1 in a conditional mouse model. Cancer Cell 4, 181-189.
Minn, A.J., Gupta, G.P., Siegel, P.M., Bos, P.D., Shu, W., Giri, D.D., Viale, A., Olshen, A.B., Gerald, W.L., and Massagué, J. (2005). Genes that mediate breast cancer metastasis to lung. Nature 436, 518-524.
Mire E., Thomasett, N., Jakeman, L., Rougon, G, (2008) Modulating Sema3A signal with a L1 mimetic peptide is not sufficient to promote motor recovery and axon regeneration after spinal cord injury. Mol. Cell. Neurosci. 37(2), 222-235.
Moody, S.E., Sarkisian, C.J., Hahn, K.T., Gunther, E.J., Pickup, S., Dugan, K.D., Innocent, N., Cardiff, R.D., Schnall, M.D., and Chodosh, L.A. (2002). Conditional activation of Neu in the mammary epithelium of transgenic mice results in reversible pulmonary metastasis. Cancer Cell 2, 451-461.
Morita, S., Sato, A., Hayakawa, H., Ihara, H., Urano, T., Takada, Y., and Takada, A. (1998). Cancer cells overexpress mRNA of urokinase-type plasminogen activator, its receptor and inhibitors in human non-small-cell lung cancer tissue: analysis by Northern blotting and in situ hybridization. Int J Cancer 78, 286-292.
Muller, W.J., Sinn, E., Pattengale, P.K., Wallace, R., and Leder, P. (1988). Single-step induction of mammary adenocarcinoma in transgenic mice bearing the activated c-neu oncogene. Cell 54, 105-115.
Nayeem, N., Silletti, S., Yang, X., Lemmon, V.P., Reisfeld, R.A., Stallcup, W.B., and Montgomery, A.M. (1999). A potential role for the plasmin(ogen) system in the posttranslational cleavage of the neural cell adhesion molecule L1. J Cell Sci 112 (Pt 24), 4739-4749.
Nguyen, D.X., Bos, P.D., and Massagué, J. (2009a). Metastasis: from dissemination to organ-specific colonization. Nat Rev Cancer 9, 274-284.
Nguyen, D.X., Chiang, A.C., Zhang, X.H., Kim, J.Y., Kris, M.G., Ladanyi, M., Gerald, W.L., and Massagué, J. (2009b). WNT/TCF signaling through LEF1 and HOXB9 mediates lung adenocarcinoma metastasis. Cell 138, 51-62.
Palmieri, D., Bronder, J.L., Herring, J.M., Yoneda, T., Weil, R.J., Stark, A.M., Kurek, R., Vega-Valle, E., Feigenbaum, L., Halverson, D., et al. (2007). Her-2 overexpression increases the metastatic outgrowth of breast cancer cells in the brain. Cancer Res 67, 4190-4198.
Pang, P.T., Teng, H.K., Zaitsev, E., Woo, N.T., Sakata, K., Zhen, S., Teng, K.K., Yung, W.H., Hempstead, B.L., and Lu, B. (2004). Cleavage of proBDNF by tPA/plasmin is essential for long-term hippocampal plasticity. Science 306, 487-491.
Perera, M., Ribot, E.J., Percy, D.B., McFadden, C., Simedrea, C., Palmieri, D., Chambers, A.F., and Foster, P.J. (2012). In vivo magnetic resonance imaging for investigating the development and distribution of experimental brain metastases due to breast cancer. Transl Oncol 5, 217-225.
Polleux, F., and Ghosh, A. (2002). The slice overlay assay: a versatile tool to study the influence of extracellular signals on neuronal development. Sci STKE 136, 19-29.
Qian, Y., Hua, E., Bisht, K., Woditschka, S., Skordos, K.W., Liewehr, D.J., Steinberg, S.M., Brogi, E., Akram, M.M., Killian, J.K., et al, (2011). Inhibition of Polo-like kinase 1 prevents the growth of metastatic breast cancer cells in the brain. Clin Exp Metastasis 28, 899-908.
Regales, L., Gong, Y., Shen, R., de Stanchina, E., Vivanco, I., Goel, A., Koutcher, J.A., Spassova, M., Ouerfelli, O., Mellinghoff, I.K., et al. (2009). Dual targeting of EGFR can overcome a major drug resistance mutation in mouse models of EGFR mutant lung cancer. J Clin Invest 119, 3000-3010.
Schafer, M.K., and Altevogt, P. (2010). L1CAM malfunction in the nervous system and human carcinomas. Cell Mol Life Sci 67, 2425-2437.
Schildge, S., Bohrer, C., Beck, K., and Schachtrup, C. (2013). Isolation and culture of mouse cortical astrocytes. Journal of visualized experiments : JoVE.
Schmidt-Kittler, O., Ragg, T., Daskalakis, A., Granzow, M., Ahr, A., Blankenstein, T.J., Kaufmann, M., Diebold, J., Arnholdt, H., Muller, P., et al. (2003). From latent disseminated cells to overt metastasis: genetic analysis of systemic breast cancer progression. Proc Natl Acad Sci U S A 100, 7737-7742.
Schouten, L.J., Rutten, J., Huveneers, H.A., and Twijnstra, A. (2002). Incidence of brain metastases in a cohort of patients with carcinoma of the breast, colon, kidney, and lung and melanoma. Cancer 94, 2698-2705.
Schreiber, R.D., Old, L.J., and Smyth, M.J. (2011). Cancer immunoediting: integrating immunity's roles in cancer suppression and promotion. Science 331, 1565-1570.
Schroder, C., Schumacher, U., Fogel, M., Feuerhake, F., Muller, V., Wirtz, R.M., Altevogt, P., Krenkel, S., Janicke, F., and Milde-Langosch, K. (2009). Expression and prognostic value of L1-CAM in breast cancer. Oncol Rep 22, 1109-1117.
Seike, T., Fujita, K., Yamakawa, Y., Kido, M.A., Takiguchi, S., Teramoto, N., Iguchi, H., and Noda, M. (2011). Interaction between lung cancer cells and astrocytes via specific inflammatory cytokines in the microenvironment of brain metastasis. Clin Exp Metastasis 28, 13-25.
Siegel, P.M., Shu, W., Cardiff, R.D., Muller, W.J., and Massagué, J. (2003). Transforming growth factor beta signaling impairs Neu-induced mammary tumorigenesis while promoting pulmonary metastasis. Proc Natl Acad Sci U S A 100, 8430-8435.
Silletti, S., Mei, F., Sheppard, D., and Montgomery, A.M. (2000). Plasmin-sensitive dibasic sequences in the third fibronectin-like domain of L1-cell adhesion molecule (CAM) facilitate homomultimerization and concomitant integrin recruitment. J Cell Biol 149, 1485-1502.
Sledge, G.W., Jr. (2011). HER2011: the changing face of HER2-positive breast cancer. Clin Breast Cancer 11, 9.
Sofroniew, M.V., and Vinters, H.V. (2010). Astrocytes: biology and pathology. Acta Neuropathol Suppl (Berl) 119, 7-35.
Steeg, P.S., Camphausen, K.A., and Smith, Q.R. (2011). Brain metastases as preventive and therapeutic targets. Nat Rev Cancer 11, 352-363.
Stemmler, H.J., Kahlert, S., Siekiera, W., Untch, M., Heinrich, B., and Heinemann, V. (2006). Characteristics of patients with brain metastases receiving trastuzumab for HER2 overexpressing metastatic breast cancer. Breast 15, 219-225.
Takehara, S., Onda, M., Zhang, J., Nishiyama, M., Yang, X., Mikami, B., and Lomas, D.A. (2009). The 2.1-A crystal structure of native neuroserpin reveals unique structural elements that contribute to conformational instability. J Mol Biol 388, 11-20.
Teesalu, T., Hinkkanen, A.E., and Vaheri, A. (2001). Coordinated induction of extracellular proteolysis systems during experimental autoimmune encephalomyelitis in mice. Am J Pathol 159, 2227-2237.
Thies, A., Schachner, M., Moll, I., Berger, J., Schulze, H.J., Brunner, G., and Schumacher, U. (2002). Overexpression of the cell adhesion molecule L1 is associated with metastasis in cutaneous malignant melanoma. Eur J Cancer 38, 1708-1716.
Timmer, T., de Vries, E.G., and de Jong, S. (2002). Fas receptor-mediated apoptosis: a clinical application? J Pathol 196, 125-134.
Tischler, V., Pfeifer, M., Hausladen, S., Schirmer, U., Bonde, A.K., Kristiansen, G., Sos, M.L., Weder, W., Moch, H., Altevogt, P., et al. (2011). L1CAM protein expression is associated with poor prognosis in non-small cell lung cancer. Mol Cancer 10, 127-137.
Tsutsumi, S., Morohashi, S., Kudo, Y., Akasaka, H., Ogasawara, H., Ono, M., Takasugi, K., Ishido, K., Hakamada, K., and Kijima, H. (2011). L1 Cell adhesion molecule (L1CAM) expression at the cancer invasive front is a novel prognostic marker of pancreatic ductal adenocarcinoma. J Surg Oncol 103, 669-673.
United States Patent No. 8,138,313, International Patent Application Publication No. WO 2007114550, and International Patent Application Publication No. WO 2008151819.
Valastyan, S., and Weinberg, R.A. (2011). Tumor metastasis: molecular insights and evolving paradigms. Cell 147, 275-292.
Vanharanta, S., and Massagué, J. (2013). Origins of metastatic traits. Cancer Cell. 2013 Oct 14;24(4):410-21. doi: 10.1016/j.ccr.2013.09.007.
Vos, Y.J., and Hofstra, R.M. (2010). An updated and upgraded L1CAM mutation database. Hum Mutat 31, E1 102-1109.
Voura, E.B., Ramjeesingh, R.A., Montgomery, A.M., and Siu, C.H. (2001). Involvement of integrin alpha(v)beta(3) and cell adhesion molecule L1 in transendothelial migration of melanoma cells. Mol Biol Cell 12, 2699-2710.
Wang, X., Haroon, F., Karray, S., Martina, D., and Schluter, D. (2013). Astrocytic Fas ligand expression is required to induce T-cell apoptosis and recovery from experimental autoimmune encephalomyelitis. Eur J Immunol 43, 115-124.
Wiencken-Barger, A.E., Mavity-Hudson, J., Bartsch, U., Schachner, M., and Casagrande, V.A. (2004). The role of L1 in axon pathfinding and fasciculation. Cereb Cortex 14, 121-131.
Winslow, M.M., Dayton, T.L., Verhaak, R.G., Kim-Kiselak, C., Snyder, E.L., Feldser, D.M., Hubbard, D.D., DuPage, M.J., Whittaker, C.A., Hoersch, S., et al. (2011). Suppression of lung adenocarcinoma progression by Nkx2-1. Nature 473, 101-104.
Yepes, M., Sandkvist, M., Wong, M.K., Coleman, T.A., Smith, E., Cohan, S.L., and Lawrence, D.A. (2000). Neuroserpin reduces cerebral infarct volume and protects neurons from ischemia-induced apoptosis. Blood 96, 569-576.
Zhu, C.Q., Ding, K., Strumpf, D., Weir, B.A., Meyerson, M., Pennell, N., Thomas, R.K., Naoki, K., Ladd-Acosta, C., Liu, N., et al. (2010). Prognostic and predictive gene signature for adjuvant chemotherapy in resected non-small-cell lung cancer. J Clin Oncol 28, 4417-4424.
Zou Z, Anisowicz A, Hendrix MJ, Thor A, Neveu M, Sheng S, Rafidi K, Seftor E, Sager R. (1994). "Maspin, a serpin with tumor-suppressing activity in human mammary epithelial cells". Science 263 (5146): 526-9. doi:10.1126/science.8290962. PMID 8290962.

## Claims

1. An L1CAM inhibitor for use in inhibiting metastatic spread of a cancer in a subject, where it has been determined in a cell of the cancer that the cell (i) overexpresses a serpin, wherein the metastasis inhibited is metastasis to the brain or a bone in the subject.

2. The L1CAM inhibitor for use of claim 1, wherein the cell (ii) expresses L1CAM.

3. The L1CAM inhibitor for use of claim 1 or 2, wherein the serpin is neuroserpin, serpin B2, serpin D1 or serpin E2.

4. The L1CAM inhibitor for use of any one of claims 1-3, wherein the cancer is breast cancer or lung cancer.

5. The L1CAM inhibitor for use of any one of claims 1-4, wherein the L1CAM inhibitor is an immunoglobulin or an interfering RNA.

6. A method of predicting inhibition of metastatic spread of a cancer by an L1CAM inhibitor, comprising determining whether a cell of the cancer overexpresses a serpin, wherein overexpression of the serpin indicates that the L1CAM inhibitor is likely to inhibit metastatic spread of the cancer, and the metastasis inhibited is metastasis to brain or a bone.

7. The method of claim 6, wherein the serpin is neuroserpin, serpin B2, serpin D1 or serpin E2.

8. The method of claim 6 or 7, wherein the cancer is breast cancer or lung cancer.

9. Use of a probe, primer(s), microarray, an immunoglobulin, a fragment thereof, or a pair of PCR primers for determining whether a cell of a cancer overexpresses a serpin for predicting inhibition of metastatic spread of a cancer to brain or a bone by an L1CAM inhibitor, wherein overexpression of the serpin indicates that the L1CAM inhibitor is likely to inhibit metastatic spread of the cancer.

10. Use of a combination of a means for predicting inhibition of metastatic spread of the cancer by an L1CAM inhibitor, wherein overexpression of the serpin and expression of L1CAM indicates that L1CAM inhibitor is likely to inhibit metastatic spread of the cancer, wherein the metastasis inhibited is metastasis to brain or a bone wherein the means for determining whether the cell of the cancer expresses L1CAM is selected from the group consisting of a probe, primer(s), microarray, an immunoglobulin, a fragment thereof, and a pair of PCR primers, and
wherein the means for determining whether a cell of the cancer overexpresses a serpin is selected from the group consisting of a probe, primer(s), microarray, an immunoglobulin, a fragment thereof, and a pair of PCR primers.

11. The means for use of claim 9 or 10, wherein the serpin is neuroserpin, serpin B2, serpin D1 or serpin E2.

12. Use of a kit for predicting inhibition of metastatic spread of a cancer by an L1CAM inhibitor, comprising a means for determining whether a cell of the cancer overexpresses a serpin and a means for determining whether the cell expresses L1CAM, wherein overexpression of the serpin in the cell indicates that the L1CAM inhibitor is likely to inhibit metastatic spread of the cancer, wherein the metastasis inhibited is metastasis to brain or a bone,
wherein the means for determining whether the cell of the cancer expresses L1CAM is selected from the group consisting of a probe, primer(s), microarray, an immunoglobulin, a fragment thereof, and a pair of PCR primers, and
wherein the means for determining whether a cell of the cancer overexpresses a serpin is selected from the group consisting of a probe, primer(s), microarray, an immunoglobulin, a fragment thereof, and a pair of PCR primers.

13. The use of the kit of claim 12, wherein the serpin is neuroserpin, serpin B2, serpin D1 or serpin E2.

## Patentansprüche

1. L1CAM-Hemmer zur Verwendung bei der Hemmung einer metastatischen Ausbreitung eines Krebses in einem Individuum, wobei es in einer Zelle des Krebses bestimmt wurde, dass die Zelle (i) ein Serpin überexprimiert, wobei die gehemmte Metastase eine Metastase im Gehirn oder in einem Knochen des Individuums ist.

2. L1CAM-Hemmer zur Verwendung nach Anspruch 1, wobei die Zelle (ii) L1CAM exprimiert.

3. L1CAM-Hemmer zur Verwendung nach Anspruch 1 oder 2, wobei das Serpin Neuroserpin, Serpin B2, Serpin D1 oder Serpin E2 ist.

4. L1CAM-Hemmer zur Verwendung nach einem der Ansprüche 1-3, wobei der Krebs Brustkrebs oder Lungenkrebs ist.

5. L1CAM-Hemmer zur Verwendung nach einem der Ansprüche 1-4, wobei der L1CAM-Hemmer ein Immunoglobulin oder eine interferierende RNA ist.

6. Verfahren zur Vorhersage der Hemmung einer metastatischen Ausbreitung eines Krebses durch einen L1CAM-Hemmer, umfassend das Bestimmen, ob eine Zelle des Krebses ein Serpin überexprimiert, wobei eine Überexpression des Serpins anzeigt, dass der L1CAM-Hemmer eine metastatische Ausbreitung des Krebses wahrscheinlich hemmen wird, und die gehemmte Metastase eine Metastase im Gehirn oder in einem Knochen ist.

7. Verfahren nach Anspruch 6, wobei das Serpin Neuroserpin, Serpin B2, Serpin D1 oder Serpin E2 ist.

8. Verfahren nach Anspruch 6 oder 7, wobei der Krebs Brustkrebs oder Lungenkrebs ist.

9. Verwendung einer Sonde, eines Primers oder von Primern, eines Microarrays, eines Immunoglobulins, eines Fragmentes davon oder eines Paares von PCR-Primern zum Bestimmen, ob eine Zelle eines Krebses ein Serpin überexprimiert, um die Hemmung einer metastatischen Ausbreitung eines Krebses in einem Gehirn oder einem Knochen durch einen L1CAM-Hemmer vorherzusagen, wobei eine Überexpression des Serpins anzeigt, dass der L1CAM-Hemmer eine metastatische Ausbreitung des Krebses wahrscheinlich hemmen wird.

10. Verwendung einer Kombination eines Mittels zur Vorhersage einer Hemmung einer metastatischen Ausbreitung des Krebses durch einen L1CAM-Hemmer, wobei eine Überexpression des Serpins und eine Überexpression von L1CAM anzeigt, dass der L1CAM-Hemmer eine metastatische Ausbreitung des Krebses wahrscheinlich hemmen wird, wobei die gehemmte Metastase eine Metastase im Gehirn oder in einem Knochen ist, wobei das Mittel zum Bestimmen, ob die Zelle des Krebses L1CAM exprimiert, aus der Gruppe bestehend aus einer Sonde, einem Primer oder aus Primern, einem Microarray, einem Immunoglobulin, einem Fragment davon und einem Paar von PCR-Primern ausgewählt ist, und
wobei das Mittel zum Bestimmen, ob eine Zelle des Krebses ein Serpin überexprimiert, aus der Gruppe bestehend aus einer Sonde, einem Primer oder aus Primern, einem Microarray, einem Immunoglobulin, einem Fragment davon und einem Paar von PCR-Primern ausgewählt ist.

11. Mittel zur Verwendung nach Anspruch 9 oder 10, wobei das Serpin Neuroserpin, Serpin B2, Serpin D1 oder Serpin E2 ist.

12. Verwendung eines Kits zur Vorhersage der Hemmung einer metastatischen Ausbreitung eines Krebses durch einen L1CAM-Hemmer, umfassend das Bestimmen, ob eine Zelle des Krebses ein Serpin überexprimiert, und ein Mittel zum Bestimmen, ob die Zelle L1CAM exprimiert, wobei eine Überexpression des Serpins in der Zelle anzeigt, dass der L1CAM-Hemmer eine metastatische Ausbreitung des Krebses wahrscheinlich hemmen wird, wobei die gehemmte Metastase eine Metastase im Gehirn oder in einem Knochen ist, wobei das Mittel zum Bestimmen, ob die Zelle des Krebses L1CAM überexprimiert, aus der Gruppe bestehend aus einer Sonde, einem Primer oder aus Primern, einem Microarray, einem Immunoglobulin, einem Fragment davon und einem Paar von PCR-Primern ausgewählt ist, und
wobei das Mittel zum Bestimmen, ob eine Zelle des Krebses ein Serpin überexprimiert, aus der Gruppe bestehend aus einer Sonde, einem Primer oder aus Primern, einem Microarray, einem Immunoglobulin, einem Fragment davon und einem Paar von PCR-Primern ausgewählt ist.

13. Verwendung des Kits nach Anspruch 12, wobei das Serpin Neuroserpin, Serpin B2, Serpin D1 oder Serpin E2 ist.

## Revendications

1. Inhibiteur de L1CAM pour l'usage dans l'inhibition de la propagation métastatique d'un cancer chez un sujet, où on a déterminé dans une cellule du cancer que la cellule (i) surexprime une serpine, la métastase inhibée étant une métastase au cerveau ou un os chez le sujet.

2. Inhibiteur de L1CAM pour l'usage selon la revendication 1, dans lequel la cellule (ii) exprime L1CAM.

3. Inhibiteur de L1CAM pour l'usage selon la revendication 1 ou 2, dans lequel la serpine est la neuroserpine, la serpine B2, la serpine D1 ou la serpine E2.

4. Inhibiteur de L1CAM pour l'usage selon l'une quelconque des revendications 1 à 3, dans lequel le cancer est un cancer du sein ou un cancer du poumon.

5. Inhibiteur de L1CAM pour l'usage selon l'une quelconque des revendications 1 à 4, dans lequel l'inhibiteur de L1CAM est une immunoglobuline ou un ARN interférant.

6. Procédé de prédiction de l'inhibition de la propagation métastatique d'un cancer au moyen d'un inhibiteur de L1CAM, comprenant la détermination de savoir si une cellule du cancer surexprime une serpine, la surexpression de la serpine indiquant que l'inhibiteur de L1CAM est susceptible d'inhiber la propagation métastatique du cancer, et la métastase inhibée étant une métastase au cerveau ou un os.

7. Procédé selon la revendication 6, dans lequel la serpine est la neuroserpine, la serpine B2, la serpine D1 ou la serpine E2.

8. Procédé selon la revendication 6 ou 7, dans lequel le cancer est un cancer du sein ou un cancer du poumon.

9. Usage d'une sonde, d'un ou de plusieurs amorces, d'un micro-réseau, d'une immunoglobuline, d'un fragment de celle-ci ou d'une paire d'amorces de PCR pour la détermination de savoir si une cellule du cancer surexprime une serpine pour la prédiction d'une propagation métastatique d'un cancer au cerveau ou un os par un inhibiteur de L1CAM, la surexpression de la serpine indiquant que l'inhibiteur de L1CAM est susceptible d'inhiber la propagation métastatique du cancer.

10. Usage d'une combinaison d'un moyen pour la prédiction de l'inhibition d'une propagation métastatique du cancer par un inhibiteur de L1CAM, la surexpression de la serpine et l'expression de L1CAM indiquant que l'inhibiteur de L1CAM est susceptible d'inhiber la propagation métastatique du cancer, dans lequel la métastase inhibée est une métastase au cerveau ou un os, dans lequel le moyen pour déterminer si la cellule du cancer exprime L1CAM est choisi parmi le groupe composé d'une sonde, d'un ou de plusieurs amorces, d'un micro-réseau, d'une immunoglobuline, d'un fragment de celle-ci et d'une paire d'amorces de PCR, et
dans lequel le moyen pour déterminer si une cellule du cancer surexprime une serpine est choisi parmi le groupe composé d'une sonde, d'un ou de plusieurs amorces, d'un micro-réseau, d'une immunoglobuline, d'un fragment de celle-ci et d'une paire d'amorces de PCR.

11. Moyen pour l'usage selon la revendication 9 ou 10, dans lequel la serpine est la neuroserpine, la serpine B2, la serpine D1 ou la serpine E2.

12. Usage d'une trousse pour la prédiction de l'inhibition de la propagation métastatique d'un cancer au moyen d'un inhibiteur de L1CAM, comprenant un moyen pour déterminer si une cellule du cancer surexprime une serpine, et un moyen pour déterminer si la cellule surexprime L1CAM, la surexpression de la serpine dans la cellule indiquant que l'inhibiteur de L1CAM est susceptible d'inhiber la propagation métastatique du cancer, la métastase inhibée étant une métastase au cerveau ou un os,
dans lequel le moyen pour déterminer si la cellule du cancer exprime L1CAM est choisi parmi le groupe composé d'une sonde, d'un ou de plusieurs amorces, d'un micro-réseau, d'une immunoglobuline, d'un fragment de celle-ci et d'une paire d'amorces de PCR, et
dans lequel le moyen pour déterminer si une cellule du cancer surexprime une serpine est choisi parmi le groupe composé d'une sonde, d'un ou de plusieurs amorces, d'un micro-réseau, d'une immunoglobuline, d'un fragment de celle-ci et d'une paire d'amorces de PCR.

13. Usage de la trousse selon la revendication 12, dans lequel la serpine est la neuroserpine, la serpine B2, la serpine D1 ou la serpine E2.
